(19) Europäisches Patentamt / European Patent Office / Office européen des brevets

(11) **EP 1 765 753 B1**

(12) **EUROPÄISCHE PATENTSCHRIFT**

(45) Veröffentlichungstag und Bekanntmachung des Hinweises auf die Patenterteilung:
**05.10.2016 Patentblatt 2016/40**

(51) Int Cl.:
*C07C 51/25* (2006.01)          *B01J 8/04* (2006.01)
*C07C 45/33* (2006.01)          *C07C 51/215* (2006.01)
*C07C 45/35* (2006.01)

(21) Anmeldenummer: **05745477.9**

(22) Anmeldetag: **04.05.2005**

(86) Internationale Anmeldenummer:
**PCT/EP2005/004868**

(87) Internationale Veröffentlichungsnummer:
**WO 2006/002708 (12.01.2006 Gazette 2006/02)**

(54) **VERFAHREN ZUR HERSTELLUNG VON ACROLEIN ODER ACRYLSÄURE ODER DEREN GEMISCH DURCH HETEROGEN KATALYSIERTE PARTIELLE GASPHASENOXIDATION VON PROPYLEN**

METHOD FOR THE PRODUCTION OF ACROLEIN, ACRYLIC ACID, OR A MIXTURE THEREOF BY MEANS OF HETEROGENEOUSLY CATALYZED PARTIAL GAS PHASE OXIDATION OF PROPYLENE

PROCEDE DE PRODUCTION D'ACROLEINE OU D'ACIDE ACRYLIQUE ET DE LEUR MELANGE PAR OXYDATION PARTIELLE EN PHASE GAZEUSE A CATALYSE HETEROGENE DE PROPYLENE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität:
**01.07.2004 DE 102004032129**
**01.07.2004 US 584469 P**
**01.03.2005 DE 102005009885**
**01.03.2005 US 656874 P**
**02.03.2005 US 657374 P**

(43) Veröffentlichungstag der Anmeldung:
**28.03.2007 Patentblatt 2007/13**

(73) Patentinhaber: **BASF SE**
**67056 Ludwigshafen am Rhein (DE)**

(72) Erfinder:
• **DIETERLE, Martin**
  **68167 Mannheim (DE)**
• **DIEFENBACHER, Armin**
  **76726 Germersheim (DE)**
• **SCHINDLER, Götz-Peter**
  **68219 Mannheim (DE)**
• **KLANNER, Catharina**
  **68163 Mannheim (DE)**
• **MÜLLER-ENGEL, Klaus Joachim**
  **76297 Stutensee (DE)**
• **ADAMI, Christoph**
  **69469 Weinheim (DE)**

(56) Entgegenhaltungen:
**WO-A-00/53556      WO-A-00/53558**

**Beschreibung**

Vorliegende Erfindung betrifft ein Verfahren zur Herstellung von Acrolein oder Acrylsäure oder deren Gemisch durch heterogen katalysierte partielle Gasphasenoxidation von Propen, bei dem man ein die Reaktanden Propylen und molekularer Sauerstoff sowie die inerten Verdünnungsgase molekularer Stickstoff und Propan enthaltendes Reaktionsgasausgangsgemisch, das den molekularen Sauerstoff und das Propylen in einem molaren Verhältnis $O_2 : C_3H_6 \geq 1$ enthält, bei erhöhter Temperatur durch ein Katalysatorfestbett führt, dessen Aktivmasse wenigstens ein die Elemente Mo, Fe und Bi enthaltendes Multimetalloxid ist.

Acrolein bildet ein reaktives Monomer, das insbesondere als Zwischenprodukt, z. B. bei der Herstellung von Acrylsäure durch zweistufige heterogen katalysierte partielle Gasphasenoxidation von Propen von Bedeutung ist. Acrylsäure ist als solche oder in Form ihrer Alkylester z. B. zu Herstellung von Polymerisaten, die u.a. als Klebstoffe oder Wasser absorbierende Materialien Verwendung finden können, geeignet.

Die Herstellung von Acrolein durch das in der Präambel dieser Schrift beschriebene Verfahren der heterogen katalysierten partiellen Gasphasenoxidation ist bekannt (vgl. z. B. EP-A 11 06 598, WO 97/36849, EP-A 293 224, WO 01/96271, DE-A 198 37 517, EP-A 274 681, DE-A 198 37 519, DE-A 198 37 520, EP-A 117 146, WO 03/11804, US 3,161,670, WO 01/96270, DE-A 195 08 558, DE-A 33 13 573, DE-A 102 45 585, WO 03/076370, DE-A 103 16 039, WO 04/031106, sowie die deutsche Anmeldung DE-A 10 2004 032 129 und den in diesen Schriften zitierten Stand der Technik). Üblicherweise bildet sie die erste Stufe einer zweistufigen heterogen katalysierten Gasphasenpartialoxidation von Propen zu Acrylsäure. In der ersten Reaktionsstufe wird das Propen im wesentlichen partiell zu Acrolein oxidiert und in der zweiten Reaktionsstufe wird das in der ersten Reaktionsstufe gebildete Acrolein im wesentlichen partiell zu Acrylsäure oxidiert. Von Bedeutung ist dabei, dass die technische Ausführungsform normalerweise so ausgestaltet ist, dass das in der ersten Reaktionsstufe gebildete Acrolein nicht abgetrennt, sondern als Bestandteil des die erste Reaktionsstufe verlassenden Produktgasgemischs, gegebenenfalls durch molekularen Sauerstoff und Inertgas ergänzt, und gegebenenfalls durch direkte und/oder indirekte Kühlung abgekühlt, in die zweite Reaktionsstufe geführt wird.

Zielprodukt einer heterogen katalysierten Gasphasenpartialoxidation von Propen zu Acrolein ist Acrolein.

Problempunkt aller heterogen katalysierten Gasphasenpartialoxidationen im Katalysatorfestbett ist, dass das Reaktionsgasgemisch beim Durchströmen durch das Katalysatorfestbett einen Höchstwert, den sogenannten Heißpunktwert durchläuft. Dieser Höchstwert setzt sich zusammen aus der externen Temperierung des Katalysatorfestbetts und der Reaktionswärme.

Aus Gründen der Zweckmäßigkeit werden daher die Temperatur des Katalysatorfestbetts und die effektive Temperatur des Katalysatorfestbetts voneinander unterschieden. Dabei wird unter der Temperatur des Katalysatorfestbetts die Temperatur des Katalysatorfestbetts bei Ausübung des Partialoxidationsverfahrens, jedoch in fiktiver Abwesenheit einer chemischen Reaktion (d. h., ohne den Einfluss der Reaktionswärme) verstanden. Unter effektiver Temperatur des Katalysatorfestbetts wird dagegen die tatsächliche Temperatur des Katalysatorfestbetts unter Einbezug der Reaktionswärme der Partialoxidation verstanden. Ist die Temperatur des Katalysatorfestbetts längs des Katalysatorfestbetts nicht konstant (z. B. im Fall von mehreren Temperaturzonen), so meint der Begriff Temperatur des Katalysatorfestbetts den (Zahlen) Mittelwert der Temperatur entlang des Katalysatorfestbetts. Selbstverständlich kann die Temperatur des Katalysatorfestbetts über seine Länge auch so gestaltet sein, dass die Temperatur über einen gewissen Längenabschnitt konstant ist, sich dann sprunghaft ändert und über einen weiteren Längenabschnitt auf diesem neuen Wert verharrt u.s.w.. Man spricht dann von einem mehr als eine Temperaturzone (oder auch Reaktionszone) aufweisenden oder sich in mehr als einer Temperaturzone (oder auch Reaktionszone) befindlichen Katalysatorfestbett (Festbettkatalysatorschüttung). Derartige Temperatur-zonen (oder auch Reaktionszonen) realisierende, mit Katalysator beschickte Reaktoren werden in entsprechender Weise als "Ein-" oder "Mehr-Zonen-Reaktoren" bezeichnet (vgl. z. B. WO 04/085369). Mit der Temperatur des Reaktionsgasgemischs durchläuft die effektive Temperatur des Katalysatorfestbetts in Strömungsrichtung des Reaktionsgasgemischs gleichfalls den Heißpunktwert.

Ein Ziel der heterogen katalysierten partiellen Gasphasenoxidationen von Propylen zu Acrolein besteht nun darin, die Heißpunkttemperatur und mit ihr ihre Sensitivität gegenüber einer Erhöhung der Temperatur des Katalysatorfestbetts möglichst günstig zu gestalten. So mindern zu hohe Heißpunkttemperaturen in der Regel sowohl die Lebensdauer des Katalysatorfestbetts, als auch die Selektivität der Zielproduktbildung (hier: Acrolein). Mit niederen Heißpunkttemperaturen geht normalerweise gleichzeitig eine Minderung der Sensitivität derselben gegenüber einer Erhöhung der Temperatur des Katalysatorfestbetts einher. Dies erweist sich z. B. bei einer Durchführung der Partialoxidation in Rohrbündelreaktoren als günstig, wenn die einzelnen Kontaktrohre infolge von über den Reaktorquerschnitt bestehenden Temperaturgradienten unterschiedliche Temperaturen des im jeweiligen Kontaktrohr befindlichen Katalysatorfestbetts bedingen. Ein Beisein von Propan im Reaktionsgasgemisch begünstigt infolge der vergleichsweise erhöhten spezifischen Wärme des Propans niedere Heißpunkttemperaturen (vgl. z. B. EP-A 293 224). Weiterhin begünstigt ein Beisein von Propan aufgrund seiner Brennbarkeit das Explosionsverhalten des Reaktionsgasgemischs (z. B. DE-A 195 08 558).

Auf der anderen Seite ist bekannt, dass ein Beisein von Propan im Reaktionsgasgemisch die unerwünschte Bildung von Propionaldehyd und/oder Propionsäure in unerwünschter Weise begünstigt (vgl. z. B. WO 01/96270). Die WO 01/96270 empfiehlt daher, das Reaktionsgasgemisch mit molekularem Stickstoff zu verdünnen und, als Sauerstoffquelle beispielsweise Luft zu verwenden. Ein Vorteil dieser Verfahrensweise wäre gleichzeitig die Nutzung einer vergleichsweise ökonomischen Sauerstoffquelle. Eine weitere Zielsetzung der Verfahren im Stand der Technik besteht darin, als Propenquelle eine heterogen katalysierte Oxidehydrierung und/oder Dehydrierung von Propan zu Propylen einsetzen zu können und dabei auf eine nachfolgende Abtrennung des gebildeten Propylen vom nicht umgesetzten Propan zu verzichten, um so über eine wirtschaftliche Propylenquelle zu verfügen (vgl. z. B. WO 03/011804. DE-A 198 37 517, DE-A 198 37 519, DE-A 198 37 520, WO 01/96370, DE-A 102 45 585 und WO 03/76370).

In der WO 01/96270 wird diesbezüglich eine heterogen katalysierte Propandehydrierung mit vergleichsweise niederen Propanumsätzen empfohlen.

Auf der anderen Seite fordern z. B. sowohl die EP-A 990 636 als auch die EP-A 10 70 700 möglichst hohe Propengehalte im Reaktionsgasausgangsgemisch einer Propenpartialoxidation zu Acrolein, um möglichst hohe Raum-Zeit-Ausbeuten am Zielprodukt erzielen zu können. Das Reaktionsgasausgangsgemisch der WO 00/53556 weist kein Propan auf und enthält zwischen 77,4 und 81,6 Vol.-% $N_2$.

Hohe Propanumsätze begünstigen nun zwar die Möglichkeit einer Einstellung von erhöhten Propylengehalten im Reaktionsgasausgangsgemisch der Propylenpartialoxidation. Sie sind jedoch insofern nachteilig, als einerseits der verbleibende Propananteil gemindert wird, was sich ungünstig auf die Heißpunktbildung und das Explosionsverhalten auswirkt und gleichzeitig nimmt mit hohen Propanumsätzen die mit der Dehydrierung bzw. Oxydehydrierung einhergehende Nebenkomponentenbildung (z.B. $H_2$ oder $H_2O$) zu. Verwendet man das resultierende Dehydrier- und/oder Oxydehydriergemisch in diesem Fall als solches für die Erzeugung des Reaktionsgasausgangsgemischs der nachfolgenden Propylenpartialoxidation, wie es z. B. die EP-A 117 146, die DE-A 33 13 573 und die US-A 3,161,670 empfehlen, resultiert ein vergleichsweise voluminöses Reaktionsgasausgangsgemisch. Unter dem Aspekt einer möglichst wirtschaftlichen Förderung der Reaktionsgasströme sind jedoch kleine Volumina vorteilhaft. Insbesondere dann, wenn hohe Belastungen der Katalysatorbeschickung gemäß dem in den Schriften WO 04/85365, WO 04/85367, WO 04/85369, WO 04/85370, WO 04/85363, WO 00/53559, WO 04/85362, WO 00/53557 und DE-A 199 48 248 niedergelegten Prinzip verwirklicht werden sollen.

Andererseits sind hohe Dehydrier- bzw. Oxidehydrierumsätze im Sinne einer möglichst wirtschaftlichen Propylenerzeugung günstig. Umgekehrt erfordert ein erhöhter Propengehalt im Reaktionsgasausgangsgemisch ein erhöhtes molares Verhältnis von molekularem Sauerstoff zu Propylen in selbigem, um die Aktivität der Katalysatorbeschickung optimal zur Entfaltung kommen zu lassen.

Gleichzeitig erweist sich ein zu hoher Restsauerstoffgehalt im Produktgasgemisch der Partialoxidation insofern als nachteilig, als er bei einer vorteilhaften Rückführung des nach Abtrennung des Zielprodukts aus diesem Produktgasgemisch verbleibenden, nicht umgesetztes Propan enthaltenden, Restgases in die zur Propylenerzeugung angewandte heterogen katalysierte Propandehydrierung das zu dehydrierende Propan in unerwünschter Weise angreift und die Selektivität der Propenbildung mindert. Generell wird eine heterogen katalysierte Propandehydrierung gegenüber einer heterogen katalysierten Propanoxidehydrierung bevorzugt.

Im Unterschied zur exotherm verlaufenden heterogen katalysierten Oxidehydrierung, die durch anwesenden Sauerstoff erzwungen und bei der intermediär kein freier Wasserstoff gebildet wird (der dem zu dehydrierenden Propan entrissene Wasserstoff wird unmittelbar als Wasser ($H_2O$) entrissen) bzw. nachweisbar ist, soll unter einer heterogen katalysierten Dehydrierung eine ("konventionelle") Dehydrierung verstanden werden, deren Wärmetönung im Unterschied zur Oxidehydrierung endotherm ist (als Folgeschritt kann eine exotherme Wasserstoffverbrennung in die heterogen katalysierte Dehydrierung einbezogen sein) und bei der wenigstens intermediär freier molekularer Wasserstoff gebildet wird. Dazu bedarf es in der Regel anderer Reaktionsbedingungen und anderer Katalysatoren als zur Oxidehydrierung.

Unter Frischpropan wird in dieser Schrift Propan verstanden, das noch an keiner chemischen Reaktion teilgenommen hat. In der Regel wird es Roh-Propan (das vorzugsweise die Spezifikation gemäß DE-A 102 46 119 und DE-A 102 45 585 erfüllt) sein, das in geringen Mengen auch von Propan verschiedene Komponenten enthält.

Das Reaktionsgasausgangsgemisch für die Propenpartialoxidation zu Acrolein erfüllt in dieser Schrift in zweckmäßiger Weise gleichfalls die in der DE-A 102 46 119 und DE-A 102 45 585 empfohlenen Spezifikationen.

Unter der Belastung eines einen Reaktionsschritt katalysierenden Katalysatorbetts mit Reaktionsgas(ausgangs)gemisch wird in dieser Schrift die Menge an Reaktionsgas(ausgangs)gemisch in Normlitern (=Nl; das Volumen in Litern, das die entsprechende Reaktionsgas(ausgangs)gemischmenge bei Normalbedingungen (0°C, 1 bar) einnehmen würde) verstanden, die pro Stunde durch einen Liter Katalysatorfestbett geführt wird.
Die Belastung kann auch nur auf einen Bestandteil des Reaktionsgas(ausgangs)gemischs bezogen sein. Dann ist es die Menge dieses Bestandteils in Nl/l·h, die pro Stunde durch einen Liter des Katalysatorfestbetts geführt wird (reine Inertmaterialschüttungen werden nicht zum Katalysatorfestbett gerechnet).

Als ein Inertgas soll in dieser Schrift ein Reaktionsgasbestandteil verstanden werden, der sich unter den Bedingungen der entsprechenden Reaktion im wesentlichen als inert verhält und - jeder inerte Reaktionsgasbestandteil für sich betrachtet - zu mehr als 95 mol-%, vorzugsweise zu mehr als 99 mol% chemisch unverändert erhalten bleibt.

Nachteilig an den vorstehend zusammengestellten Empfehlungen des Standes der Technik für eine heterogen katalysierte Partialoxidation von Propen zu Acrolein ist, dass sie jeweils nur Einzelaspekte beleuchten. Die Aufgabe der vorliegenden Erfindung bestand daher darin, ein verbessertes Verfahren der heterogen katalysierten Partialoxidation von Propen zu Acrolein zur Verfügung zu stellen, das den verschiedenen im Stand der Technik angeführten Einzelaspekten in ihrer Gesamtheit in optimierender Weise Rechnung trägt.

Demgemäß wurde ein Verfahren zur Herstellung von Acrolein oder Acrylsäure oder deren Gemisch durch heterogen katalysierte partielle Gasphasenoxidation von Propen, bei dem man ein die Reaktanden Propylen und molekularer Sauerstoff sowie die inerten Verdünnungsgase molekularer Stickstoff und Propan enthaltendes Reaktionsgasausgangsgemisch 2, das den molekularen Sauerstoff und das Propylen in einem molaren Verhältnis $O_2 : C_3H_6 \geq 1$ enthält, bei erhöhter Temperatur durch ein Katalysatorfestbett (durch eine Festbettkatalysatorschüttung) führt, dessen Aktivmasse wenigstens ein die Elemente Mo, Fe und Bi enthaltendes Multimetalloxid ist, gefunden, das dadurch gekennzeichnet ist, dass das Reaktionsgasausgangsgemisch 2, bezogen auf sein Gesamtvolumen, die nachfolgenden Gehalte

| | |
|---|---|
| 6 bis 9 Vol.-% | Propylen, |
| 8 bis 18 Vol.-% | molekularer Sauerstoff, |
| 6 bis 30 Vol.-% | Propan und |
| 32 bis 72 Vol.-% | molekularer Stickstoff |

mit der Maßgabe aufweist, dass das molare Verhältnis $V_1$ von im Reaktionsgasausgangsemisch 2 enthaltenem Propan zu im Reaktionsgasausgangsgemisch 2 enthaltenem Propylen 1 bis 4, das molare Verhältnis $V_2$ von im Reaktionsgasausgangsgemisch 2 enthaltenem molekularem Stickstoff zu im Reaktionsgasausgangsgemisch 2 enthaltenem molekularem Sauerstoff 2 bis 6 und das molare Verhältnis $V_3$ von im Reaktionsgasausgangsgemisch 2 enthaltenem molekularem Sauerstoff zu im Reaktionsgasausgangsgemisch 2 enthaltenem Propylen 1,3 bis 2,4 beträgt.

Das erfindungsgemäße Verfahren unterscheidet sich von den Verfahren des Standes der Technik vor allem durch die Forderung $V_1 = 1$ bis 4. In allen Ausführungsbeispielen des Standes der Technik beträgt $V_1 \geq 4,5$. Letzteres wirkt sich nachteilig auf die Selektivität der Nebenproduktbildung an Propionaldehyd und Propionsäure aus und beschränkt den möglichen Umsatz einer heterogen katalysierten Propandehydrierung als Propylenquelle, ohne mit Blick auf das Explosionsverhalten unabdingbar zu sein.

Erfindungsgemäß bevorzugt gilt für die Gehalte des Reaktionsgasausgangsgemischs 2

| | |
|---|---|
| 7 bis 9 Vol.% | Propylen, |
| 9,8 bis 16 Vol.-% | molekularer Sauerstoff, |
| 9 bis 25 Vol.% | Propan und |
| 35 bis 65 Vol.% | molekularer Stickstoff, |

mit der Maßgabe, dass

$V_1 = 1$ bis 3,5
$V_2 = 3$ bis 4,5 und
$V_3 = 1,4$ bis 2,2

beträgt.

Erfindungsgemäß besonders bevorzugt gilt für die Gehalte das Reaktionsgasausgangsgemischs 2

| | |
|---|---|
| 7 bis 9 Vol.-% | Propylen, |
| 9,8 bis 15 Vol.% | molekularer Sauerstoff, |
| 10,5 bis 20 Vol.-% | Propan und |
| 40 bis 60 Vol.% | molekularer Stickstoff, |

mit der Maßgabe, dass

$V_1 = 1,5$ bis 2,5
$V_2 = 3,5$ bis 4,5 (vorzugsweise 3,5 bis 4)

$V_3$ = 1,4 bis 2,14 (vorzugsweise 1,5 bis 2,0)

beträgt.

Erfindungsgemäß ganz besonders bevorzugt gilt für die Gehalte des Reaktionsgasausgangsgemischs 2

| | |
|---|---|
| 7 bis 9 Vol.-% | Propylen, |
| 9,8 bis 15,5 Vol.% | molekularer Sauerstoff, |
| 10, 5 bis 15,5 Vol.-% | Propan und |
| 40 bis 60 Vol.-% | molekularer Stickstoff |

mit der Maßgabe, dass

$V_1$ = 1,5 bis 2,2
$V_2$ = 3,5 bis 4,5 (vorzugsweise 3,5 bis 4)
$V_3$ = 1,4 bis 2,14 (vorzugsweise 1,5 bis 2,0)

beträgt.

Bei der erfindungsgemäß bevorzugtesten Ausgestaltung des erfindungsgemäßen Verfahrens gilt für die Gehalte des Reaktionsgasausgangsgemischs 2

| | |
|---|---|
| 7 bis 8 Vol.% | Propylen, |
| 11,9 bis 15,5 Vol.-% | molekularer Sauerstoff, |
| 11,9 bis 15,5 Vol.% | Propan und |
| 50 bis 60 Vol.-% | molekularer Stickstoff, |

mit der Maßgabe, dass

$V_1$ = 1,7 bis 2,1,
$V_2$ = 3,5 bis 4,5 (vorzugsweise 3,5 bis 4)
$V_3$ = 1,7 bis 2,1 (vorzugsweise 1,8 bis 2,0)

beträgt.

Bei einer alternativen Ausführungsform des erfindungsgemäßen Verfahrens gilt für die Gehalte des Reaktionsgasausgangsgemischs 2

| | |
|---|---|
| 7 bis 9 Vol.-% | Propylen, |
| 9,8 bis 15 Vol.% | molekularer Sauerstoff, |
| 21 bis 28 Vol.% | Propan und |
| 40 bis 60 Vol.% | molekularer Stickstoff, |

mit der Maßgabe, dass

$V_1$ = 3 bis 4,
$V_2$ = 3,5 bis 4,5 (vorzugsweise 3,5 bis 4)
$V_3$ = 1,4 bis 2,14 (vorzugsweise 1,5 bis 2,0)

beträgt.

Ganz generell ist es für alle genannten erfindungsgemäßen Reaktionsgasausgangsgemische 2 günstig, wenn ihr Gesamtgehalt an von Propylen, molekularem Sauerstoff, Propan und molekularem Stickstoff verschiedenen Bestandteilen ≤ 10 Vol.-% beträgt. Von diesen bis zu 10 Vol.-% sonstigen Bestandteilen können bis zu 8 Vol.% Ethan und/oder Methan (aber auch sonst kann dieser Gehalt an Ethan und Methan vorliegen) sein. In der Regel liegt der Gesamtgehalt erfindungsgemäßer Reaktionsgasausgangsgemische 2 an Ethan und/oder Methan jedoch ≤ 5 Vol.%, meist ≤ 3 Vol.%, und vielfach ≤ 2 Vol.-%. Solche Gehalte von ≥ 0,5 Vol.-% sind infolge ihres weitgehend inerten Verhaltens erfindungsgemäß jedoch durchaus möglich und aufgrund der günstigen Wärmeleitfähigkeit des Methan und Ethan erfindungsgemäß vorteilhaft. Erfindungsgemäß vorteilhaft umfassen alle genannten erfindungsgemäßen Reaktionsgas-

ausgangsgemische $2 \leq 5$ Vol.-% Wasser und $\leq 5$ Vol.-% Kohlenoxide ($CO_2$ und/oder CO). Besonders vorteilhaft umfassen alle genannten erfindungsgemäßen Reaktionsgasausgangsgemische $2 \leq 3$ Vol.-% Wasser und $\leq 3$ Vol.-% Kohlenoxide. Die gleichen Gehaltsgrenzen gelten vorzugsweise für molekularen Wasserstoff, dessen Gehalt besonders vorteilhaft verschwindend ist. Ganz besonders vorteilhaft umfassen alle genannten erfindungsgemäßen Reaktionsgasausgangs- gemische $2 \leq 2$ Vol.-% Wasser und $\leq 2$ Vol.-% Kohlenoxide. Günstig sind in der Regel Wassergehalte $\leq 0,5$ Vol.-% im Reaktionsgasausgangsgemisch 2.

Ferner ist erfindungsgemäß günstig, wenn der Gesamtgehalt aller vorgenannten erfindungsgemäßen Reaktionsgasausgangsgemische 2 an von Propylen, molekularem Sauerstoff, Propan und molekularem Stickstoff verschie- denen Bestandteilen $\leq 5$ Vol.-%, besonders vorteilhaft $\leq 3$ Vol.% beträgt. Erfindungsgemäß besonders günstig beträgt $V_2$ für alle vorgenannten erfindungsgemäßen Reaktionsgasausgangsgemische im wesentlichen 3,73. Die bei den aufgeführten erfindungsgemäßen Reaktionsgasausgangsgemischen genannten Vorzugsbereiche $V_2$ und $V_3$ gelten unab- hängig voneinander.

Vorteilhaft für das erfindungsgemäße Verfahren ist, dass als Propenquelle das in Verfahren der kontinuierlichen heterogen katalysierten partiellen Dehydrierung und/oder Oxidehydrierung von Propan in der Gasphase gebildete Propylen in Betracht kommt, ohne dass zuvor von diesem Propylen das in der heterogen katalysierten partiellen Dehydrierung und/oder Oxidehydrierung nicht umgesetzte Propan abgetrennt werden muß.

Prinzipiell kommen dazu alle bekannten heterogen katalysierten partiellen Dehydrierungen des Propans in Betracht, wie sie z. B. aus den Schriften WO 03/076370, WO 01/96271, EP-A 117 146, WO 03/011804, US 3,161,670, WO 01/96270, DE-A 33 13 573, DE-A 102 45 585, DE-A 103 16 039 sowie aus der deutschen Anmeldung DE-A 10 2004 032 129 vorbekannt sind.

Ebenso kommen alle im Stand der Technik dafür bekannten Dehydrierkatalysatoren in Betracht. Mit Vorteil wird man im Katalysatorfestbett dehydrieren.

Die Dehydrierkatalysatoren lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit sowohl alle Dehydrierkatalysatoren eingesetzt werden, die in der DE-A 102 19 879, WO 01/96270, der EP-A 731077, der DE-A 10211275, der DE-A 10131297, der WO 99/46039, der US-A 4 788 371, der EP- A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden, als auch der Katalysator gemäß Beispiel 4 der DE-A 102 19 879. Im besonderen können sowohl der Katalysator gemäß Beispiel 1, Beispiel 2, Beispiel 3, und Beispiel 4 der DE-A 199 37 107 als auch der Katalysator gemäß Beispiel 4 der DE-A 102 19 879 sowie die Katalysatoren der WO 02/51547, WO 02/51540 und der DE-A 102005002127 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der in den Beispielen und Vergleichsbeispielen dieser Schrift eingesetzte Dehydrierkatalysator.

Generell kann es sich bei den Dehydrierkatalysatoren um Katalysatorstränge (Durchmesser typisch 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln.

In der Regel sind die Dehydrierkatalysatoren (insbesondere die in dieser Schrift beispielhaft verwendeten sowie die in der DE-A 19937107 empfohlenen (insbesondere die beispielhaften Katalysatoren dieser DE-A) so beschaffen, dass sie sowohl die Dehydrierung von Propan als auch die Verbrennung von molekularem Wasserstoff zu katalysieren vermögen. Die Wasserstoffverbrennung verläuft dabei im Vergleich zur Dehydrierung des Propans im Fall einer Konkurrenzsituation an den Katalysatoren sehr viel schneller.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierungen und Dehydrierkatalysatoren zitierten Schriften des Standes der Technik.

Charakteristisch für die partielle heterogen katalysierte Dehydrierung von Propan ist, dass sie endotherm verläuft. Das heißt, die für die Einstellung der erforderlichen Reaktionstemperatur sowie die für die Reaktion benötigte Wärme (Energie) muss entweder dem Reaktionsgasausgangsgemisch (das Reaktionsgas(ausgangs)gemisch für die heterogen katalysierte Propandehydrierung soll in dieser Schrift als Reaktionsgas(ausgangs)gemisch 1 bezeichnet werden) vorab und/oder im Verlauf der heterogen katalysierten Dehydrierung zugeführt werden. Gegebenenfalls muss das Reaktionsgasgemisch die benötigte Reaktionswärme sich selbst entziehen.

Ferner ist es für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende, Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit (bei Bedarf täglich) bei erhöhter Temperatur mit einem Sauerstoff enthaltenden Gas (zweckmäßig in Abwesenheit von Kohlenwasserstoffen) zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan ein Gemisch aus Wasserdampf und molekularem Wasserstoff zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten. Teiloxidation von solchermaßen zugesetztem Wasserstoff vermag gleichfalls benötigte Reaktionswärme zu liefern.

Erfindungsgemäß wesentlich ist, dass als Propenquelle auch heterogen katalysierte partielle Propandehydrierungen in Betracht kommen, bei denen der Propanumsatz 20 bis 30 mol.% beträgt (auf den einmaligen Durchgang von Frischpropan durch die Dehydrierung bezogen). Besonders günstig für das erfindungsgemäße Verfahren sind als Propenquelle jedoch heterogen katalysierte partielle Propandehydrierungen, bei denen der vorgenannte Propanumsatz 30 bis 60 mol.-%, vorzugsweise 35 bis 55 mol.-% und besonders bevorzugt 35 bis 45 mol.% beträgt.

Für die Realisierung der vorgenannten Propanumsätze ist es günstig, die heterogen katalysierte Propandehydrierung bei einem Arbeitsdruck von 0,3 bis 10 bar, bzw. vorteilhaft bis 3 bar durchzuführen. Ferner ist es günstig, das heterogen katalytisch zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit von Edelmetall enthaltenden Dehydrierkatalysatoren aus, insbesondere im Fall von angestrebten hohen Propanumsätzen. Bei Bedarf kann als weiterer Bestandteil auch molekularer Wasserstoff zugegeben werden. Das molare Verhältnis von molekularem Wasserstoff zu Propan ist dabei im Reaktionsgasausgangsgemisch 1 in der Regel ≤ 5. Das molare Verhältnis von Wasserdampf zu Propan wird im Reaktionsgasausgangsgemisch 1 zweckmäßig ≥ 0,05 bis 2 bzw. bis 1 betragen.

Generell werden möglichst kleine Wasserdampfgehalte im Reaktionsgasausgangsgemisch 1 bevorzugt und angestrebt. Bei auf Frischpropan bezogenen Propanumsätzen im Bereich von 20 bis 30 mol.-% ist üblicherweise die in gegebenenfalls ins Reaktionsgasausgangsgemisch 1 rückgeführtem Oxidationskreisgas enthaltene Wasserdampfmenge als Wasserdampfversorgung für die heterogen katalysierte Dehydrierung ausreichend. Für höhere auf Frischpropan bezogene Propanumsätze wird normalerweise darüber hinaus Wasserdampf zugegeben, bei welchem es sich z. B. um abgeschiedenes Prozesswasser handeln kann. Als Oxidationskreisgas (vgl. z. B. EP-A 11 80 508 und die deutsche Anmeldung DE-A 10 2004 032 129) wird üblicherweise das Restgas bezeichnet, das nach einer einstufigen oder mehrstufigen heterogen katalysierten Gasphasenpartialoxidation wenigstens einer organischen Verbindung (hier: Propylen und/oder Acrolein) dann verbleibt, wenn man aus dem Produktgasgemisch der Partialoxidation das Zielprodukt mehr oder weniger selektiv (z. B. durch Absorption in ein geeignetes Lösungsmittel oder durch fraktionierende Kondensation) abgetrennt hat. Im Regelfall besteht es überwiegend aus den für die Partialoxidation verwendeten inerten Verdünnungsgasen sowie aus bei der Partialoxidation üblicherweise als Nebenprodukt gebildetem (oder als Verdünnungsgas zugesetztem, was erfindungsgemäß weniger bevorzugt ist) Wasserdampf und durch unerwünschte vollständige Oxidation gebildeten Kohlenoxiden.

Außerdem enthält es normalerweise noch Restmengen an bei der Partialoxidation nicht verbrauchtem Sauerstoff und an nicht umgesetzten organischen Ausgangsverbindungen (hier: Propylen, Acrolein) und geringste Zielproduktmengen.

Bei der erfindungsgemäßen Partialoxidation enthält das Oxidationskreisgas verbliebenes Propan und wird deshalb vorteilhaft in die zweckmäßigerweise als Propylenquelle dienende heterogen katalysierte partielle Propandehydrierung rückgeführt.

Grundsätzlich kann eine als Propylenquelle fungierende heterogen katalysierte partielle Propandehydrierung (quasi) adiabat und dabei endotherm durchgeführt werden. Dabei wird das Reaktionsgasausgangsgemisch in der Regel zunächst auf eine Temperatur von 500 bis 700°C (beziehungsweise von 550 bis 650°C) erhitzt (zum Beispiel durch Direktbefeuerung der es umgebenden Wandung). Beim adiabaten Durchgang durch das wenigstens eine Katalysatorbett wird sich das Reaktionsgasgemisch dann je nach Umsatz und Verdünnung um etwa 30°C bis 200°C abkühlen. Ein Beisein von Wasserdampf als Wärmeträger macht sich auch unter dem Gesichtspunkt einer adiabaten Fahrweise vorteilhaft bemerkbar. Niedrigere Reaktionstemperaturen ermöglichen längere Standzeiten des verwendeten Katalysatorbetts. Höhere Reaktionstemperaturen fördern erhöhte Umsätze.

Anwendungstechnisch zweckmäßig wird man eine heterogen katalysierte Propandehydrierung als Propylenquelle für das erfindungsgemäße Verfahren als Hordenreaktor verwirklichen.

Dieser enthält räumlich aufeinanderfolgend zweckmäßig mehr als ein die Dehydrierung katalysierendes Katalysatorbett. Die Katalysatorbettenanzahl kann 1 bis 20, zweckmäßig 2 bis 8, aber auch 3 bis 6 betragen. Mit zunehmender Hordenzahl lassen sich zunehmend leichter erhöhte Propanumsätze erreichen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber oder darunter liegende Segment zu führen.

In zweckmäßiger Weise wird das Reaktionsgasgemisch 1 auf seinem Weg von einem Katalysatorbett zum nächsten Katalysatorbett, zum Beispiel durch Überleiten über mit heißen Gasen erhitzte Wärmetauscheroberflächen (z. B. Rippen) oder durch Leiten durch mit heißen Brenngasen erhitzte Rohre, im Hordenreaktor einer Zwischenerhitzung unterworfen.

Wird der Hordenreaktor im übrigen adiabat betrieben, ist es für auf Frischpropan bezogene Propanumsätze ≤ 30 mol-%, vor allem bei Verwendung der in der DE-A 199 37 107 beschriebenen Katalysatoren, insbesondere der beispielhaften Ausführungsformen, ausreichend, das Reaktionsgasgemisch 1 auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist. Für höhere Propanumsätze wird das Reaktionsgasgemisch 1 zweckmäßig auf höhere Temperaturen vorerhitzt in den Dehydrierreaktor geführt (diese können bis zu 700°C betragen) und innerhalb des Hordenreaktors in diesem erhöhten Temperaturbereich gehalten.

Noch geschickter ist es, die vorstehend geschilderte Zwischenerhitzung auf direktem Weg durchzuführen (autotherme Fahrweise). Dazu wird dem Reaktionsgasgemisch 1 entweder bereits vor Durchströmung des ersten Katalysatorbettes (dann sollte das Reaktionsgasausgangsgemisch 1 molekularen Wasserstoff zugesetzt enthalten) und/oder zwischen den nachfolgenden Katalysatorbetten in begrenztem Umfang molekularer Sauerstoff zugesetzt. So kann (in der Regel durch die Dehydrierkatalysatoren selbst katalysiert) eine begrenzte Verbrennung von im Reaktionsgasgemisch 1 enthaltenem, im Verlauf der heterogen katalysierten Propandehydrierung gebildetem und/oder dem Reaktionsgasgemisch 1 zugesetztem molekularem Wasserstoff (gegebenenfalls begleitet von einer in geringem Umfang erfolgenden Propanverbrennung) bewirkt werden (es kann auch anwendungstechnisch zweckmäßig sein, im Hordenreaktor Katalysatorbetten einzufügen, die mit Katalysator beschickt sind, der besonders spezifisch (selektiv) die Verbrennung von Wasserstoff katalysiert (als solche Katalysatoren kommen zum Beispiel jene der Schriften US 4,788,371, US 4,886,928, US 5,430,209, US 5,530,171, US 5,527,979 und US 5,563,314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme (die Bruttowärmetönung ist im wesentlichen Null) Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht (im Extremfall kann zur Gewährleistung der Autothermie auch nur Propan verbrannt werden).

Generell sollte erfindungsgemäß eine wie vorstehend beschriebene Sauerstoffeinspeisung so vorgenommen werden, dass der Sauerstoffgehalt des Reaktionsgasgemisches 1, bezogen auf die darin enthaltene Menge an molekularem Wasserstoff, 0,5 bis 50 bzw. bis 30, vorzugsweise 10 bis 25 Vol.-% beträgt. Als Sauerstoffquelle kommen dabei sowohl reiner molekularer Sauerstoff oder mit Inertgas, zum Beispiel CO, $CO_2$, $N_2$ und/oder Edelgase, verdünnter Sauerstoff, insbesondere aber auch Luft (abgesehen von Oxidationskreisgas wird bevorzugt ausschließlich Luft als Sauerstoffquelle verwendet), in Betracht. Die resultierenden Verbrennungsgase wirken in der Regel zusätzlich verdünnend und fördern dadurch die heterogen katalysierte Propandehydrierung. Dies gilt insbesondere für den im Rahmen der Verbrennung gebildeten Wasserdampf.

Die Isothermie der für das erfindungsgemäße Verfahren in zweckmäßiger Weise als Propylenquelle fungierenden heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige) anbringt. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Selbstredend lässt sich die heterogen katalysierte Propandehydrierung auch wie in der DE-A 102 11 275 beschrieben (als "Schlaufenvariante") realisierten, die einen integralen Bestandteil dieser Patentanmeldung bildet.

D. h., vorteilhaft für das erfindungsgemäße Verfahren ist, dass als Propenquelle ein Verfahren der kontinuierlichen heterogen katalysierten partiellen Dehydrierung von Propan in der Gasphase fungieren kann, bei dem

- einer Dehydrierzone ein das zu dehydrierende Propan enthaltende Reaktionsgasausgangsgemisch 1 kontinuierlich zugeführt wird,
- das Reaktionsgasausgangsgemisch 1 in der Dehydrierzone durch wenigstens ein Katalysatorfestbett geführt wird, an welchem durch katalytische Dehydrierung molekularer Wasserstoff und (partiell) Propylen gebildet werden,
- dem Reaktionsgasausgangsgemisch 1 vor und/oder nach Eintritt in die Dehydrierzone wenigstens ein molekularen Sauerstoff enthaltendes Gas zugesetzt wird,
- der molekulare Sauerstoff in der Dehydrierzone im Reaktionsgasgemisch 1 enthaltenen molekularen Wasserstoff teilweise zu Wasserdampf oxidiert und
- der Dehydrierzone ein Produktgas entnommen wird, das molekularen Wasserstoff, Wasserdampf, Propylen und nicht umgesetztes Propan enthält und das dadurch gekennzeichnet ist, dass das der Dehydrierzone entnommene Produktgas in zwei Teilmengen identischer Zusammensetzung aufgeteilt und eine der beiden Teilmengen als Dehydrierkreisgas (vorzugsweise als Bestandteil des Reaktionsgasausgangsgemischs 1) in die Dehydrierzone zurückgeführt wird, wie es auch die WO 03/076370 empfiehlt.

Dabei kann das Oxidationskreisgas Bestandteil des Reaktionsgasausgangsgemisch 1 sein und/oder gemäß der Lehre der deutschen Anmeldung DE-A 10 2004 032 129 erst nach bereits teilweise erfolgter Dehydrierung dem Reaktionsgasgemisch 1 zugeführt werden.

Ist das Oxidationskreisgas Bestandteil des Reaktionsgasausgangsgemischs 1, enthält dieses zweckmäßig lediglich dem Oxidationskreisgas entstammenden molekularen Sauerstoff.

Für das erfindungsgemäße Verfahren ist es im Rahmen der beschriebenen Schlaufenfahrweise günstig, wenn die Menge des Dehydrierkreisgases, bezogen auf das in der Dehydrierzone gebildete Produktgas, 30 bis 70 Vol.-%, vorteilhaft 40 bis 60 Vol.-%, vorzugsweise 50 Vol.-% beträgt.

Mit Bezug auf die erfindungsgemäße Partialoxidation enthält das Reaktionsgasausgangsgemisch 1 für den Fall einer wie beschrieben z. B. im Hordenreaktor durchgeführten Schlaufenfahrweise (Hordenschlaufenreaktor dann = Dehydrierzone) im stationären Zustand in zweckmäßiger Weise:

| | |
|---|---|
| 15 bis 25 Vol.-% | Propan, |
| 2 bis 6 Vol.-% | Propylen, |
| 5 bis 20 Vol% | Wasserdampf, |
| 2 bis 10 Vol.-% | molekularer Wasserstoff, |
| 40 bis 75 Vol.-% | molekularer Stickstoff, und |
| > 0 bis 3 Vol.-% | molekularer Sauerstoff. |

Der Umsatz an Propan (bezogen auf den einmaligen Durchgang des vorgenannten Reaktionsgasausgangsgemisch 1 durch den in Schlaufenfahrweise betriebenen Hordenreaktor) und das Schlaufenkreisgasverhältnis (Menge des Dehydrierkreisgases bezogen auf die in der Dehydrierzone insgesamt anfallende Produktgasmenge) werden mit Blick auf die erfindungsgemäße heterogen katalysierte Gasphasenpartialoxidation des Propylens mit Vorteil (z. B. im Hordenschlaufenreaktor als Dehydrierzone) so gewählt, dass das in der Dehydrierzone gebildete Produktgas nicht umgesetztes Propan und gewünschtes Propylen im molaren Verhältnis Propen zu Propan von 0,25 bzw. 0,3 bis 0,5 (gegebenenfalls bis 0,66) enthält. Bei einem Schlaufenkreisgasverhältnis von 0,5 entspricht dem ein auf den einmaligen Durchgang des Reaktionsgasausgangsgemisch 1 durch die Dehydrierzone bezogener Umsatz des darin enthaltenen Propan von 15 bis 25 mol-%.

Typische Belastungen der Dehydrierkatalysatorbetten mit Reaktionsgasgemisch 1 betragen 250 bis 5000 h$^{-1}$ (bei Hochlastfahrweise auch bis 40000 h$^{-1}$), vorzugsweise 10000 bis 25000 Nl/l·h, besonders bevorzugt 15000 bis 20000 Nl/l·h. Die entsprechenden Belastungen mit Propan liegen typisch bei 50 bis 1000 h$^{-1}$ (bei Hochlastfahrweise auch bis 40000 h$^{-1}$), vorzugsweise bei 2000 bis 5000 Nl/l·h, besonders bevorzugt 3000 bis 4000 Nl/l·h.

Das der Dehydrierzone (dem Dehydrierreaktor) als Propylenquelle entnommene Dehydrierproduktgas befindet sich entsprechend den für die heterogen katalysierte Propandehydrierung gewählten Reaktionsbedingungen bei einem Druck von 0,3 bis 10 bar, vorzugsweise 1 bis 3 bar, und weist häufig eine Temperatur von 450 bis 650°C, vielfach eine Temperatur von 500 bis 600°C auf. In der Regel enthält es Propan, Propen, $H_2$, $N_2$, $H_2O$, Methan, Ethan (die letzteren beiden resultieren meist infolge thermischen Zerfalls einer geringen Propanmenge), Ethylen, Buten-1, sonstige Butene wie iso-Buten, andere $C_4$-Kohlenwasserstoffe wie n-Butan, iso-Butan, Butadien etc., CO und $CO_2$, im Regelfall aber auch Oxygenate wie Alkohole, Aldehyde und Carbonsäuren (normalerweise mit ≤ 9 C-Atomen). Weiterhin können in geringen Mengen aus dem Oxidationskreisgas herrührende Bestandteile enthalten sein.

Während die EP-A 117 146, die DE-A 33 13 573 und die US-A 3,161,670 empfehlen, das in der Propandehydrierung gebildete Produktgas als solches zur Beschickung der erfindungsgemäßen Partialoxidation zu verwenden, ist es für eine erfindungsgemäße Partialoxidation vorteilhaft, aus dem das Propylen enthaltenden Produktgas der Propandehydrierung vor seiner Verwendung als Propenquelle für die erfindungsgemäße Propenpartialoxidation wenigstens eine Teilmenge der darin enthaltenen, von Propan und Propylen verschiedenen, Bestandteile abzutrennen. Dabei sollten die Erfordernisse der DE-A 102 11 275 beachtet werden.

Erfindungsgemäß vorteilhaft wird man wenigstens 50 Vol.-%, vorzugsweise wenigstens 75 Vol.-%, besonders bevorzugt wenigstens 90 Vol.-% und ganz besonders bevorzugt wenigstens 95 Vol.-% der im Produktgas der Propandehydrierung enthaltenen, von Propan und Propylen verschiedenen, Bestandteile abtrennen, bevor selbiges als Propenquelle für die erfindungsgemäße Partialoxidation eingesetzt wird.

Eine für die erfindungsgemäßen Bedürfnisse zweckmäßige Möglichkeit dazu besteht z.B. darin, das vorzugsweise abgekühlte (vorzugsweise auf Temperaturen von 10 bis 100 bzw. 70°C) Produktgasgemisch der Propandehydrierung, z. B. bei einem Druck von 0,1 bis 50 bar, vorzugsweise 5 bis 15 bar und einer Temperatur von z. B. 0 bis 100°C, vorzugsweise 20 bis 40°C, mit einem (vorzugsweise hochsiedenden) organischen Lösungsmittel (vorzugsweise ein hydrophobes), in welchem Propan und Propylen (gegenüber den anderen Bestandteilen des Produktgasgemischs der Propandehydrierung zweckmäßig bevorzugt) absorbiert werden, in Kontakt zu bringen (z. B. durch einfaches Durchleiten). Durch nachfolgende Desorption, Rektifikation und/oder Strippung mit einem bezüglich der erfindungsgemäßen Partialoxidation sich inert verhaltenden und/oder in dieser Partialoxidation als Reaktand benötigten Gas (z. B. Luft oder ein anderes Gemisch aus molekularem Sauerstoff und Inertgas) können das Propan und Propylen im Gemisch in gereinigter Form rückgewonnen und dieses Gemisch für die Partialoxidation als Propylenquelle verwendet werden (vorzugsweise wird wie im Vergleichsbeispiel 1 der deutschen Anmeldung DE-A 10 2004 032 129 beschrieben vorgegangen) Das gegebenenfalls molekularen Wasserstoff enthaltende Abgas einer solchen Absorption kann man z.B. wieder einer Druckwechseladsorptions- und/oder Membrantrennung (z.B. gemäß DE-A 10235419) unterwerfen und dann, bei Bedarf, den abgetrennten Wasserstoff mitverwenden.

Allerdings ist der C3-Kohlenwasserstoffe/C4-Kohlenwasserstoffe Trennfaktor beim vorstehenden Trennverfahren vergleichsweise begrenzt und für die in der DE-A 10245585 beschriebenen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propylen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 bar) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlenwasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl® (z. B. von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in DE-A 33 13 573 beschriebenen Paraffinöle mit 8 bis 16 Kohlenstofatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z. B. solche des Typs PKWFaf. Falls sie einen geringen Restaromatengehalt enthalten, kann dieser vorab des beschriebenen Einsatzes vorteilhaft rektifikativ und/oder adsorptiv gemindert und auf Werte signifikant unter 1000 gew.ppm gedrückt werden.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 1 bis 50 bar, bevorzugt 2 bis 20 bar, stärker bevorzugt 5 bis 15 bar, und einer Temperatur von 0 bis 100°C, insbesondere von 20 bis 50 bzw. 40°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glockenund/oder Siebböden), Kolonnen mit strukturierten Packungen (zum Beispiel Blechpackungen mit einer spezifischen Oberfläche von 100 bis 1000, oder bis 750 m$^2$/m$^3$, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propylens von dem Absorptionsmittel kann durch Strippung, Entspannungsverdampfung (Flashen) und/oder Destillation erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200°C, insbesondere 20 bis 100°C, stärker bevorzugt 30 bis 70°C, besonders bevorzugt 30 bis 50°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor und/oder während des Strippprozesses ein Gas zuzusetzen, das den Explosionsbereich reduziert. Besonders geeignet dafür sind Gase mit einer spezifischen Wärmekapazität ≥ 29 J/mol·K bei 20°C, wie zum Beispiel Methan, Ethan, Propan (bevorzugt), Propen, Benzol, Methanol, Ethanol, sowie Ammoniak, Kohlendioxid, und Wasser. C4-Kohlenwasserstoffe sind erfindungsgemäß bevorzugt als solche Zusätze jedoch zu vermeiden. Besonders geeignet für die Strippung sind auch Blasensäulen mit und ohne Einbauten.

Die Abtrennung des Propans und Propylens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300°C, insbesondere 150 bis 250°C.

Eine durch Strippen aus dem Absorptionsmittel gewonnene für das erfindungsgemäße Verfahren prinzipiell geeignete Propylenquelle kann vor ihrer Verwendung zur Beschickung der Partialoxidation noch einer weiteren Verfahrensstufe zugeführt werden, um z. B. die Verluste an mitgestripptem Absorptionsmittel zu reduzieren (z. B. Abscheidung in Demistern und/oder Tiefenfiltern) und die erfindungsgemäße Partialoxidation so gleichzeitig vor Absorptionsmittel zu schützen oder um die Trennwirkung zwischen C3-/C4-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z. B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen (geringe Wassermengen wirken sich förderlich auf die Aktivität der Katalysatoren für die erfindungsgemäße Partialoxidation aus). Diese Wäsche beziehungsweise das Quenchen kann z. B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden wenigstens einen Partialzone normalerweise nicht stört. Nachdem das Wasser das Absorptionsmittel aus dem mit Propan und Propylen beladenen Ausgangstrom herausgewaschen hat, kann das Wasser/Absorptionsmittel-Gemisch einer Phasentrennung zugeführt und der behandelte, volumenarme, Ausgangstrom, unmittelbar der erfindungsgemäßen Partialoxidation zugeführt werden.

In für das erfindungsgemäße Verfahren vorteilhafter Weise lassen sich insbesondere dann, wenn das Propylen/Propan-Gemisch aus dem Absorbat mittels Luft freigestrippt wird, in der Regel unmittelbar anspruchsgemäße Reaktionsgasausgangsgemische 2 gewinnen. Für den Fall, dass deren Propangehalt erfindungsgemäß noch nicht befriedigen sollte, kann ihnen vor ihrer Verwendung für die erfindungsgemäße Partialoxidation des enthaltenen Propylens noch Frischpropan zugesetzt werden. Über das Oxidationskreisgas wird selbiges dann zweckmäßig in die heterogen katalysierte Dehydrierung (als Bestandteil des Reaktionsgasausgangsgemischs 1) rückgeführt werden. Um die entsprechende Propanmenge kann dann die Zufuhr an Frischpropan ins Reaktionsgasausgangsgemisch 1 gemindert werden. Im Extremfall kann die in der heterogen katalysierten Propandehydrierung erforderliche Zufuhr an Frischpropan dann vollständig entfallen, wenn diese Zufuhr an Frischpropan vor der Durchführung der erfindungsgemäßen Partialoxidation des Propylens vollständig ins Reaktionsgasausgangsgemisch 2 hinein erfolgt, von wo aus es dann als verbleibender Bestandteil im Oxidationskreisgas erst nach Durchlaufen der erfindungsgemäßen Partialoxidation dem Reaktionsgasausgangsgemisch 1 für die heterogen katalysierte Propandehydrierung zugeführt wird. Gegebenenfalls kann eine Frischpropanzufuhr auch in eine gegebenenfalls zwischen heterogen katalysierte Dehydrierung und Propylenpartialoxidation befindliche $C_3$-Abtrennung hinein (z. B. als Stripgas) erfolgen.

Handelt es sich um eine zweistufige Partialoxidation von Propylen zu Acrylsäure, kann ein Teil oder auch die vollständige Zufuhr an Frischpropan auch in das Reaktionsgasausgangsgemisch 3 hinein erfolgen (allerdings ist das Reaktionsgasausgangsgemisch 3 manchmal bereits dann nicht explosiv, wenn diese Qualifizierung schon auf das Reaktionsgasausgangsgemisch 2 zutraf). Dies ist vor allem deshalb vorteilhaft, weil die unerwünschte Nebenreaktion von Propan zu Propionaldehyd und/oder Propionsäure vor allem von der ersten Reaktionsstufe unter deren Bedingungen ausgeht. Vorteilhaft ist es auch, eine Frischpropanzufuhr auf die erste und auf die zweite Oxidationsstufe im wesentlichen gleichmäßig zu verteilen.

Durch diese Möglichkeit der Zufuhr von Frischpropan ins Reaktionsgasausgangsgemisch 2 und/oder 3 hinein, lässt sich die Zusammensetzung der Reaktionsgasausgangsgemische 2 und 3 zielsicher nicht explosiv gestalten. Gegebenenfalls kann auch eine Teilmenge an Oxidationskreisgas zu diesem Zweck direkt in die Propylen- und/oder Acroleinpartialoxidation rückgeführt werden. Bei Bedarf kann auch ein Gemisch aus Frischpropan und Oxidationskreisgas für diesen Zweck verwendet werden. Entscheidend für die Beantwortung der Frage, ob das Reaktionsgasausgangsgemisch 2 bzw. 3 explosiv ist oder nicht, ist, ob sich in dem unter bestimmten Ausgangsbedingungen (Druck, Temperatur) befindlichen Reaktionsgasausgangsgemisch 2 bzw. 3 eine durch eine örtliche Zündquelle (z. B. glühender Platindraht) eingeleitete Verbrennung (Entzündung, Explosion) ausbreitet oder nicht (vgl. DIN51649 und die Untersuchungsbeschreibung in der WO 04/007405). Erfolgt eine Ausbreitung, soll das Gemisch hier als explosiv bezeichnet werden. Erfolgt keine Ausbreitung, wird das Gemisch in dieser Schrift als nicht explosiv eingeordnet. Ist das Reaktionsgasausgangsgemische 2 bzw. 3 nicht explosiv, gilt dies auch für die im Verlauf der erfindungsgemäßen Partialoxidation des Propylens gebildeten Reaktionsgasgemische 2 bzw. 3 (vgl. WO 04/007405).

D. h., das erfindungsgemäße Verfahren umfasst insbesondere ein erfindungsgemäßes Verfahren zur Herstellung von Acrolein oder Acrylsäure oder deren Gemisch durch heterogen katalysierte partielle Gasphasenoxidation von Propylen, das dadurch gekennzeichnet ist, dass man

- in einer vorgeschalteten ersten Stufe Propan als Bestandteil eines Reaktionsgasausgangsgemisch 1 einer partiellen heterogen katalysierten Dehydrierung in der Gasphase unter Bildung eines Produktgasgemischs 1, das Propylen und nicht umgesetztes Propan enthält, unterwirft;
- aus dem Propylen und nicht umgesetztes Propan enthaltenden Produktgasgemisch 1 der vorgeschalteten Stufe von den darin enthaltenen, von Propan und Propylen verschiedenen, Bestandteilen gegebenenfalls eine Teilmenge (vorteilhaft wenigstens 50 Vol.-%, mit Vorteil wenigstens 75 Vol.%, besonders bevorzugt wenigstens 90 Vol.% und ganz besonders bevorzugt wenigstens 95 Vol.-% und am besten 100 Vol.-%) abtrennt und es dann als Bestandteil des Reaktionsgasausgangsgemisch 2 für die Herstellung von Acrolein oder Acrylsäure oder deren Gemisch als Zielprodukt durch die heterogen katalysierte partielle Gasphasenoxidation (einstufige oder zweistufige) des im Reaktionsgasausgangsgemisch 2 enthaltenen Propylens verwendet;
- aus dem im Rahmen der partiellen Oxidation des Propylens anfallenden Produktgasgemisch 2 bzw. 3 in einer Abtrennstufe Zielprodukt (Acrolein, oder Acrylsäure, oder deren Gemisch) abtrennt und wenigstens dabei verbleibendes nicht umgesetztes Propan in die vorgeschaltete erste Stufe der partiellen heterogen katalysierten Propandehydrierung zurückführt (bevorzugt wird die gesamte bei der Zielproduktabtrennung verbleibende Restgasmenge als Oxidationskreisgas in die vorgeschaltete erste Stufe rückgeführt).

Insbesondere betrifft die vorliegende Erfindung eine vorteilhafte Ausgestaltung des vorstehenden Verfahrens, bei dem man das für das Verfahren benötigte Propan (Frischpropan) vollständig dem Reaktionsgasausgangsgemisch 1 zuführt. Ferner betrifft die vorliegende Erfindung eine vorteilhafte Ausgestaltung des vorstehenden Verfahrens, bei dem man das für das Verfahren benötigte Propan (Frischpropan) höchstens teilweise (z. B. nur zu 75 %, oder nur zu 50 %, oder nur zu 25 %) dem Reaktionsgasausgangsgemisch 1 und wenigstens teilweise (in der Regel die Restmenge, gegebenenfalls die Gesamtmenge) dem Reaktionsgasausgangsgemisch 2 (und/oder dem Reaktionsgasausgangsgemisch 3) zuführt. Im übrigen kann wie in der WO 01/96170 beschrieben verfahren werden, die einen integralen Bestandteil dieser Anmeldung bildet.

In an sich bekannter Weise läuft die heterogen katalysierte Gasphasen-Partialoxidation von Propylen zu Acrylsäure mit molekularem Sauerstoff grundsätzlich in zwei längs der Reaktionskoordinate aufeinanderfolgenden Schritten ab, von denen der erste zum Acrolein und der zweite von Acrolein zu Acrylsäure führt.

Dieser Reaktionsablauf in zwei zeitlich aufeinanderfolgenden Schritten eröffnet in an sich bekannter Weise die Möglichkeit, das erfindungsgemäße Verfahren auf der Stufe des Acrolein (der Stufe überwiegender Acroleinbildung) abzubrechen und die Zielproduktabtrennung auf dieser Stufe vorzunehmen, oder das erfindungsgemäße Verfahren bis zur überwiegenden Acrylsäurebildung fortzuführen und die Zielproduktabtrennung erst dann vorzunehmen.

Führt man das erfindungsgemäße Verfahren bis zur überwiegenden Acrylsäurebildung, ist es erfindungsgemäß vorteilhaft, das Verfahren zweistufig, d. h. in zwei hintereinander angeordneten Oxidationsstufen auszuführen, wobei zweckmäßigerweise in jeder der beiden Oxidationsstufen das zu verwendende Katalysatorfestbett und vorzugsweise auch die sonstigen Reaktionsbedingungen, wie z. B. die Temperatur des Katalysatorfestbetts, in optimierender Weise angepasst werden.

Zwar vermögen die für die Katalysatoren der ersten Oxidationsstufe (Propylen -> Acrolein) als Aktivmassen besonders geeigneten, die Elemente Mo, Fe, Bi enthaltenden Multimetalloxide, in gewissem Umfang auch die zweite Oxidationsstufe (Acrolein -> Acrylsäure) zu katalysieren, doch werden für die zweite Oxidationsstufe normalerweise Katalysatoren bevorzugt, deren Aktivmasse wenigstens ein die Elemente Mo und V enthaltendes Multimetalloxid ist.

Damit eignet sich das erfindungsgemäße Verfahren der heterogen katalysierten Partialoxidation von Propylen an Katalysatorfestbetten, deren Katalysatoren als Aktivmasse wenigstens ein die Elemente Mo, Fe und Bi enthaltendes Multimetalloxid aufweisen, insbesondere als einstufiges Verfahren zur Herstellung von Acrolein (sowie gegebenenfalls Acrylsäure) oder als erste Reaktionsstufe zur zweistufigen Herstellung von Acrylsäure.

Die Realisierung der einstufigen heterogen katalysierten Partialoxidation von Propylen zu Acrolein sowie gegebenenfalls Acrylsäure bzw. der zweistufigen heterogen katalysierten Partialoxidation von Propylen zu Acrylsäure unter Verwendung eines erfindungsgemäßen Reaktionsgasausgangsgemischs 2 kann dabei im einzelnen wie in den Schriften EP-A 70 07 14 (erste Reaktionsstufe; wie dort beschrieben, aber auch in entsprechender Gegenstromfahrweise von Salzbad und Reaktionsgasausgangsgemisch über den Rohrbündelreaktor), EP-A 70 08 93 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/85363, DE-A 103 13 212 (erste Reaktionsstufe), EP-A 11 59 248 (als zweistufiges Verfahren), EP-A 11 59 246 (zweite Reaktionsstufe), EP-A 11 59 247 (als zweistufiges Verfahren), DE-A 199 48 248 (als zweistufiges Verfahren), DE-A 101 01 695 (einstufig oder zweistufig), WO 04/085368 (als zweistufiges Verfahren), DE 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 99 06 36, EP-A 10 07 007 und EP-A 11 06 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe (Propylen zu Acrolein) ein erfindungsgemäßes Reaktionsgasausgangsgemisch 2 eingesetzt wird. Die übrigen Parameter betreffend wird wie in den Ausführungsbeispielen der genannten Schriften verfahren (insbesondere die Katalysatorfestbetten und Reaktandenbelastung der Katalysatorfestbetten betreffend). Wird in den vorgenannten Ausführungsbeispielen des Standes der Technik zweistufig verfahren und erfolgt zwischen den beiden Reaktionsstufen eine Sekundärsauerstoff(Sekundärluft)einspeisung, so wird diese in entsprechender Weise vorgenommen, in ihrer Menge jedoch dahingehend angepasst, dass das molare Verhältnis von molekularem Sauerstoff zu Acrolein im Beschickungsgemisch der zweiten Reaktionsstufe demjenigen in den Ausführungsbeispielen der genannten Schriften entspricht.

Für die jeweilige Reaktionsstufe besonders geeignete Multimetalloxidkatalysatoren sind vielfach vorbeschrieben und dem Fachmann wohlbekannt. Beispielsweise verweist die EP-A 25 34 09 auf Seite 5 auf entsprechende US-Patente.

Günstige Katalysatoren für die jeweilige Oxidationsstufe offenbaren auch die DE-A 4 431 957, die DE-A 10 2004 025 445 und die DE-A 4 431 949. Dieses gilt insbesondere für jene der allgemeinen Formel I in den beiden vorgenannten Schriften. Besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe der heterogen katalysierten Gasphasen-Partialoxidation von Propylen zu Acrolein oder Acrylsäure oder deren Gemisch, kommen prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 70 07 14 genannten Multimetalloxidaktivmassen.

Ferner eignen sich für diese Reaktionsstufe die Mo, Bi und Fe enthaltenden Multimetalloxidkatalysatoren die in den Schriften DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 198 55 913 (Stöchiometrie: $Mo_{12}Co_7Fe_3Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt von Propylen zu Acrolein und gegebenenfalls Acrylsäure geeigneten Multimetalloxidaktivmassen lässt sich unter der allgemeinen Formel IV,

$$Mo_{12}Bi_aFe_bX^1_cX^2_dX^3_eX^4_fO_n \qquad (IV),$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,
a= 0,5 bis 5,
b= 0,01 bis 5, vorzugsweise 2 bis 4,
c= 0 bis 10, vorzugsweise 3 bis 10,
d= 0 bis 2, vorzugsweise 0,02 bis 2,
e= 0 bis 8, vorzugsweise 0 bis 5,
f= 0 bis 10 und
n= eine Zahl, die durch die Wertigkeit und Häufigkeit der von Sauerstoff verschiedenen Elemente in IV bestimmt wird,

subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den Schritt "Propylen → Acrolein (und gegebenenfalls Acrylsäure)" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein (sowie gegebenenfalls Acrylsäure) zu verwendende Multimetalloxidaktivmassen sind ferner Massen der allgemeinen Formel V,

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (V),$$

in der die Variablen folgende Bedeutung haben:

Y¹ = nur Wismut oder Wismut und wenigstens eines der Elemente Tellur, Antimon, Zinn und Kupfer,
Y² = Molybdän oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶ = Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
a' = 0,01 bis 8,
b' = 0,1 bis 30,
c' = 0 bis 4,

d' = 0 bis 20,

e' > 0 bis 20,

f' = 0 bis 6,

g' = 0 bis 15,

h' = 8 bis 16,

x',y'= Zahlen, die durch die Wertigkeit und Häufigkeit der von Sauerstoff verschiedenen Elemente in V bestimmt werden und

p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäße Multimetalloxidmassen V sind solche, in denen $Y^1$ nur Wismut ist.

Unter diesen werden wiederum jene bevorzugt, die der allgemeinen Formel VI,

$$[Bi_{a''}Z^2_{b''}O_{x''}]_{p''}[Z^2_{12}Z^3_{c''}Z^4_{d''}Fe_{e''}Z^5_{f''}Z^6_{g''}Z^7_{h''}O_{y''}]_{q''} \qquad (VI),$$

in der die Varianten folgende Bedeutung haben:

$Z^2$ = Molybdän oder Molybdän und Wolfram,

$Z^3$ = Nickel und/oder Kobalt,

$Z^4$ = Thallium, ein Alkalimetall und/oder ein Erdalkalimetall,

$Z^5$ = Phosphor, Arsen, Bor, Antimon, Zinn, Cer und/oder Blei,

$Z^6$ = Silicium, Aluminium, Titan und/oder Zirkonium,

$Z^7$ = Kupfer, Silber und/oder Gold,

a" = 0,1 bis 1,

b" = 0,2 bis 2,

c" = 3 bis 10,

d" = 0,02 bis 2,

e" = 0,01 bis 5, vorzugsweise 0,1 bis 3,

f' = 0 bis 5,

g" = 0 bis 10,

h" = 0 bis 1,

x",y"= Zahlen, die durch die Wertigkeit und Häufigkeit der von Sauerstoff verschiedenen Element in VI bestimmt werden,

p",q"=Zahlen, deren Verhältnis p"/q" 0,1 bis 5, vorzugsweise 0,5 bis 2 beträgt,

entsprechen, wobei diejenigen Massen VI ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt 100 mol-%) des gesamten Anteils ($Y^1_{a'}Y^2_{b'}O_{x'}]_p$ ($[Bi_{a''}Z^2_{b''}O_{x''}]_{p''}$) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ [$Bi_{a''}Z^2_{b''}O_{x''}$] vorliegen, deren Größtdurchmesser im Bereich 1 nm bis 100 $\mu$m liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

An dieser Stelle sei erwähnt, dass sich alle Katalysatoren und Multimetalloxidmassen, die für den Schritt vom Propylen zum Acrolein als geeignet empfohlen wurden, sich grundsätzlich auch für die Partialammoxidation von Propylen zu Acrylnitril eignen.

Für den zweiten Schritt(die zweite Reaktionsstufe), die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

$$Mo_{12}V_aX^1_bX^2_cX^3_dX^4_eX^5_fX^6_gO_n \qquad (VII),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,

subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:

$X^1$ = W, Nb, und/oder Cr,
$X^2$ = Cu, Ni, Co, und/oder Fe,
$X^3$ = Sb,
$X^4$ = Na und/oder K,
$X^5$= Ca, Sr und/oder Ba,
$X^6$ = Si, Al, und/oder Ti,
a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VIII,

$$Mo_{12}V_{a'}Y^1_{b'}Y^2_{c'}Y^5_{f'}Y^6_{g'}O_{n'} \qquad (VIII),$$

mit

$Y^1$ = W und/oder Nb,
$Y^2$ = Cu und/oder Ni,
$Y^5$ = Ca und/oder Sr,
$Y^6$ = Si und/oder Al,
a' = 2 bis 4,
b' = 1 bis 1,5,
c' = 1 bis 3,
f' = 0 bis 0,5
g' = 0 bis 8 und
n' = eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Schritt "Acrolein → Acrylsäure" zu verwendende Multimetalloxidaktivmassen sind ferner Massen der allgemeinen Formel IX,

$$[D]_p[E]_q \qquad (IX),$$

in der die Variablen folgende Bedeutung haben:

$D = Mo_{12}V_{a''}Z^1_{b''}Z^2_{c''}Z^3_{d''}Z^4_{e''}-Z^5_{f''}-Z^6_{g''}O_{x''}$,
$E = Z^7_{12}Cu_{h''}H_{i''}O_{y''}$,
$Z^1$ = W, Nb, Ta, Cr und/oder Ce,
$Z^2$ = Cu, Ni, Co, Fe, Mn und/oder Zn,
$Z^3$ = Sb und/oder Bi,
$Z^4$ = Li, Na, K, Rb, Cs und/oder H
$Z^5$ = Mg, Ca, Sr und/oder Ba,
$Z^6$ = Si, Al, Ti und/oder Zr,
$Z^7$ = Mo, W, V, Nb und/oder Ta, vorzugsweise Mo und/oder W
a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,

und die dadurch erhältlich sind, dass man eine Multimetalloxidmasse E

$$Z^7_{12}Cu_{h''}H_{i''}O_{y''}. \qquad (E),$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung des erfindungsgemäßen Verfahrens, vom Propylen zum Acrolein (sowie gegebenenfalls Acrylsäure), kann mit den beschriebenen Katalysatoren z. B. in einem Einzonen-Vielkontaktrohr-Festbettreaktor durchgeführt werden, wie ihn die DE-A 4 431 957 beschreibt. Dabei können Reaktionsgasgemisch 2 und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas(ausgangs)gemisch 2 beträgt vorzugsweise 1500 bis 4000 bzw. 6000 Nl/l·h oder mehr. Die Propylenlast (die Propylenbelastung des Katalysatorfestbetts) beträgt typisch 90 bis 200 Nl/l·h oder bis 300 Nl/l·h oder mehr. Propylenlasten oberhalb von 135 Nl/l·h bzw. $\geq$ 140 Nl/l·h, oder $\geq$ 150 Nl/l·h , oder $\geq$ 160 Nl/l·h sind erfin-

dungsgemäß besonders bevorzugt, da das erfindungsgemäße Reaktionsgasausgangsgemisch 2 ein günstiges Heißpunktverhalten bedingt (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat ($KNO_3$ und 40 Gew.-% Natriumnitrit ($NaNO_2$), oder aus 53 Gew.-% Kaliumnitrat ($KNO_3$), 40 Gew.-% Natriumnitrit ($NaNO_2$) und 7 Gew.-% Natriumnitrat ($NaNO_3$), eingesetzt.

Über den Reaktor betrachtet können, wie bereits gesagt, Salzschmelze und Reaktionsgasgemisch 2 sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):

- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 50 bzw. bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und

- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Durchführung der erfindungsgemäßen Partialoxidation, vom Propylen zum Acrolein (und gegebenenfalls Acrylsäure), kann mit den beschriebenen Katalysatoren aber auch z. B. in einem Zweizonen-Vielkontaktrohr-Festbettreaktor durchgeführt werden, wie es die DE-A 199 10 506 beschreibt. In beiden vorstehend beschriebenen Fällen (sowie ganz allgemein beim erfindungsgemäßen Verfahren) liegt der erzielte Propenumsatz bei einfachem Durchgang normalerweise bei Werten ≥ 90 mol%, bzw. ≥ 95 mol% und die Selektivität der Acroleinbildung bei Werten ≥ 90 mol%. Erfindungsgemäß vorteilhaft erfolgt die erfindungsgemäße Partialoxidation von Propen zu Acrolein, oder Acrylsäure oder deren Gemisch wie in der EP-A 1159244 und ganz besonders bevorzugt wie in der WO 04/085363 sowie in der WO 04/085362 beschrieben, jedoch mit dem Unterschied, dass als Reaktionsgasausgangsgemisch ein erfindungsgemäßes Reaktionsgasausgangsgemisch 2 verwendet wird. Insbesondere können alle Ausführungsbeispiele der vorgenannten Schriften so wie in diesen Schriften beschrieben durchgeführt werden, jedoch unter Anwendung eines Reaktionsgasausgangsgemisch 2 als Beschickungsgasgemisch. Insbesondere mit den in dieser Schrift als besonders bevorzugt und als beispielhaft ausgeführten Reaktionsgasausgangsgemischen 2.

Die Schriften EP-A 1159244, WO 04/085363 und WO 04/085362 werden als integraler Bestandteil dieser Schrift betrachtet.

D. h., die erfindungsgemäße Partialoxidation des Propylens lässt sich besonders vorteilhaft an einem Katalysatorfestbett mit erhöhter Propylenbelastung durchführen, das wenigstens zwei Temperaturzonen aufweist.

D.h., eine vorteilhafte Ausführungsform der erfindungsgemäßen Partialoxidation des Propylens zu Acrolein, oder Acrylsäure oder deren Gemisch ist ein erfindungsgemäßes Verfahren, bei dem man das Reaktionsgasausgangsgemisch 2 mit der Maßgabe über (durch) eine Festbettkatalysatorschüttung, deren Aktivmasse wenigstens ein die Elemente Mo, Fe und Bi enthaltendes Multimetalloxid ist, führt, dass der Propylenumsatz bei einmaligem Durchgang ≥ 90 mol-% und die damit einhergehende Selektivität der Acroleinbildung sowie der Acrylsäurenebenproduktbildung zusammengenommen ≥ 90 mol-% betragen und das dadurch gekennzeichnet ist, dass

a) die Belastung der Festbettkatalysatorschüttung mit dem im Reaktionsgasausgangsgemisch 2 enthaltenen Propen ≥ 160 Nl Propen/l Festbettkatalysatorschüttung • h beträgt,

b) die Festbettkatalysatorschüttung aus einer in zwei räumlich aufeinanderfolgenden Reaktionszonen A*, B* angeordneten Festbettkatalysatorschüttung besteht, wobei die Temperatur der Reaktionszone A* 300 bis 390°C und die Temperatur der Reaktionszone B* 305 bis 420°C beträgt und gleichzeitig wenigstens 5°C oberhalb der Temperatur der Reaktionszone A* liegt,

c) das Reaktionsgasausgangsgemisch 2 die Reaktionszonen A*, B* in der zeitlichen Abfolge "erst A*", "dann B*" durchströmt und

d) sich die Reaktionszone A* bis zu einem Umsatz des Propens von 40 bis 80 mol-% erstreckt.

Im übrigen wird auf die EP-A 1159244 verwiesen.

D. h. aber auch, eine ganz besonders bevorzugte Ausführungsform der erfindungsgemäßen Partialoxidation des Propylens zu Acrolein, oder Acrylsäure, oder deren Gemisch, ist ein erfindungsgemäßes Verfahren, bei dem man das Reaktionsgasausgangsgemisch 2 mit der Maßgabe über eine Festbettkatalysatorschüttung, deren Aktivmasse wenigstens ein die Elemente Mo, Fe und Bi enthaltendes Multimetalloxid ist, führt, dass

- die Festbettkatalysatorschüttung in zwei räumlich aufeinanderfolgenden Temperaturzonen A, B angeordnet ist,

- sowohl die Temperatur der Temperaturzone A als auch die Temperatur der Temperaturzone B eine Temperatur im Bereich von 290 bis 380°C ist,

- die Festbettkatalysatorschüttung aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt,

- sich die Temperaturzone A bis zu einem Umsatz des Propens von 40 bis 80 mol.-% erstreckt,

- bei einmaligem Durchgang des Reaktionsgasausgangsgemisches 2 durch die gesamte Festbettkatalysatorschüttung der Propenumsatz $\geq$ 90 mol.-% und die Selektivität der Acroleinbildung, bezogen auf umgesetztes Propen, $\geq$ 90 mol.-% beträgt,

- die zeitliche Abfolge, in der das Reaktionsgasgemisch 2 die Temperaturzonen A, B durchströmt, der alphabetischen Abfolge der Temperaturzonen entspricht,

- die Belastung der Festbettkatalysatorschüttung mit dem im Reaktionsgasausgangsgemisch 2 enthaltenen Propen $\geq$ 90 Nl Propen/l Festbettkatalysatorschüttung • h beträgt, und

- die Differenz $T^{maxA}$ - $T^{maxB}$, gebildet aus der höchsten Temperatur $T^{maxA}$, die das Reaktionsgasgemisch 2 innerhalb der Temperaturzone A aufweist, und der höchsten Temperatur $T^{maxB}$, die das Reaktionsgasgemisch 2 innerhalb der Temperaturzone B aufweist, $\geq$ 0°C beträgt,

und das zusätzlich dadurch gekennzeichnet ist, dass der Übergang von der Temperaturzone A in die Temperaturzone B in der Festbettkatalysatorschüttung nicht mit einem Übergang von einer Festbettkatalysatorschüttungszone in eine andere Festbettkatalysatorschüttungszone zusammenfällt.

Nähere Angaben zu dieser Verfahrensweise finden sich in der WO 04/085362, die integraler Bestandteil dieser Schrift ist, sowie im weiteren Verlauf dieser Schrift bei der Beschreibung der besonders bevorzugten zweistufigen Partialoxidation von Propylen zu Acrylsäure.

Die Durchführung des zweiten Schrittes im Fall einer zweistufigen Partialoxidation von Propylen zu Acrolein, nämlich die Partialoxidation vom Acrolein zur Acrylsäure, kann mit den beschriebenen Katalysatoren z. B. in einem Einzonen-Vielkontaktrohr-Festbettreaktor durchgeführt werden, wie es die DE-A 44 31 949 beschreibt. Dabei können Reaktiongsgasgemisch 3 und Wärmeträger über den Reaktor betrachtet im Gleichstrom geführt werden. In der Regel wird das Produktgasgemisch der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärluftzugabe erfolgen) desselben), d.h., ohne Nebenkomponentenabtrennung, in die zweite Reaktionsstufe, d.h. in die Acroleinpartialoxidation geführt.

Der für den zweiten Schritt, die Acroleinpartialoxidation, benötigte molekulare Sauerstoff kann (dabei) schon im Reaktionsgasausgangsgemisch 2 für die erfindungsgemäße Propylenpartialoxidation zum Acrolein enthalten sein. Er kann aber auch teilweise oder vollständig dem Produktgasgemisch der ersten Reaktionsstufe, d. h., der erfindungsgemäßen Propylenpartialoxidation zum Acrolein, unmittelbar zugegeben werden (dies erfolgt vorzugsweise als (Sekundär)Luft, kann jedoch auch in Form von reinem Sauerstoff oder von Gemischen aus Inertgas oder Sauerstoff erfolgen). Unabhängig davon wie vorgegangen wird, weist das Beschickungsgasgemisch (das Reaktionsgasausgangsgemisch 3) einer solchen, für sich aus den gleichen Gründen wie die erfindungsgemäße Partialoxidation des Propylens zu Acrolein, oder Acrylsäure, oder deren Gemisch, erfinderischen Partialoxidation des Acroleins zu Acrylsäure, mit Vorteil

EP 1 765 753 B1

nachfolgende Gehalte auf:

| | |
|---|---|
| 4,5 bis 8 Vol.% | Acrolein, |
| 2,25 bzw. 4,5 bis 9 Vol.% | molekularer Sauerstoff, |
| 6 bis 30 Vol.-% | Propan, |
| 32 bis 72 Vol.-% | molekularer Stickstoff und |
| 5 bis 15 Vol.-% | Wasserdampf. |

Bevorzugt weist das Reaktionsgasausgangsgemisch 3 folgende Gehalte auf:

| | |
|---|---|
| 5,5 bis 8 Vol.-% | Acrolein, |
| 2,75 bzw. 5,5 bis 9 Vol.% | molekularer Sauerstoff, |
| 10 bis 25 Vol.-% | Propan, |
| 40 bis 70 Vol.-% | molekularer Stickstoff und |
| 5 bis 15 Vol.-% | Wasserdampf. |

Ganz besonders bevorzugt weist das Reaktiongsgasausgangsgemisch 3 folgende Gehalte auf:

| | |
|---|---|
| 6 bis 8 Vol.-% | Acrolein (bevorzugt 6 bis 7 Vol.-%), |
| 3 bzw. 6 bis 9 Vol.% | molekularer Sauerstoff, |
| 10 bis 20 Vol% | Propan (bevorzugt 10 bis 16 Vol.-%), |
| 50 bis 65 Vol.% | molekularer Stickstoff und |
| 7 bis 13 Vol.-% | Wasserdampf, |

wobei die Vorzugsbereiche unabhängig voneinander gelten, mit Vorteil jedoch simultan realisiert werden.

Ganz generell ist es für alle genannten erfindungsgemäßen Reaktionsgasausgangsgemische 3 günstig, wenn ihr Gesamtgehalt an von den vorstehend spezifizierten Bestandteilen verschiedenen Komponenten $\leq$ 10 Vol.%, bevorzugt $\leq$ 8 Vol.%, besonders bevorzugt $\leq$ 6 bzw. $\leq$ 5 Vol.-% und ganz besonders bevorzugt $\leq$ 4 Vol.-% betragen. Besonders vorteilhaft ist der Gesamtgehalt des Reaktionsgasausgangsgemischs 3 an Kohlenoxiden $\leq$ 5 Vol.% und ganz besonders vorteilhaft $\leq$ 3 Vol.-% bzw. $\leq$ 2 Vol.%.

Der Gehalt des Reaktionsgasausgangsgemischs 3 an Propylen liegt vorzugsweise bei $\leq$ 2 Vol.% und besonders vorteilhaft bei $\leq$ 1 Vol.-%.

Weiterhin ist es für das Reaktionsgasausgangsgemisch 3 von Vorteil, wenn das molare Verhältnis von im Reaktionsgasausgangsgemisch 3 enthaltenem molekularem Sauerstoff zu im Reaktionsgasausgangsgemisch 3 enthaltenem Acrolein $\leq$ 0,5 und $\leq$ 2, mit Vorteil $\geq$ 1 und $\leq$ 1,75, besonders vorteilhaft $\geq$ 1 und $\leq$ 1,5 und ganz besonders vorteilhaft $\geq$ 1 und $\leq$ 1,25 beträgt.

Weiterhin ist es für das Reaktionsgasausgangsgemisch 3 von Vorteil, wenn das molare Verhältnis von in ihm enthaltenem Propan zu in ihm enthaltenem Acrolein 1 bis 4, mit Vorzug 1,5 bis 3,5, besonders bevorzugt 1,5 bis 3 und ganz besonders bevorzugt 1,5 bzw. 2 bis 2,5 beträgt.

Der Gehalt des Reaktionsgasausgangsgemischs 3 an Methan und/oder Ethan wird in der Regel $\leq$ 8 Vol.-%, meist $\leq$ 5 Vol.% und üblicherweise $\leq$ 3 Vol.% bzw. $\leq$ 2 Vol.% betragen. Häufig wird er jedoch mit Vorteil $\geq$ 0,5 Vol.-% betragen.

Wie in der ersten Reaktionsstufe liegt auch in der zweiten Reaktionsstufe der Reaktionsdruck üblicherweise im Bereich von 1 bis 3 bar, und die Gesamtraumbelastung des Katalysatorfestbetts mit Reaktionsgas(ausgangs)gemisch 3 liegt vorzugsweise bei 1500 bis 4000 bzw. 6000 Nl/l·h oder mehr. Die Acroleinlast (die Acroleinbelastung des Katalysatorfestbetts) beträgt typisch 90 bis 190 Nl/l·h, oder bis 290 Nl/l·h oder mehr. Acroleinlasten oberhalb von 135 Nl/l·h, bzw. $\geq$ 140 Nl/l·h, oder $\geq$ 150 Nl/l·h, oder $\geq$ 160 Nl/l·h sind besonders bevorzugt, da das erfindungsgemäße Reaktionsgasausgangsgemisch 3 ebenfalls ein günstiges Heißpunktverhalten bedingt.

Der Acroleinumsatz, bezogen auf einmaligen Durchgang des Reaktionsgasgemischs 3 durch das Katalysatorfestbett, beträgt zweckmäßig normalerweise $\geq$ 90 mol-% und die damit einhergehende Selektivität der Acrylsäurebildung $\geq$ 90 mol-%.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat ($KNO_3$) und 40 Gew.-% Natriumnitrit ($NaNO_2$) oder aus 53 Gew.-% Kaliumnitrat ($KNO_3$), 40 Gew.-% Natriumnitrit ($NaNO_2$) und 7 Gew.-% Natriumnitrat (Na-$NO_3$) bestehend, eingesetzt. Über den Reaktor betrachtet können, wie bereits gesagt, Salzschmelze und Reaktionsgasgemisch 3 sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:

- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und

- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttempatur und Heißpunkttemperatursensitivität). Von unten nach oben zunächst mit bis zu 20 Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 443 19 49 Gesagte. Sie wird in der Regel so gewählt, daß der erzielte Acroleinumsatz bei einfachem Durchgang normalerweise ≥ 90 mol-%, bzw. ≥ 95 mol-% oder ≥ 99 mol-% beträgt.

Die Durchführung der Partialoxidation vom Acrolein zur Acrylsäure, kann mit den beschriebenen Katalysatoren aber auch z. B. in einem Zweizonen-VielkontaktrohrFestbettreaktor durchgeführt werden, wie es die DE-199 10 508 beschreibt. Für den Acroleinumsatz gilt das oben Genannte. Auch im Fall der Durchführung einer wie vorstehend beschriebenen Acroleinpartialoxidation als zweite Reaktionsstufe einer zweistufigen Propylenoxidation zu Acrylsäure in einem Zweizonen-Vielkontaktrohr-Festbettreaktor wird man zur Erzeugung des Beschickungsgasgemisches (Reaktionsgasausgangsgemischs 3) zweckmäßig unmittelbar das Produktgasgemisch der auf den ersten Schritt gerichteten Partialoxidation (gegebenenfalls nach erfolgter indirekter oder direkter (z. B. durch Zufuhr von Sekundärluft) Zwischenkühlung desselben) verwenden (wie sie vorstehend bereits beschrieben wurde). Der für die Acroleinpartialoxidation benötigte Sauerstoff wird vorzugsweise als Luft (gegebenenfalls aber auch als reiner molekularer Sauerstoff oder als Gemisch aus molekularem Sauerstoff und einem Inertgas) zugesetzt und z. B. dem Produktgasgemisch des ersten Schritts der zweistufigen Partialoxidation (Propylen → Acrolein) unmittelbar zugegeben. Er kann aber auch, wie bereits beschrieben, bereits im Reaktionsgasausgangsgemisch 2 für die erste Reaktionsstufe enthalten sein.

Bei einer zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 101 21 592 beschrieben in einem Reaktor an einer Beschickung vollzogen werden.

Es sei auch nochmals erwähnt, dass ein Teil des Reaktionsgasausgangsgemischs 2 für den ersten Schritt ("Propylen → Acrolein") aus der Partialoxidation kommendes Oxidationskreisgas (Restgas) sein kann.

Hierbei handelt es sich, wie bereits gesagt, um ein molekularen Sauerstoff enthaltendes Gas, das nach der Zielproduktabtrennung (Acrolein- und/oder Acrylsäureabtrennung) vom Produktgasgemisch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan, molekularen Sauerstoff und gegebenenfalls nicht umgesetztes Propylen enthaltendes Oxidationskreisgas jedoch vorteilhaft ausschließlich in die gegebenenfalls als Propylenquelle fungierende heterogen katalysierte Propandehydrierung rückgeführt.

Insgesamt bildet ein Rohrbündelreaktor innerhalb dessen sich die Katalysatorbeschickung längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 91 13 13, die EP-A 97 98 13, die EP-A 99 06 36 und die DE-A 28 30 765) die einfachste Realisierungsform zweier Oxidationsstufen für die beiden Schritte der Partialoxidation von Propylen zu Acrylsäure. Gegebenenfalls wird dabei die Beschickung der Kontaktrohre mit Katalysator durch eine Inertmaterialschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertmaterialschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Die Reaktionstemperatur in der ersten Reaktionsstufe (Propylen → Acrolein) liegt in der Regel bei 300 bis 450°C, bevorzugt bei 320 bis 390°C. Die Reaktionstemperatur in der zweiten Reaktionsstufe (Acrolein → Acrylsäure) liegt in der Regel bei 200 bis 370°C, häufig bei 220 bis 330°C. Der Reaktionsdruck beträgt in beiden Oxidationszonen zweckmäßig 0,5 bis 5, vorteilhaft 1 bis 3 bar. Die Belastung (Nl/l·h) der Oxidationskatalysatoren mit Reaktionsgas beträgt in beiden Reaktionsstufen häufig 1500 bis 2500 Nl/l·h bzw. bis 4000 Nl/l·h. Die Belastung mit Propylen kann dabei bei 100 bis 200 oder 300 und mehr Nl/l·h liegen.

Prinzipiell können die beiden Oxidationsstufen beim erfindungsgemäßen Verfahren so gestaltet werden, wie es z. B. in der DE-A 198 37 517, der DE-A 199 10 506, der DE-A 199 10 508 sowie der DE-A 198 37 519 beschrieben ist.

In beiden Reaktionsstufen wirkt sich dabei ein Überschuss an molekularem Sauerstoff relativ zur reaktionsstöchiometrisch erforderlichen Menge in der Regel vorteilhaft auf die Kinetik der jeweiligen Gasphasenpartialoxidation aus.

Prinzipiell ist die erfindungsgemäße heterogen katalysierte Gasphasen-Partialoxidation von Propylen zu Acrylsäure aber auch in einem einzigen Einzonenrohrbündelreaktor wie folgt realisierbar. Beide Reaktionsschritte erfolgen in einem Oxidationsreaktor der mit einem oder mehreren Katalysatoren beschickt ist, deren Aktivmasse ein die Elemente Mo, Fe und Bi enthaltendes Multimetalloxid ist, das die Umsetzung beider Reaktionsschritte zu katalysieren vermag. Selbstredend kann sich auch diese Katalysatorbeschickung längs der Reaktionskoordinate kontinuierlich oder abrupt ändern. Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassenden Produktgasgemisch in selbigem enthaltenes, in der ersten Oxidationsstufe als Nebenprodukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch einer Abtrennung nicht vorsieht.

Als Quellen für eine zwischen beiden Oxidationsstufen durchgeführte Sauerstoffzwischeneinspeisung kommen, wie bereits gesagt, neben Luft (bevorzugt) sowohl reiner molekularer Sauerstoff als auch mit Inertgas wie $CO_2$, CO, Edelgasen, $N_2$ und/oder gesättigten Kohlenwasserstoffen verdünnter molekularer Sauerstoff in Betracht.

Durch Zudosieren von z. B. kalter Luft zu heißem Produktgasgemisch 2 kann im Rahmen des erfindungsgemäßen Verfahrens auch auf direktem Weg eine Abkühlung desselben bewirkt werden, bevor es als Bestandteil eines Reaktionsgasausgangsgemisch 3 weiterverwendet wird.

Erfindungsgemäß vorteilhaft erfolgt die Partialoxidation von Acrolein zu Acrylsäure wie in der EP-A 11 59 246, und ganz besonders bevorzugt wie in der WO 04/085365 sowie in der WO 04/085370 beschrieben. Erfindungsgemäß bevorzugt wird dabei als Acrolein enthaltendes Reaktionsgasausgangsgemisch jedoch ein Reaktionsgasausgangsgemisch 3 verwendet (dies kann insbesondere das Produktgasgemisch einer erfindungsgemäßen Erststufenpartialoxidation von Propylen zu Acrolein sein, das gegebenenfalls mit soviel Sekundärluft ergänzt wurde, dass das Verhältnis von molekularem Sauerstoff zu Acrolein in dem resultierenden Reaktionsgasausgangsgemisch 3 in jedem Fall 0,5 bis 1,5 beträgt). Insbesondere können alle Ausführungsbeispiele der vorgenannten Schriften so wie in diesen Schriften beschrieben durchgeführt werden, jedoch unter Anwendung eines Reaktionsgasausgangsgemischs 3 als Beschickungsgasgemisch. Insbesondere mit den in dieser Schrift als besonders bevorzugt und als beispielhaft ausgeführten Reaktionsgasausgangsgemischen 3. Die Schriften EP-A 1159246, WO 04/08536 und WO 04/085370 werden als integraler Bestandteil dieser Schrift betrachtet.

D.h., die erfindungsgemäße Partialoxidation von Acrolein zu Acrylsäure lässt sich vorteilhaft an einem Katalysatorfestbett mit erhöhter Acroleinbelastung durchführen, das wenigstens zwei Temperaturzonen aufweist.

D.h., eine vorteilhafte Ausführungsform der erfindungsgemäßen Partialoxidation des Acroleins zu Acrylsäure ist ein Verfahren, bei dem man das Reaktionsgasausgangsgemisch 3 mit der Maßgabe über (durch) eine Festbettkatalysatorschüttung, deren Aktivmasse wenigstens ein die Elemente Mo und V enthaltendes Multimetalloxid ist, führt, dass der Acroleinumsatz bei einmaligem Durchgang $\geq 90$ mol-% und die damit einhergehende Selektivität der Acrylsäurebildung $\geq 90$ mol-% betragen und das dadurch gekennzeichnet ist, dass

a) die Belastung der Festbettkatalysatorschüttung mit dem im Reaktionsgasausgangsgemisch 3 enthaltenen Acrolein $\geq 150$ NI Acrolein/I Festbettkatalysatorschüttung • h beträgt,
b) die Festbettkatalysatorschüttung aus einer in zwei räumlich aufeinanderfolgenden Reaktionszonen C\*, D\* angeordneten Festbettkatalysatorschüttung besteht, wobei die Temperatur der Reaktionszone C\* 230 bis 270°C und die Temperatur der Reaktionszone D\* 250 bis 300°C beträgt und gleichzeitig wenigstens 5°C oberhalb der Temperatur der Reaktionszone C\* liegt,
c) das Reaktionsgasausgangsgemisch 3 die Reaktionszonen C\*, D\* in der zeitlichen Abfolge "erst C\*", "dann D\*" durchströmt und
d) sich die Reaktionszone C\* bis zu einem Umsatz des Acroleins von 55 bis 85 mol-% erstreckt.

Im übrigen wird auf die EP-A 1159246 verwiesen.

D.h. aber auch, eine ganz besonders bevorzugte Ausführungsform der erfindungsgemäßen Partialoxidation des Acroleins zu Acrlysäure ist ein erfindungsgemäßes Verfahren, bei dem man das Reaktionsgasausgangsgemisch 3 mit der Maßgabe über eine Festbettkatalysatorschüttung, deren Aktivmasse wenigstens ein die Elemente Mo und V enthaltendes Multimetalloxid ist, führt, dass

- die Festbettkatalysatorschüttung in zwei räumlich aufeinanderfolgenden Temperaturzonen C, D angeordnet ist,

- sowohl die Temperatur der Temperaturzone C als auch die Temperatur der Temperaturzone D eine Temperatur im Bereich von 230 bis 320°C ist,

- die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 3 beim Übergang von einer Festbettkatalysatorschüttungszone in eine andere Festbettkatalysatorschüttungszone sprunghaft zunimmt,

- sich die Temperaturzone C bis zu einem Umsatz des Acroleins von 45 bis 85 mol-% erstreckt,

- bei einmaligem Durchgang des Reaktionsgasausgangsgemisches 3 durch die gesamte Festbettkatalysatorschüttung der Acroleinumsatz $\geq 90$ mol-% und die Selektivität der Acrylsäurebildung, bezogen auf umgesetztes Acrolein, $\geq 90$ mol-% beträgt,

  - die zeitliche Abfolge, in der das Reaktionsgasgemisch 3 die Temperaturzonen C, D durchströmt, der alphabetischen Abfolge der Temperaturzonen entspricht,

  - die Belastung der Festbettkatalysatorschüttung mit dem im Reaktionsgasausgangsgemisch 3 enthaltenen Acrolein $\geq 70$ NI Acrolein/I Festbettkatalysatorschüttung • h beträgt, und

  - die Differenz $T^{maxC} - T^{maxD}$, gebildet aus der höchsten Temperatur $T^{maxC}$, die das Reaktionsgasgemisch 3 innerhalb der Temperaturzone C aufweist, und der höchsten Temperatur $T^{maxD}$, die das Reaktionsgasgemisch 3 innerhalb der Temperaturzone D aufweist, $\geq 0$°C beträgt,

und das zusätzlich dadurch gekennzeichnet ist, dass der Übergang von der Temperaturzone C in die Temperaturzone D in der Festbettkatalysatorschüttung nicht mit einem Übergang von einer Festbettkatalysatorschüttungszone in eine andere Festbettkatalysatorschüttungszone zusammenfällt.

Nähere Angaben zu dieser Verfahrensweise finden sich in der WO 04/085370, die integraler Bestandteil dieser Schrift ist, sowie im weiteren Verlauf dieser Schrift bei der Beschreibung der besonders bevorzugten zweistufigen Partialoxidation von Propylen zu Acrylsäure.

EP 1 765 753 B1

Eine solche bevorzugte zweistufige Partialoxidation von Propylen zu Acrylsäure kann vorteilhaft wie in der EP-A 1159248 sowie in der WO 04/085367 beschrieben durchgeführt werden, jedoch mit dem Unterschied, dass als Reaktionsgasausgangsgemisch für die erste Oxidationsstufe (Propylen zu Acrolein) ein erfindungsgemäßes Reaktionsgasausgangsgemisch 2 verwendet wird (insbesondere auch in den Ausführungsbeispielen der EP-A 1159248 sowie der WO 04/085367; beide Schriften bilden einen integralen Bestandteil dieser Schrift).

D.h., man wird zunächst ein erfindungsgemäßes Reaktionsgasausgangsgemisch 2 in einer ersten Reaktionsstufe mit der Maßgabe über (durch) eine Festbettkatalysatorschüttung 1, deren Aktivmasse wenigstens ein die Elemente Mo, Fe und Bi enthaltendes Multimetalloxid ist, führen, dass der Propylenumsatz bei einmaligem Durchgang $\geq$ 90 mol-% und die damit einhergehende Selektivität der Acroleinbildung sowie der Acrylsäurenebenproduktbildung zusammengenommen $\geq$ 90 mol-% betragen, die Temperatur des die erste Reaktionsstufe verlassenden Produktgasgemischs 2 durch indirekte und/oder direkte Kühlung gegebenenfalls verringern und dem Produktgasgemisch 2 gegebenenfalls molekularen Sauerstoff und/oder Inertgas zugeben, und danach als Acrolein, molekularen Sauerstoff und wenigstens ein Inertgas enthaltendes Reaktionsgasausgangsgemisch 3, das den molekularen Sauerstoff und das Acrolein in einem molaren Verhältnis $O_2:C_3H_4O \geq 0,5$ enthält, in einer zweiten Reaktionsstufe mit

der Maßgabe über (durch) eine Festbettkatalysatorschüttung 2, deren Aktivmasse wenigstens ein Molybdän und Vanadin enthaltendes Multimetalloxid ist, führen, dass der Acroleinumsatz bei einmaligem Durchgang $\geq$ 90 mol-% und die Selektivität der über beide Reaktionsstufen bilanzierten Acrylsäurebildung, bezogen auf umgesetztes Propylen, $\geq$ 80 mol-% beträgt und dabei weiterhin so verfahren, dass

a) die Belastung der Festbettkatalysatorschüttung 1 mit dem im Reaktionsgasausgangsgemisch 2 enthaltenen Propen $\geq$ 160 NI Propen/I Festbettkatalysatorschüttung 1 • h beträgt,

b) die erste Festbettkatalysatorschüttung aus einer in zwei räumlich aufeinanderfolgenden Reaktionszonen A*, B* angeordneten Festbettkatalysatorschüttung besteht, wobei die Temperatur der Reaktionszone A* 300 bis 390°C und die Temperatur der Reaktionszone B* 305 bis 420°C beträgt und gleichzeitig wenigstens 5°C oberhalb der Temperatur der Reaktionszone A* liegt,

c) das Reaktionsgasausgangsgemisch 2 die Reaktionszonen A*, B* in der zeitlichen Abfolge "erst A*", "dann B*" durchströmt,

d) die Reaktionszone A* sich bis zu einem Umsatz des Propens von 40 bis 80 mol-% erstreckt,

e) die Belastung der Festbettkatalysatorschüttung 2 mit dem im Reaktionsgasausgangsgemisch 3 enthaltenen Acrolein $\geq$ 140 NI Acrolein/I Festbettkatalysatorschüttung 2 • h beträgt,

f) die Festbettkatalysatorschüttung 2 aus einer in zwei räumlich aufeinanderfolgenden Reaktionszonen C*, D* angeordneten Festbettkatalysatorschüttung 2 besteht, wobei die Temperatur der Reaktionszone C* 230 bis 270°C und die Temperatur der Reaktionszone D* 250 bis 300°C beträgt und gleichzeitig wenigstens 10°C oberhalb der Reaktionszone C* liegt,

g) das Reaktionsgasausgangsgemisch 3 die Rektionszonen C*, D* in der zeitlichen Abfolge "erst C*", "dann D*" durchströmt und

h) sich die Reaktionszone C* bis zu einem Umsatz des Acroleins von 55 bis 85 mol-% erstreckt.

Im übrigen wird auf die EP-A 1159248 verwiesen.

Besonders bevorzugt wird man sie jedoch gemäß der WO 04/085369 durchführen, die integraler Bestandteil dieser Schrift ist, jedoch mit dem Unterschied, dass als Reaktionsgasausgangsgemisch für die erste Oxidationsstufe (Propylen zu Acrolein) ein erfindungsgemäßes Reaktionsgasausgangsgemisch 2 verwendet wird (insbesondere auch in den Ausführungsbeispielen der WO 04/085369).

D. h., man wird zunächst ein erfindungsgemäßes Reaktionsgasausgangsgemisch 2 in einer ersten Reaktionsstufe mit der Maßgabe über eine Festbettkatalysatorschüttung 1, deren Aktivmasse wenigstens ein die Elemente Mo, Fe und Bi enthaltendes Multimetalloxid ist, führen, dass

- die Festbettkatalysatorschüttung 1 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol-% erstreckt,
- bei einmaligem Durchgang des Reaktionsgasausgangsgemisches 2 durch die gesamte Festbettkatalysatorschüttung 1 der Propenumsatz $\geq$ 90 mol-% und die Selektivität der Acroleinbildung sowie der Acrylsäurenebenproduktbildung zusammengenommen und bezogen auf umgesetztes Propen $\geq$ 90 mol-% beträgt,

- die zeitliche Abfolge, in der das Reaktionsgasgemisch 2 die Temperaturzonen A, B durchströmt, der alphabetischen Abfolge der Temperaturzonen A, B entspricht,
- die Belastung der Festbettkatalysatorschüttung 1 mit dem im Reaktionsgasausgangsgemisch 2 enthaltenen Propen $\geq$ 90 NI Propen/I Festbettkatalysatorschüttung 1 · h beträgt, und
- die Differenz $T^{maxA}$ - $T^{maxB}$, gebildet aus der höchsten Temperatur $T^{maxA}$, die das Reaktionsgasgemisch 2 innerhalb der Temperaturzone A aufweist, und der höchsten Temperatur $T^{maxB}$, die das Reaktionsgasgemisch 2 innerhalb der Temperaturzone B aufweist, $\geq$ 0°C beträgt,

danach die Temperatur des die erste Reaktionsstufe verlassenden Produktgasgemisches 2 durch Kühlung gegebenenfalls verringern und dem Produktgasgemisch 2 gegebenenfalls molekularen Sauerstoff und/oder Inertgas, vorzugsweise gegebenenfalls Luft, zugeben, und es anschließend als Acrolein, molekularen Sauerstoff und wenigstens ein Inertgas enthaltendes Reaktionsgasausgangsgemisch 3, das den molekularen Sauerstoff und das Acrolein in einem molaren Verhältnis $O_2:C_3H_4O \geq 0,5$ enthält, in einer zweiten Reaktionsstufe mit der Maßgabe über eine Festbettkatalysatorschüttung 2, deren Aktivmasse wenigstens ein die Elemente Mo und V enthaltendes Multimetalloxid ist, führen, dass

- die Festbettkatalysatorschüttung 2 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung 2 aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 3 beim Übergang von einer Festbettkatalysatorschüttungszone in eine andere Festbettkatalysatorschüttungszone sprunghaft zunimmt,
- sich die Temperaturzone C bis zu einem Umsatz des Acroleins von 45 bis 85 mol-% erstreckt,
- bei einmaligem Durchgang des Reaktionsgasausgangsgemisches 3 durch die gesamte Festbettkatalysatorschüttung 2 der Acroleinumsatz $\geq$ 90 mol-% und die Selektivität der Acrylsäurebildung, bezogen auf über beide Reaktionsstufen umgesetztes Propen, $\geq$ 80 mol-% beträgt,
- die zeitliche Abfolge, in der das Reaktionsgasgemisch 3 die Temperaturzonen C, D durchströmt, der alphabetischen Abfolge der Temperaturzonen C, D entspricht,
- die Belastung der Festbettkatalysatorschüttung 2 mit dem im Reaktionsgasausgangsgemisch 3 enthaltenen Acrolein $\geq$ 70 NI Acrolein/I Festbettkatalysatorschüttung 2•h beträgt, und
- die Differenz $T^{maxC}$ - $T^{maxD}$, gebildet aus der höchsten Temperatur $T^{maxC}$, die das Reaktionsgasgemisch 3 innerhalb der Temperaturzone C aufweist, und der höchsten Temperatur $T^{maxD}$, die das Reaktionsgasgemisch 3 innerhalb der Temperaturzone D aufweist, $\geq$ 0°C beträgt,

mit der Maßgabe, dass das Verfahren zusätzlich dadurch gekennzeichnet ist, dass weder der Übergang von der Temperaturzone A in die Temperaturzone B in der Festbettkatalysatorschüttung 1, noch der Übergang von der Temperaturzone C in die Temperaturzone D in der Festbettkatalysatorschüttung 2 mit einem Übergang von einer Festbettkatalysatorschüttungszone in eine andere Festbettkatalysatorschüttungszone zusammenfällt.

Unter der Temperatur einer Temperaturzone wird dabei die Temperatur des in der Temperaturzone befindlichen Teils der Festbettkatalysatorschüttung bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur der Festbettkatalysatorschüttung längs der Reaktionszone. Wesentlich ist dabei, dass die Temperierung der einzelnen Temperaturzonen im wesentlichen unabhängig voneinander erfolgt.

Da sowohl die heterogen katalysierte partielle Gasphasenoxidation von Propen zu Acrolein als auch die heterogen katalysierte partielle Gasphasenoxidation von Acrolein zu Acrylsäure eine ausgeprägt exotherme Reaktion ist, ist sowohl die Temperatur des Reaktionsgasgemisches 2 als auch die Temperatur des Reaktionsgasgemisches 3 beim reaktiven Durchgang durch die Festbettkatalysatorschüttung 1 bzw. Festbettkatalysatorschüttung 2 in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz $T^{maxA}$ - $T^{maxB}$ nicht mehr als 80°C betragen. Erfindungsgemäß bevorzugt beträgt $T^{maxA}$ - $T^{maxB}$ $\geq$ 3°C und $\leq$ 70°C. Ganz besonders bevorzugt beträgt $T^{maxA}$ - $T^{maxB}$ beim erfindungsgemäßen Verfahren $\geq$ 20°C und $\leq$ 60°C.

Die erfindungsgemäß geforderten Differenzen $T^{maxA}$ - $T^{maxB}$ stellen sich bei der Ausübung des erfindungsgemäßen Verfahren im Fall von eher niederen ($\geq$ 90 NI/I · h und $\leq$ 160 NI/I · h) Propenbelastungen der Festbettkatalysatorschüttung 1 normalerweise dann ein, wenn einerseits sowohl die Temperatur der Reaktionszone A als auch die Temperatur der Reaktionszone B im Bereich von 290 bis 380°C liegt und andererseits die Differenz zwischen der Temperatur der Reaktionszone B ($T_B$) und der Temperatur der Reaktionszone A ($T_A$), d.h., $T_B$ - $T_A$, ≤ 0°C und ≥ -20°C oder ≥ -10°C bzw. ≤ 0°C und ≥ -5°C, oder häufig ≤ 0°C und ≥ -3°C beträgt.

Bei Ausübung des erfindungsgemäßen Verfahrens unter (erfindungsgemäß bevorzugt) erhöhten Propenbelastungen (≥ 160 Nl/l · h und ≤ 300 Nl/l · h, bzw. · 600 Nl/l · h) stellen sich die erfindungsgemäß geforderten Differenzen $T^{maxA}$ - $T^{maxB}$ normalerweise dann ein, wenn einerseits sowohl die Temperatur der Reaktionszone A als auch die Temperatur der Reaktionszone B im Bereich von 290 bis 380°C liegt und $T_B$ - $T_A$ ≥ 0°C und ≤ 50°C, oder ≥ 5°C und ≤ 45°C, oder ≥ 10°C und ≤ 40°C, oder ≥ 15°C und ≤ 30°C oder ≤ 35°C (z.B. 20°C oder 25°C) beträgt.

Die vorgenannte Aussage betreffend die Temperaturdifferenzen $T_B$ - $T_A$ gilt regelmäßig auch dann, wenn die Temperatur der Reaktionszone A im bevorzugten Bereich von 305 bis 365°C bzw. im besonders bevorzugten Bereich von 310 bis 340°C liegt.

Die Propenbelastung der Festbettkatalysatorschüttung 1 kann somit beim beschriebenen Verfahren z.B. ≥ 90 Nl/l · h und ≤ 300 Nl/l · h, oder ≥ 110 Nl/l · h und ≤ 280 Nl/l · h oder ≥ 130 Nl/l · h und ≤ 260 Nl/l · h, oder ≥ 150 Nl/l · h und ≤ 240 Nl/l · h, oder ≥ 170 Nl/l · h und ≤ 220 Nl/l · h, oder ≥ 190 Nl/l · h und ≤ 200 Nl/l · h betragen.

Erfindungsgemäß bevorzugt erstreckt sich die Temperaturzone A bis zu einem Umsatz des Propens von 50 bis 70 mol-% bzw. 60 bis 70 mol-%.

In der Regel wird beim erfindungsgemäßen Verfahren die Differenz $T^{maxC}$ - $T^{maxD}$ nicht mehr als 75°C betragen. Erfindungsgemäß bevorzugt beträgt $T^{maxC}$ - $T^{maxD}$ ≥ 3°C und ≤ 60°C. Ganz besonders bevorzugt beträgt $T^{maxC}$ - $T^{maxD}$ beim erfindungsgemäßen Verfahren ≥ 5°C und ≤ 40°C.

Die erfindungsgemäß geforderten Differenzen $T^{maxC}$ - $T^{maxD}$ stellen sich bei der Ausübung des erfindungsgemäßen Verfahren im Fall von eher niederen (≥ 70 Nl/l · h und ≤ 150 Nl/l · h) Acroleinbelastungen der Festbettkatalysatorschüttung 2 normalerweise dann ein, wenn einerseits sowohl die Temperatur der Reaktionszone C als auch die Temperatur der Reaktionszone D im Bereich von 230 bis 320°C liegt und andererseits die Differenz zwischen der Temperatur der Reaktionszone D ($T_D$) und der Temperatur der Reaktionszone C ($T_C$), d.h., $T_D$ - $T_C$, ≤ 0°C und ≥ -20°C oder ≥ -10°C bzw. ≤ 0°C und ≥ -5°C, oder häufig ≤ 0°C und ≥ -3°C beträgt.

Bei Ausübung des erfindungsgemäßen Verfahrens unter erhöhten Propenbelastungen und damit gleichzeitig erhöhten Acroleinbelastungen (≥ 150 Nl/l · h und ≤ 300 Nl/l · h, bzw. ≤ 600 Nl/l · h) stellen sich die erfindungsgemäß geforderten Differenzen $T^{maxC}$ - $T^{maxD}$ normalerweise dann ein, wenn einerseits sowohl die Temperatur der Reaktionszone C als auch die Temperatur der Reaktionszone D im Bereich von 230 bis 320°C liegt und $T_D$ - $T_C$ ≥ 0°C und ≤ 40°C, oder ≥ 5°C und ≤ 35°C, bzw. 30°C, oder ≥ 10°C und ≤ 25°C, bzw. ≤ 20°C, oder ≤ 15°C beträgt.

Die vorgenannte Aussage betreffend die Temperaturdifferenzen $T_D$ - $T_C$ gilt regelmäßig auch dann, wenn die Temperatur der Reaktionszone C im bevorzugten Bereich von 250 bis 300°C bzw. im besonders bevorzugten Bereich von 260 bis 280°C liegt.

Die Acroleinbelastung der Festbettkatalysatorschüttung 2 kann somit beim erfindungsgemäßen Verfahren z. B. ≥ 70 Nl/l · h bzw. · 90 Nl/l · h und ≤ 300 Nl/l · h, oder ≥ 110 Nl/l · h und ≤ 280 Nl/l · h oder ≥ 130 Nl/l · h und ≤ 260 Nl/l · h, oder ≥ 150 Nl/l · h und ≤ 240 Nl/l · h, oder ≥ 170 Nl/l · h und ≤ 220 Nl/l · h, oder ≥ 190 Nl/l · h und ≤ 200 Nl/l · h betragen.

Erfindungsgemäß bevorzugt erstreckt sich die Temperaturzone C bis zu einem Umsatz des Acroleins von 50 bis 85 mol-% bzw. 60 bis 85 mol-%.

Der Arbeitsdruck kann dabei in beiden Reaktionsstufen sowohl unterhalb von Normaldruck (z. B. bis zu 0,5 bar) als auch oberhalb von Normaldruck liegen. Typischerweise wird der Arbeitsdruck in den beiden Reaktionsstufen bei Werten von 1 bis 5 bar, häufig 1 bis 3 bar liegen.

Normalerweise wird der Reaktionsdruck in keiner der beiden Reaktionsstufen 100 bar überschreiten.

In der Regel wird der auf den einfachen Durchgang der Festbettkatalysatorschüttung 1 bezogene Propenumsatz bei der beschriebenen Verfahrensweise ≥ 92 mol-% oder ≥ 94 mol-% betragen. Die Selektivität der Wertproduktbildung (Summe aus Acroleinbildung und Acrylsäurenebenproduktbildung) wird dabei bei in an sich bekannter Weise geeigneter Wahl (siehe in dieser Schrift empfohlene Katalysatoren) der Festbettkatalysatorschüttung 1 regelmäßig ≥ 92 mol-%, oder ≥ 94 mol%, häufig ≥ 95 mol-%, oder ≥ 96 mol% bzw. ≥ 97 mol-% betragen.

In der Regel wird beim vorstehend beschriebenen Verfahren die Acroleinbelastung der Festbettkatalysatorschüttung 2 ferner etwa 10 Nl/l · h, häufig etwa 20 bzw. 25 Nl/l · h unterhalb der Propenbelastung der Festbettkatalysatorschüttung 1 liegen. Dies ist primär darauf zurückzuführen, dass in der ersten Reaktionsstufe sowohl der Umsatz des Propens als auch die Selektivität der Acroleinbildung in der Regel keine 100 % erreichen.

In der Regel wird der auf den einfachen Durchgang der Festbettkatalysatorschüttung 2 bezogene Acroleinumsatz beim vorstehend beschriebenen Verfahren ≥ 92 mol-%, oder ≥ 94 mol-%, oder ≥ 96 mol-%, oder ≥ 98 mol-% und häufig sogar ≥ 99 mol-% oder mehr betragen.

Bei in an sich bekannter Weise geeigneter Wahl der Festbettkatalysatorschüttungen 1 und 2 (siehe die in dieser Schrift gegebenen Katalysatorempfehlungen) wird die bei der vorstehend beschriebenen Verfahrensweise über beide Reaktionsstufen bilanzierte Selektivität der Acrylsäurebildung, bezogen auf umgesetztes Propen, bei Werten ≥ 83 mol-%, häufig bei ≥ 85 mol-%, oder ≥ 88 mol-%, oft bei ≥ 90 mol-%, oder ≥ 93 mol-% liegen.

Für die beschriebene Verfahrensweise wesentlich ist, dass die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt.

Die volumenspezifische (d. h., die auf die Einheit der jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eigenen.

Als solche Materialien kommen z. B. poröse oder unporöse Aluminiumoxide, Siliciumdioxid, Thoriumdioxid, Zirkondioxid, Siliziumcarbid, Silikate wie Magnesium -oder Aluminiumsilikat oder der bereits erwähnte Steatit (z. B. Steatit C-220 der Fa. Ceram Tec) in Betracht.

Die Geometrie solcher inerter Verdünnungsformkörper kann im Prinzip beliebig sein. D. h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung 1 nicht verändert. D. h., die für einen einzelnen Katalysatorformkörper verwendete Aktivmasse kann zwar ein Gemisch aus verschiedenen, die Elemente Mo, Fe und Bi enthaltenden, Multimetalloxiden sein, für alle Katalysatorformkörper der Festbettkatalysatorschüttung 1 ist dann jedoch das gleiche Gemisch zu verwenden.

Eine in Strömungsrichtung des Reaktionsgasgemisches 2 über die Festbettkatalysatorschüttung zonenweise zunehmende volumenspezifische Aktivität lässt sich für das beschriebene Verfahren somit in einfacher Weise z. B. dadurch einstellen, dass man die Schüttung in einer ersten Festbettkatalysatorschüttungszone mit einem hohen Anteil an inerten Verdünnungsverformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z. B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassenanteil steigert.

Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 1 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an die Festbettkatalysatorschüttung 1 können sich ausschließlich aus Inertmaterial (z. B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertmaterialschüttung verwendeten Verdünnungsformkörper die gleiche Geometrie wie die in der Festbettkatalysatorschüttung 1 verwendeten Katalysatorformkörper aufweisen. Die Geometrie der für die Inertmaterialschüttung verwendeten Verdünnungsformkörper kann aber auch von der vorgenannten Geometrie der Katalysatorformkörper verschieden sein (z. B. kugelförmig anstelle ringförmig).

Häufig weisen die für solche Inertmaterialschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertmaterialschüttungen erstrecken. Erfindungsgemäß vorteilhaft erfassen sowohl die Temperaturzone A als auch die Temperaturzone B jeweils nicht mehr als drei Festbettkatalysatorschüttungszonen (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 2) der Festbettkatalysatorschüttung 1 sollte (bei einheitlicher Aktivmasse über die gesamte Festbettkatalysatorschüttung 1) die volumenspezifische Aktivmasse (d. h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 1). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung 1 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß nicht mehr als 50 Gew.-%, vorzugsweise nicht mehr als 40 Gew.-% und in der Regel nicht mehr als 30 Gew.-% betragen.

Häufig wird beim erfindungsgemäßen Verfahren die Festbettkatalysatorschüttung 1 aus nur zwei Festbettkatalysatorschüttungszonen bestehen.

Erfindungsgemäß bevorzugt ist die in Strömungsrichtung des Reaktionsgasgemisches 2 letzte Festbettkatalysatorschüttungszone der Festbettkatalysatorschüttung 1 unverdünnt. D. h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist

Besteht die Festbettkatalysatorschüttung 1 nur aus zwei Festbettkatalysatorschüttungszonen, ist es erfindungsgemäß in der Regel vorteilhaft (wie ganz allgemein beim erfindungsgemäßen Verfahren), wenn die Festbettkatalysatorschüttungszone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D. h., in günstiger Weise wird die Festbettkatalysatorschüttungszone mit der geringeren volumenspezifischen Aktivität in die Temperaturzone B hineinragen und die Festbettkatalysatorschüttungszone mit der höheren volumenspezifischen Aktivität in der Temperaturzone B beginnen und enden. (d. h., hinter dem Übergang von der Temperaturzone A in die Temperaturzone B ihren Anfang haben.

Besteht die Festbettkatalysatorschüttung 1 nur aus drei Festbettkatalysatorschüttungszonen, ist es erfindungsgemäß in der Regel gleichfalls vorteilhaft, wenn die Festbettkatalysatorschüttungszone mit der höheren volumenspezifischen Aktivität nicht bis in die Temperaturzone A hineinragt sondern in der Temperaturzone B beginnt und endet, d. h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D. h., normalerweise wird in diesem Fall die Festbettkatalysatorschüttungszone mit der zweithöchsten volumenspezifischen Aktivität sowohl in die Temperaturzone A als auch in die Temperaturzone B hineinragen.

Besteht die Festbettkatalysatorschüttung 1 aus vier Festbettkatalysatorschüttungszonen, ist es erfindungsgemäß in der Regel vorteilhaft, wenn die Festbettkatalysatorschüttungszone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 2 strömt).

Im Fall einer Gleichstromführung von Reaktionsgasgemisch und Wärmeträgern in den Temperaturzonen A und B kann es vorteilhaft sein, wenn beim erfindungsgemäßen Verfahren innerhalb der Festbettkatalysatorschüttung 1 die Festbettkatalysatorschüttungszone mit der höchstens volumenspezifischen Aktivität in die Temperaturzone A hineinragt.

Generell lässt sich die volumenspezifische Aktivität zwischen zwei Festbettkatalysatorschüttungszonen einer Festbettkatalysatorschüttung 1 experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Propen enthaltende Reaktionsgasgemisch geführt wird. Die höhere umgesetzte Menge an Propen weist die höhere volumenspezifische Aktivität aus.

Beträgt die Gesamtlänge der Festbettkatalysatorschüttung 1 $L^1$, ist es erfindungsgemäß vorteilhaft, wenn sich im Bereich $X^1 \pm L^1 \dfrac{4}{100}$ bzw. im Bereich $X^1 \pm L^1 \dfrac{3}{100}$ bzw. im Bereich $X^1 \pm L^1 \dfrac{2}{100}$ kein Übergang von einer Festbettkatalysatorschüttungszone in eine andere Festbettkatalysatorschüttungszone befindet, wobei X der Ort (die Stelle)

innerhalb der Festbettkatalysatorschüttung 1 ist, an dem der Übergang von der Temperaturzone A in die Temperaturzone B erfolgt.

Bevorzugt ist beim vorstehend beschrieben Verfahren die Festbettkatalysatorschüttung 1 in Strömungsrichtung des Reaktionsgasgemisches 2 wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d. h. z. B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung befindet sich dann vorteilhaft bis zum Ende der Länge der Festbettkatalysatorschüttung (d. h., z. B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der am Ende der Festbettkatalysatorschüttung 1 gegebenenfalls verwendeten Schalenkatalysatorformkörper.

Eine reine Inertmaterialschüttung, deren Länge, bezogen auf die Länge der Festbettkatalysatorschüttung 1, zweckmäßig 5 bis 20 % beträgt, leitet in Strömungsrichtung des Reaktionsgasgemisches 2 in der Regel die Festbettkatalysatorschüttung 1 ein. Sie wird normalerweise als Aufheizzone für das Reaktionsgasgemisch 2 genutzt. Anstelle der Inertmaterialschüttung kann als Aufheizzone aber auch ein mit Inertmaterial verdünntes Katalysatorfestbett verwendet werden.

Erfindungsgemäß vorteilhaft erstreckt sich nun bei den vorgenannten Festbettkatalysatorschüttungen 1 die Festbettkatalysatorschüttungszone mit der geringeren volumenspezifischen Aktivität noch auf 5 bis 20 %, häufig auf 5 bis 15 % ihrer Länge in die Temperaturzone B.

Zweckmäßig erstreckt sich die Temperaturzone A auch auf eine zur Festbettkatalysatorschüttung 1 gegebenenfalls angewandte Vorschüttung aus Inertmaterial.

Für die Vorteilhaftigkeit der beschriebenen Verfahrensweise wesentlich ist ferner, dass die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 3 beim Übergang von einer Festbettkatalysatorschüttungszone in eine andere Festbettkatalysatorschüttungszone sprunghaft zunimmt.

Die volumenspezifische (d. h., die auf die Einheit des jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z. B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z. B. Steatit C-220 der Fa. Cerm Tec) in Betracht.

Die Geometrie solcher inerter Verdünnungsformkörper kann im Prinzip beliebig sein. D. h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung 2 nicht verändert. D.h., die für einen einzelnen Katalysatorformkörper verwendete Aktivmasse kann zwar ein Gemisch aus verschiedenen, die Elemente Mo und V enthaltenden, Multimetalloxiden sein, für alle Katalysatorformkörper der Festbettkatalysatorschüttung 2 ist dann jedoch das gleiche Gemisch zu verwenden.

Eine in Strömungsrichtung des Reaktionsgasgemisches 3 über die Festbettkatalysatorschüttung 2 zonenweise zunehmende volumenspezifische Aktivität lässt sich für das beschriebene Verfahren somit in einfacher Weise z. B. dadurch einstellen, dass man die Schüttung in einer ersten Festbettkatalysatorschüttungszone mit einem hohen Anteil an inerten Verdünnungsformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z. B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z. B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 2 aber auch Mischungen aus Katalysatoren mit chemisch unterschiedlicher Aktivzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an die Festbettkatalysatorschüttung 2 können sich ausschließlich aus Inertmaterial (z. B. nur Verdünnungsformkörpern) bestehende Schüttungen befinden (sie werden in dieser Schrift begrifflich nicht der Festbettkatalysatorschüttung 2 zugerechnet, da sie keine Formkörper enthalten, die Multimetalloxidaktivmasse aufweisen). Dabei können die für die Inertmaterialschüttung verwendeten Verdünnungsformkörper die gleich Geometrie wie die in der Festbettkatalysatorschüttung verwendeten Verdünnungsformkörper aufweisen. Die Geometrie der für die Inertmaterialschüttung verwendeten Verdünnungsformkörper kann aber auch von der vorgenannten Geometrie der Katalysatorformkörper verschieden sein (z. B. kugelförmige anstatt ringförmig).

Häufig weisen die für solche Inertmaterialschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzone C und D können sich beim erfindungsgemäßen Verfahren auch auf die Inertmaterialschüttungen erstrecken. Erfindungsgemäß vorteilhaft erfassen sowohl die Temperaturzone C als auch die Temperaturzone D jeweils nicht mehr als drei Festbettkatalysatorschüttungszonen (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung 2 nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 3) sollte (bei einheitlicher Aktivmasse über die gesamte Festbettkatalysatorschüttung 2) die volumenspezifische Aktivmasse (d. h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 2). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung 2 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß nicht mehr als 50 Gew.-%, vorzugsweise nicht mehr als 40 Gew.-% und besonders bevorzugt nicht mehr als 30 Gew.-% betragen.

Häufig wird beim erfindungsgemäßen Verfahren die Festbettkatalysatorschüttung 2 aus nur zwei Festbettkatalysatorschüttungszonen bestehen.

Erfindungsgemäß bevorzugt ist die in Strömungsrichtung des Reaktionsgasgemisches 3 letzte Festbettkatalysatorschüttungszone der Festbettkatalysatorschüttung 2 unverdünnt. D. h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist. Besteht die Festbettkatalysatorschüttung 2 nur aus zwei Festbettkatalysatorschüttungszonen, ist es erfindungsgemäß in der Regel vorteilhaft (wie ganz allgemein beim erfindungsgemäßen Verfahren), wenn die Festbettkatalysatorschüttungszone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt).

Besteht die Festbettkatalysatorschüttung 2 nur aus drei Festbettkatalysatorschüttungszonen, ist es erfindungsgemäß in der Regel gleichfalls vorteilhaft, wenn die Festbettkatalysatorschüttungszone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt).

Besteht die Festbettkatalysatorschüttung 2 aus vier Festbettkatalysatorschüttungszonen, ist es erfindungsgemäß in der Regel vorteilhaft, wenn die Festbettkatalysatorschüttungszone mit der zweithöchsten volumenspezifischen Aktivität sowohl in die Temperaturzone C als auch in die Temperaturzone D hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt.

Im Fall einer Gleichstromführung von Reaktionsgasgemisch 3 und Wärmeträgern in den Temperaturzonen C und D kann es erfindungsgemäß vorteilhaft sein, wenn innerhalb der Festbettkatalysatorschüttung 2 die Festbettkatalysatorschüttungszone mit der höchsten volumenspezifischen Aktivität nicht in die Temperaturzone C hineinragt, sondern erst hinter dem Übergang von der Temperaturzone C in die Temperaturzone D ihren Anfang hat.

Die volumenspezifische Aktivität zwischen zwei Festbettkatalysatorschüttungszonen innerhalb der Festbettkatalysatorschüttung 2 lässt sich experimentell in einfacher Weise so differenzieren, dass unter identischen Randbedingungen (vorzugsweise den Bedingungen des ins Auge gefassten Verfahrens) über Festbettkatalysatorschüttungen der selben Länge, aber jeweils der Zusammensetzung der jeweiligen Festbettkatalysatorschüttungszone entsprechend, das gleiche Acrolein enthaltende Reaktionsgasausgangsgemisch geführt wird. Die höhere umgesetzte Menge an Acrolein weist die höhere volumenspezifische Aktivität aus.

Beträgt die Gesamtlänge der Festbettkatalysatorschüttung 2 $L^2$, ist es erfindungsgemäß vorteilhaft, wenn sich im Bereich $X^2 \pm L^2 \dfrac{4}{100}$ bzw. im Bereich $X^2 \pm L^2 \dfrac{3}{100}$ bzw. im Bereich $X^2 \pm L^2 \dfrac{2}{100}$ kein Übergang von einer Festbettkatalysatorschüttungszone in eine andere Festbettkatalysatorschüttungszone befindet, wobei X der Ort innerhalb der Festbettkatalysatorschüttung 2 ist, an dem der Übergang von der Temperaturzone C in die Temperaturzone D erfolgt.

Bevorzugt ist beim vorstehend beschriebenen Verfahren die Festbettkatalysatorschüttung 2 in Strömungsrichtung des Reaktionsgasgemisches 3 wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der Fest-bettkatalysatorschüttung 2, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleich Geometrie aufweisen), wobei der Anteil der Verdünnungsformkörper so bemessen ist, dass die volumenspezifische Aktivmasse, bezogen auf eine nur aus den Katalysatorformkörpern bestehende Schüttung, um 10 bis 50 Gew.-%, vorzugsweise 20 bis 45 Gew.-% und besonders bevorzugt 25 bis 35 Gew.-% abgesenkt ist. Im Anschluss an diese erste bzw. an diese beiden ersten Zonen befindet sich dann erfindungsgemäß vorteilhaft bis zum Ende der Länge der Festbettkatalysatorschüttung 2 (d. h., z. B. auf einer Länge von 2,00 bis 3,00 m, bevorzugt 2,50 bis 3,00 m) entweder eine nur in geringerem Umfang (als in der ersten bzw. in den ersten beiden Zonen) verdünnte Schüttung der Katalysatorformkörper, oder, ganz besonders bevorzugt, eine alleinige Schüttung derselben Katalysatorformkörper, die auch in den ersten Zonen verwendet worden sind.

Das Vorgenannte trifft insbesondere dann zu, wenn in der Festbettkatalysatorschüttung 2 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der Schalenkatalysatorformkörper am Ende der Festbettkatalysatorschüttung.

Eine reine Inertmaterialschüttung, deren Länge, bezogen auf die Länge der Festbettkatalysatorschüttung 2, zweckmäßig 5 bis 20 % beträgt, leitet in Strömungsrichtung des Reaktionsgasgemisches 3 in der Regel die Festbettkatalysatorschüttung 2 ein. Sie dient normalerweise dem Zweck der Temperierung des Reaktionsgasgemisches 3. Anstelle der Inertmaterialschüttung kann als Aufheizzone aber auch ein mit Inertmaterial verdünntes Katalysatorfestbett verwendet werden.

Erfindungsgemäß vorteilhaft erstreckt sich nun bei den vorgenannten Festbettkatalysatorschüttungen 2 die Temperaturzone C (die sich erfindungsgemäß vorteilhaft auch auf die Vorschüttung aus Inertmaterial erstreckt) noch auf 5 bis 20 %, häufig auf 5 bis 15 % der Länge der in Strömungsrichtung des Reaktionsgasgemisches 3 letzten (volumenspezifisch aktivsten) Festbettkatalysatorschüttungszone der Festbettkatalysatorschüttung 2.

In anwendungstechnisch zweckmäßiger Weise erfolgt die Durchführung der ersten Reaktionsstufe des vorstehend beschriebenen Verfahrens in einem Zweizonenrohrbündelreaktor, wie er z. B. in den DE-A's 199 10 508, 199 48 523, 199 10 506 und 199 48 241 beschrieben ist. Eine bevorzugte Variante eines erfindungsgemäß einsetzbaren Zweizonenrohrbündelreaktors offenbart die DE-C 28 30 765. Aber auch die in der DE-C 25 13 405, der US-A 3,147,084, der DE-A 22 01 528, der EP-A 38 32 24 und der DE-A 29 03 218 offenbarten Zweizonenrohrbündelreaktoren sind für eine Durchführung der ersten Reaktionsstufe des vorstehend beschriebenen Verfahrens geeignet.

D. h., in einfachster Weise befindet sich die zu verwendende Festbettkatalysatorschüttung 1 (eventuell mit vor- und/oder nachangeordneten Inertmaterialschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D. h., in einfachster Weise umströmt z. B. ein Salzbad A denjenigen Abschnitt der Rohre (die Temperaturzone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Temperaturzonen A, B weitere Reaktionszonen anschließen, die auf individuellen Temperaturen gehalten werden).

Anwendungstechnisch zweckmäßig umfasst die erste Reaktionsstufe des beschriebenen Verfahrens keine weiteren Temperaturzonen. D. h., das Salzbad B umströmt zweckmäßig den Abschnitt der Rohre, in welchem sich die oxidative Anschlussumsetzung des Propens (beim einfachen Durchgang) bis zu einem Umsatzwert $\geq$ 90 mol-%, oder $\geq$ 92 mol-% oder $\geq$ 94 mol-% oder mehr vollzieht.

Üblicherweise liegt der Beginn der Temperaturzone B hinter dem Heißpunktmaximum der Temperaturzone A.

Die beiden Salzbäder A, B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700 714 oder in der EP-A 700 893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird beim beschriebenen Verfahren das Reaktionsgasausgangsgemisch 2 der Festbettkatalysatorschüttung 1 auf die Reaktionstemperatur vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung 1 wenigstens 60 % bzw. wenigstens 75 %, oder wenigstens 90 % der Länge der Zone. Die gegebenenfalls verbleibende Restlänge wird gegebenenfalls von einer Inertmaterialschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z. B. EP-B 468 290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die Temperaturzone bis zur Austrittstelle aus der Temperaturzone (bedingt durch die Exothermie der Reaktion) um 0 bis 15°C ansteigt. D. h., das vorgenannte ΔT kann erfindungsgemäß 1 bis 10°C, oder 2 bis 8°C oder 3 bis 6°C betragen.

Die Eintrittstemperatur des Wärmeaustauschmittels in die Temperaturzone A liegt erfindungsgemäß normalerweise im Bereich von 290 bis 380°C, bevorzugt im Bereich von 305 bis 365°C und besonders bevorzugt im Bereich von 310 bis 340°C bzw. bei 330°C. Bei Propenbelastungen der Festbettkatalysatorschüttung 1 von ≥ 90 Nl/l·h und ≤ 160 Nl/l·h wird die Eintrittstemperatur des Wärmeaustauschmittels in die Temperaturzone B erfindungsgemäß ebenfalls im Bereich von 290 bis 380°C, gleichzeitig aber normalerweise erfindungsgemäß zweckmäßig ≥ 0°C bis ≤ 20°C, oder ≤ 10°C, bzw. ≥ 0°C und ≤ 5°C, oder häufig ≥ 0°C und ≤ 3°C unterhalb der Eintrittstemperatur des in die Temperaturzone A eintretenden Wärmeaustauschmittels liegen. Bei Propenbelastungen der Festbettkatalysatorschüttung 1 von ≥ 160 Nl/l·h und (in der Regel) ≤ 300 Nl/l·h (bzw. 600 Nl/l·h) wird die Eintrittstemperatur des Wärmeaustauschmittels in die Temperaturzone B erfindungsgemäß ebenfalls im Bereich von 290 bis 380°C, aber normalerweise erfindungsgemäß zweckmäßig ≥ 0°C bis ≤ 50°C, oder ≥ 5°C und ≤ 45°C, oder ≥ 10°C und ≤ 40°C, oder ≥ 15°C und ≤ 30°C oder ≤ 35°C (z. B. 20°C oder 25°C) oberhalb der Eintrittstemperatur des in die Temperaturzone A eintretenden Wärmeaustauschmittels liegen.

Es sei an dieser Stelle auch noch einmal darauf hingewiesen, dass für eine Durchführung der Reaktionsstufe 1 des erfindungsgemäßen Verfahrens insbesondere auch der in der DE-AS 22 01 528 beschriebene Zweizonenrohrbündelreaktortyp verwendet werden kann, der die Möglichkeit beinhaltet, vom heißeren Wärmeaustauschmittel der Temperaturzone B eine Teilmenge an die Temperaturzone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Temperaturzone wie in der EP-A 382 098 beschrieben gestaltet werden. Im Übrigen hat es sich als zweckmäßig erwiesen, das die erste Reaktionsstufe verlassende Produktgasgemisch vor dem Eintritt in die zweite Reaktionsstufe auf direkte und/oder indirekte Weise abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z. B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

In der Regel wird der auf den einfachen Durchgang bezogene Propenumsatz beim erfindungsgemäßen Verfahren in der ersten Reaktionsstufe ≥ 92 mol-% oder ≥ 94 mol-% betragen. Die in der ersten Reaktionsstufe bei einfachem Durchgang resultierende Selektivität der Acroleinbildung sowie der Acrylsäurenebenproduktbildung wird dabei erfindungsgemäß zusammen regelmäßig ≥ 92 mol-% oder ≥ 94 mol-%, häufig ≥ 95 mol-% oder ≥ 96 mol-% bzw. ≥ 97 mol-% betragen.

Anwendungstechnisch zweckmäßig wird das Produktgasgemisch der ersten Reaktionsstufe im bereits erwähnten Nachkühler auf eine Temperatur von 210 bis 290°C, häufig 230 bis 280°C oder 250 bis 270°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Vorteilhaft wird das beschriebene zweistufige Verfahren ferner so verwirklicht, dass man den Sauerstoffbedarf in der zweiten Reaktionsstufe nicht bereits durch einen entsprechend hohen Sauerstoffgehalt des Reaktionsgasausgangsgemisches 2 deckt, sondern den benötigten Sauerstoff im Bereich zwischen erster und zweiter Reaktionsstufe zugibt ("Sekundärsauerstoffzusatz"). Dies kann vor, während, nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z. B. Luft (ist erfindungsgemäß bevorzugt) oder an molekularem Stickstoff entreicherte Luft (z. B. ≥ 90 Vol-% O₂, ≤ 10 Vol-% N₂) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der zweiten Reaktionsstufe bereits durch einen entsprechend hohen Sauerstoffbedarf in der ersten Reaktionsstufe gedeckt werden. Natürlich kann bei Bedarf als Sekundärgas auch ein inertes Verdünnungsgas zugesetzt werden.

Wie die Durchführung der ersten Reaktionsstufe erfolgt auch die Durchführung der zweiten Reaktionsstufe des erfindungsgemäßen Verfahrens in anwendungstechnisch zweckmäßiger Weise in einem Zweizonenrohrbündelreaktor, wie er für die erste Reaktionsstufe bereits beschrieben wurde. Die Ausführungen hinsichtlich des Zweizonenrohrbündelreaktors für die erste Reaktionsstufe gelten deshalb auch für den Zweizonenrohrbündelreaktor für die zweite Reaktionsstufe.

D. h., in einfacher Weise befindet sich die erfindungsgemäß zu verwendende Festbettkatalysatorschüttung 2 (gegebenenfalls einschließlich der Inertmaterialschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone.

D. h., in einfacher Weise umströmt z. B. ein Salzbad C diejenigen Abschnitte der Rohre (die Temperaturzone C), in welchem sich die oxidative Umsetzung des Acroleins (beim einfachen Durchgang) bis zum Erreichen eines Umsatzwertes im Bereich von 45 bis 85 mol-% (bevorzugt 50 bis 85 mol-%, besonders bevorzugt 60 bis 85 mol-%) vollzieht und ein Salzbad D umströmt den Abschnitt der Rohre (die Temperaturzone D), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Temperaturzonen C, D weitere Temperaturzonen anschließen, die auf individuellen Temperaturen gehalten werden).

Anwendungstechnisch zweckmäßig umfasst die Reaktionsstufe 2 des erfindungsgemäßen Verfahrens keine weiteren Temperaturzonen. D. h., das Salzbad D umströmt zweckmäßig den Abschnitt der Rohre, in welchem sich die oxidative Anschlussumsetzung des Acroleins (beim einfachen Durchgang) bis zu einem Umsatzwert von $\geq$ 92 mol-%, oder $\geq$ 94 mol-% oder $\geq$ 96 mol-% oder $\geq$ 98 mol-% und häufig sogar $\geq$ 99 mol-% oder mehr vollzieht.

Üblicherweise liegt der Beginn der Temperaturzone D hinter dem Heißpunktmaximum der Temperaturzone C

Die beiden Salzbäder C, D können erfindungsgemäß relativ zur Strömungsrichtung des durch die Reaktionsrohre strömenden Reaktiongasgemisches im Gleichstrom oder im Gegenstrom durch den die Reaktionsrohre umgebenden Raum geführt werden. Selbstverständlich kann erfindungsgemäß auch in der Temperaturzone C eine Gleichströmung und in der Temperaturzone D eine Gegenströmung (oder umgekehrt) angewandt werden.

Selbstredend kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700 714 oder in der EP-A 700 893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren für die zweite Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertmaterialschüttung belegt. Anwendungstechnisch zweckmäßig beläuft sich die im Rohrbün-delbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der zweiten Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Temperaturzone bis zur Austrittstelle aus der Temperaturzone um 0 bis 15°C ansteigt. D. h., das vorgenannte $\Delta T$ kann erfindungsgemäß 1 bis 10°C, oder 2 bis 8°C oder 3 bis 6°C betragen.

Die Eintrittstemperatur des Wärmeaustauschmittels in die Temperaturzone C liegt erfindungsgemäß normalerweise im Bereich von 230 bis 320°C, bevorzugt im Bereich von 250 bis 300°C und besonders bevorzugt im Bereich von 260 bis 280°C. Bei Acro-leinbelastungen der Festbettkatalysatorschüttung 2 von $\geq$ 70 Nl/l·h und $\leq$ 150 Nl/l·h wird die Eintrittstemperatur des Wärmeaustauschmittels in die Temperaturzone D erfindungsgemäß ebenfalls im Bereich von 230 bis 320°C, gleichzeitig aber normalerweise erfindungsgemäß zweckmäßig $\geq$ 0°C bis $\leq$ 20°C oder $\leq$ 10°C, bzw. $\geq$ 0°C und $\leq$ 5°C, oder häufig $\geq$ 0°C und $\leq$ 3°C unterhalb der Eintrittstemperatur des in die Temperaturzone C eintretenden Wärmeaustauschmittels liegen. Bei Acroleinbelastung der Festbettkatalysatorschüttung von $\geq$ 150 Nl/l·h und (in der Regel) $\leq$ 300 Nl/l·h (bzw. 600 Nl/l·h) wird die Eintrittstemperatur des Wärmeaustauschmittels in die Temperaturzone D erfindungsgemäß ebenfalls im Bereich von 230 bis 320°C, gleichzeitig aber normalerweise erfindungsgemäß zweckmäßig $\geq$ 0°C bis $\leq$ 40°C, oder $\geq$ 5°C und $\leq$ 35°C, bzw. 30°C, oder $\geq$ 10°C und $\leq$ 25°C, bzw. 20°C, oder 15°C oberhalb der Eintrittstemperatur des in die Temperaturzone C eintretenden Wärmeaustauschmittels liegen.

Es sei an dieser Stelle auch noch einmal darauf hingewiesen, dass für eine Durchführung der zweiten Reaktionsstufe des beschriebenen Verfahrens insbesondere auch der in der DE-AS 22 01 528 beschriebene Zweizonenrohrbündelreaktortyp verwendet werden kann, der die Möglichkeit beinhaltet, vom heißeren Wärmeaustauschmittel der Temperaturzone D eine Teilmenge an die Temperaturzone C abzuführen, um gegebenenfalls ein Anwärmen eines zu kalten Reaktionsgasausgangsgemisches 3 zu bewirken. Ferner kann die Rohrbündelcharakteristik, innerhalb einer individuellen Reaktionszone wie in der EP-A 382 098 beschrieben gestaltet werden.

Selbstredend können zur Durchführung des beschriebenen Verfahrens zwei Zweizonenrohrbündelreaktoren zu einem Vierzonenrohrbündelreaktor verschmolzen werden, wie es in der WO 01/36364 beschrieben ist. Normalerweise befindet sich in diesen Fällen zwischen der Festbettkatalysatorschüttung 1 und der Festbettkatalysatorschüttung 2 eine Inertmaterialschüttung. Allerdings kann auf eine solche Zwischeninertschüttung auch verzichtet werden. Die Länge der Reaktionsrohre entspricht im Verschmelzungsfall vielfach der Summe der Längen der nicht verschmolzenen Rohrbündelreaktoren.

An dieser Stelle sei noch festgehalten, dass als Aktivmassen sowohl für die Festbettkatalysatorschüttung 1 als auch für die Festbettkatalysatorschüttung 2 auch die Multimetalloxidmassen der DE-A 102 61 186 günstig sind.

Günstige Ausgestaltungen eines Zweizonenrohrbündelreaktors für die erste Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

Kontaktrohre:

| | |
|---|---|
| Material der Kontaktrohre: | ferritischer Stahl; |
| Abmessungen der Kontaktrohre: | z. B. 3500 mm Länge; |
| | z. B. 30 mm Außendurchmesser; |
| | z. B. 2 mm Wandstärke; |

Anzahl der Kontaktrohre im Rohrbündel: z. B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z. B. der selben Länge und Wandstärke, aber mit einem Außendurchmesser von z. B. 33,4 mm und einer zentrierten Thermohülse von z. B. 10 mm Außendurchmesser und z. B. 1 mm Wandstärke;

Reaktor (Material wie die Kontaktrohre):

Zylinderförmiger Behälter eines Innendurchmessers von 6000 - 8000 mm;

Reaktorhauben plattiert mit Edelstahl des Typs 1,4541; Plattierungsdicke: einige mm;

ringförmig angeordnetes Rohrbündel, z. B. mit einem freien zentralen Raum;

Durchmesser des zentralen freien Raumes: z. B. 1000 - 2500 mm (z. B. 1200 mm, oder 1400 mm, oder 1600 mm, oder 1800 mm, oder 2000 mm, oder 2200 mm, oder 2400 mm);

normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z. B. 36 mm, oder 38 mm, oder 40 mm, oder 42 mm, oder 44 mm;

die Kontaktrohre sind mit ihren Enden in Kontaktrohrböden (oberer Boden und unterer Boden z. B. mit einer Dicke von 100 - 200 mm) abdichtend befestigt und münden am oberen Ende in eine mit dem Behälter verbundene Haube, die einen Zulass für das Reaktionsgasausgangsgemisch 2 aufweist; ein z. B. auf der halben Kontaktrohrlänge befindliches Trennblech einer Dicke von 20 - 100 mm, teilt den Reaktorraum symmetrisch in zwei Temperaturzonen A (obere Zone) und B (untere Zone); jede Temperaturzone wird durch eine Umlenkscheibe in 2 äquidistante Längsabschnitte geteilt;

die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;

jede Zone wird durch eine eigene Salzpumpe mit Salzschmelze als Wärmeträger versorgt; die Zufuhr der Salzschmelze ist z. B. unterhalb der Umlenkscheibe und die Entnahme ist z. B. oberhalb der Umlenkscheibe;

aus beiden Salzschmelzekreisen wird z. B. ein Teilstrom entnommen und z. B. in einem gemeinsamen oder zwei getrennten indirekten Wärmetauschern abgekühlt (Dampferzeugung);

im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;

durch im Reaktormantel befindliche Fenster gelangt die Salzschmelze zum Rohrbündel; die Einströmung erfolgt z. B. in radialer Richtung zum Rohrbündel;

die Salzschmelze fließt in jeder Zone der Vorgabe des Umlenkblechs folgend z. B. in der Abfolge

- von außen nach innen,
- von innen nach außen,

um die Kontaktrohre;

durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;

über jede Temperaturzone wird die Salzschmelze von unten nach oben geführt;

Das Reaktionsgasgemisch verlässt den Reaktor der ersten Stufe mit einer Temperatur wenige Grad höher als die Salzbadeintrittstemperatur des ersten Reaktors. Das Reaktionsgasgemisch wird für die Weiterverarbeitung zweckmäßigerweise in einem separaten Nachkühler, der dem Reaktor der 1. Stufe nachgeschaltet ist, auf 220°C bis 280°C, bevorzugt 240°C bis 260°C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 m$^3$/h betragen.

Stromführung:

das Reaktionsgasausgangsgemisch 2 strömt zweckmäßig von oben nach unten durch den Erststufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z. B.:

Abschnitt 1: 50 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3: 160 cm Länge

Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9 \times 2WO_3]_{0,5} [Mo_{12}Co_{5,5}Fe_{2,94}Si_{1,59}K_{0,08}O_x]_1$).

Günstige Ausgestaltungen eines Zweizonenrohrbündelreaktors für die zweite Reaktionsstufe können wie folgt beschaffen sein:

Alles wie beim Zweizonenrohrbündelreaktor für die erste Reaktionsstufe. Die Dicke des oberen und unteren Kontaktrohrbodens beträgt jedoch häufig 100 - 200 mm, z. B. 110 mm, oder 130 mm, oder 150 mm, oder 170 mm, oder 190 mm.

Der Nachkühler entfällt; statt dessen münden die Kontaktrohre mit ihren unteren Öff-nungen in eine am unteren Ende mit dem Behälter verbundene Haube mit Auslaß für das Produktgasgemisch; die obere Temperaturzone ist die Zone C und die untere Temperaturzone ist die Temperaturzone D. Zwischen Auslaß "Nachkühler" und Einlass "Reaktor für die zweite Reaktionsstufe" besteht zweckmäßig eine Zuführmöglichkeit für komprimierte Luft.

Die Kontaktrohr- und Thermorohrbeschickung (von oben nach unten) kann z. B. wie folgt sein:

Abschnitt 1: 20 cm Länge

Steatitringe der Geometrie 7 mm x 7 mm x 4 mm (Außendurchmesser x Länge x Innendurchmesser) als Vorschüttung.

Abschnitt 2: 90 cm Länge

Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.

Abschnitt 3: 50 cm Länge

Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 100 46 928 (Stöchiometrie: $Mo_{12}V_3W_{1,2}Cu_{2,4}O_x$).

Die Zweitstufen-Kontaktrohr- und Thermorohrbeschickung kann (von oben nach unten) auch so aussehen:

Abschnitt 1: 20 cm Länge

Steatitringe der Geometrie 7 mm x 7 mm x 4 mm (Außendurchmesser x Länge x Innendurchmesser) als Vorschüttung.

Abschnitt 2: 140 cm Länge

Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.

Abschnitt 3: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 100 46 928 (Stöchiometrie: $Mo_{12}V_3W_{1,2}Cu_{2,4}O_x$).

In der genannten Erststufenbeschickung kann der Vollkatalysator aus Beispiel 1 der DE-A 100 46 957 auch ersetzt werden durch:

a) einen Katalysator gemäß Beispiel 1 c aus der EP-A 15 565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x \cdot 10\ SiO_2$ aufweist;

b) Beispiel Nr. 3 aus der DE-A 198 55 913 als Hohlzylindervollkatalysator der Geometrie 5 mm x 3 mm x 2 mm bzw.

5 mm x 2 mm x 2 mm;

c) Multimetalloxid II - Vollkatalysator gemäß Beispie 1 der DE-A 197 46 210;

d) einen der Schalenkatalysatoren 1, 2 und 3 aus der DE-A 100 63 162, jedoch bei gleicher Schalendicke auf Trägerringe der Geometrie 5 mm x 3 mm x 1,5 mm bzw. 7 mm x 3 mm x 1,5 mm aufgebracht;

e) die Katalysatoren, insbesondere die Ausführungsbeispiele, der DE-A 10344149 und der DE-A 10353954.

In allen vorgenannten Zweitstufenbeschickungen kann der Schalenkatalysator gemäß Herstellungsbeispiel 5 der DE-A 100 46 928 ersetzt werden durch:

a) Schalenkatalysator S1 oder S7 aus der DE-A 4442346 mit einem Aktivmassenanteil von 27 Gew.-% und einer Schalendicke von 230 $\mu$m;

b) einem Schalenkatalysator gemäß den Beispielen 1 bis 5 aus der DE-A 198 15 281, jedoch auf Trägerringe der Geometrie 7 mm x 3 mm x 4 mm mit einem Aktivmassenanteil von 20 Gew.-% aufgebracht;

c) Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, hergestellt gemäß DE-A 197 36 105 und mit einem Aktivmassenanteil von 20 Gew.-% auf den vorgenannten 7 mm x 3 mm x 4 mm Träger aufgebracht.

Im Übrigen werden die Festbettkatalysatorschüttung 1 und die Festbettkatalysatorschüttung 2 erfindungsgemäß zweckmäßig so gewählt (z. B. durch Verdünnung mit z. B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperaturunterschied $\leq$ 70°C, häufig liegt er bei 20 bis 70°C, vorzugsweise ist dieser Temperaturunterschied gering. Außerdem werden die Festbettkatalysatorschüttungen 1 und 2 aus Sicherheitsgründen in dem Fachmann an sich bekannter Weise so gewählt (z. B. durch Verdünnung mit z. B. Inertmaterial), dass die "peak-to-salttemperature sensitivity" gemäß Definition in der EP-A 1106598 $\leq$ 9°C, oder $\leq$ 7°C, oder $\leq$ 5°C, oder $\leq$ 3°C beträgt.

Nachkühler und Reaktor für die zweite Stufe sind durch ein Verbindungsrohr verbunden, dessen Länge weniger als 25 m beträgt.

In den in dieser Schrift enthaltenen zweizonig ausgeführten Beispielen sowie in der vorstehenden Reaktoranordnung können in der zweiten Reaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden.

Das die erfindungsgemäße Partialoxidation (nach der ersten und/oder zweiten Reaktionsstufe) verlassende Produktgasgemisch (hier Produktgasgemisch 2 (nach erster Reaktionsstufe) bzw. 3(nach zweiter Reaktionsstufe) genannt) ist im Fall einer Herstellung von Acrolein und/oder Acrylsäure in der Regel im wesentlichen zusammengesetzt aus dem Zielprodukt Acrolein oder Acrylsäure oder dessen Gemisch mit Acrolein, nicht umgesetztem molekularem Sauerstoff (mit Blick auf die Lebensdauer der verwendeten Katalysatoren ist es günstig, wenn der Sauerstoffgehalt sowohl im Produktgasgemisch 2 als auch im Produktgasgemisch 3 noch wenigstens 1,5 bis 4 Vol.-% beträgt), Propan, nicht umgesetztem Propylen, molekularem Stickstoff, als Nebenprodukt entstandenem und/oder als Verdünnungsgas mitverwendetem Wasserdampf, als Nebenprodukt entstandenen und/oder als Verdünnungsgas mitverwendeten Kohlenoxiden, sowie geringen Mengen sonstiger niederer Aldehyde, niederer Alkancarbonsäuren (z. B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiterer Kohlenwasserstoffen, wie z. B. C4-Kohlenwasserstoffe (z. B. Buten-1 und eventuell sonstige Butene), und anderen inerten Verdünnungsgasen.

Das Zielprodukt kann aus dem Produktgasgemisch 3 bzw. 2 in an sich bekannter Weise in einer Trennzone abgetrennt werden (z. B. durch partielle oder vollständige sowie gegebenenfalls fraktionierende Kondensation der Acrylsäure oder durch Absorption von Acrylsäure in Wasser oder in einem hochsiedenden hydrophoben organischen Lösungsmittel oder durch Absorption von Acrolein in Wasser oder in wässrigen Lösungen niederer Carbonsäuren sowie anschließende Aufarbeitung der Kondensate und/oder Absorbate; erfindungsgemäß bevorzugt wird das Produktgasgemisch 3 bzw. 2 fraktionierend kondensiert werden; vgl. z. B. EP-A 13 88 533, EP-A 13 88 532, DE-A 102 35 847, EP-A 79 28 67, WO 98/01415, EP-A 10 15 411, EP-A 1015 410, WO 99/50219, WO 00/53560, WO 02/09839, DE-A 102 35 847, WO 03/041833, DE-A 102 23 058, DE-A 102 43 625, DE-A 103 36 386, EP-A 85 41 29, US-A 4,317,926, DE-A 198 37 520, DE-A 196 06 877, DE-A 190 50 1325, DE-A 102 47 240, DE-A 197 40 253, EP-A 69 57 36, EP-A 98 22 87, EP-A 10 41 062, EP-A 11 71 46, DE-A 43 08 087, DE-A 43 35 172, DE-A 44 36 243, DE-A 19 924 532, DE-A 103 32 758 sowie DE-A 19 924 533). Eine Acrylsäureabtrennung kann auch wie in der EP-A 98 22 87, der EP-A 98 22 89, der DE-A 103 36 386, der DE-A 101 15 277, der DE-A 196 06 877, der DE-A 197 40 252, der DE-A 196 27 847, der EP-A 92 04 08, der EP-A 10 68 174, der EP-A 10 66 239, der EP-A 10 66 240, der WO 00/53560, der WO 00/53561, der DE-A 100 53 086 und der EP-A 98 22 88 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 2004/063138, WO 2004/035514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Gemeinsames Merkmal der vorgenannten Trennverfahren ist (wie eingangs bereits erwähnt), dass am Kopf der jeweiligen trennwirksame Einbauten enthaltenden Trennkolonne, in deren unteren Teil das Produktgemisch 3 bzw. 2, normalerweise nach vorheriger direkter und/oder indirekter Kühlung desselben, zugeführt wird, normalerweise ein Restgasstrom verbleibt, der im wesentlichen diejenigen Bestandteile des Produktgasgemischs 2 bzw. 3 enthält, deren Siedepunkt bei Normaldruck (1 bar) $\leq$ -30°C beträgt (d. h., die schwer kondensierbaren oder auch leicht flüchtigen Bestandteile).

Im unteren Teil der Trennkolonne fallen normalerweise die schwerer flüchtigen Bestandteile des Produktgasgemischs 3 bzw. 2, einschließlich des jeweiligen Zielprodukts, in kondensierter Phase an.

Die Restgasbestandteile sind in erster Linie Propan, gegebenenfalls in der Partialoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein.

Von diesem Hauptgasrest wird erfindungsgemäß bevorzugt wenigstens eine (vorzugsweise Restgaszusammensetzung aufweisende) Propan, molekularen Sauerstoff und gegebenenfalls nicht umgesetztes Propylen enthaltende Teilmenge (vorzugsweise die Gesamtmenge, gegebenenfalls aber auch nur die Hälfte, oder zwei Drittel, oder drei Viertel dieser Gesamtmenge) als ein Propan enthaltender Zufuhrstrom in die gegebenenfalls als Propenquelle dienende heterogen katalysierte Propandehydrierung und/oder Propanoxidehydrierung rückgeführt (Oxidationskreisgas). Restgasteilmengen können aber auch in eine oder in beide Stufen der Partialoxidation rückgeführt und/oder zum Zweck der Energieerzeugung verbrannt werden.

Bei der Aufarbeitung der kondensierten Phase (zum Zweck der Abtrennung des Zielproduktes) können weitere restliche Gase anfallen, da normalerweise versucht werden wird, die insgesamt im Produktgasgemisch 3 bzw. 2 enthaltene Menge an nicht umgesetztem Propan in die gegebenenfalls als Propylenquelle dienende heterogen katalysierte Propandehydrierung und/oder Propanoxidehydrierung zurückzuführen und im Rahmen der Zielabtrennung wiederzugewinnen. Diese enthalten in der Regel zwar noch Propan sowie gegebenenfalls Propylen, häufig jedoch keinen molekularen Sauerstoff mehr. Üblicherweise werden sie mit dem Hauptrestgas zu einem Gesamtrestgas vereint in die gegebenenfalls als Propylenquelle dienende heterogen katalysierte Propandehydrierung und/oder Propanoxidehydrierung rückgeführt. Es ist aber auch eine separate Verwertung solcher weiteren restliche Gase möglich.

Durch die vorzugsweise vollständige Rückführung des Gesamtrestgases kann so im kontinuierlichen Betrieb eine kontinuierliche Umsetzung von Propan zu Acrylsäure und/oder Acrolein erfolgen.

Wesentlich ist dabei, dass durch die beschriebene Rückführung des in die gegebenenfalls als Propylenquelle dienende heterogen katalysierte Propandehydrierung und/oder Propanoxidehydrierung in letzteren eine Überführung von Propan zu Propylen mit nahezu hundertprozentiger Selektivität erreichbar ist.

Die Vorteilhaftigkeit einer solchen Verfahrensweise ist dabei sowohl bei niederen ($\leq$ 30 mol-%) als auch bei hohen ($\geq$ 30 mol-%) Dehydrierumsätzen (bezogen auf einmaligen Durchgang von Frischpropan durch die Dehydrierung) gegeben. Generell ist es bei einer solchen Rückführung von Oxidationskreisgas günstig, wenn der Wasserstoffgehalt im Reaktionsgasausgangsgemisch 1 in einem wenigstens stöchiometrischen Verhältnis (bezüglich einer Sauerstoffverbrennung zu Wasser) zur über das Oxidationskreisgas ins Reaktionsgasausgangsgemisch 1 zurückgeführten Sauerstoffmenge steht.

Vorgenannte Kreisgasfahrweise ist in entsprechender Weise anwendbar, wenn die Partialoxidation eine partielle Ammoxidation von Propen zu Acrylnitril ist. Es ist auch dann entsprechend anwendbar, wenn in der Dehydrierung das Propan durch iso-Butan ersetzt wird und das dabei resultierende iso-Buten in entsprechender Weise in einer Partialoxidation zu Methacrolein und/oder Methacrylsäure partialoxidiert wird.

An dieser Stelle sei nochmals festgehalten, dass die Abtrennung von Acrylsäure aus einem erfindungsgemäß erhaltenen Produktgasgemisch 3 (insbesondere von beispielhaft ausgeführten) bevorzugt so erfolgt, dass man das zuvor gegebenenfalls durch direkte und/oder indirekte Kühlung abgekühlte Produktgasgemisch 3 in einer trennwirksame Einbauten enthaltenden Kolonne unter Seitenabzug einer rohen Acrylsäure (z. B. in sich selbst) aufsteigend fraktionierend kondensiert und/oder mit Wasser bzw. wässriger Lösung absorbiert, wie es die WO 2004/035514 und die DE-A 102 43 625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische oder um eine mechanische Waschkolonne. Im einzelnen kann der Beschreibung der WO 01/77056, der WO 03/041832 sowie der WO 03/041833 gefolgt werden. D. h., vorzugsweise wird verbliebene Mutterlauge in die fraktionierende Kondensation rückgeführt (vgl. auch EP-A 10 15 410). Der Nebenkomponentenauslaß ist normalerweise unterhalb des Seitenabzugs der rohen Acrylsäure als purge-Strom.

Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf Basis von Poly-Na-Acrylat eignet. Auch sei noch festgehalten, dass ein Vorteil der erfindungsgemäßen Verfahrensweise grundsätzlich darin besteht, dass an allen Stellen dieser Schrift, einschließlich der Ausführungsbeispiele, dort wo mit Inertmaterial verdünnte Katalysatorbeschickungen beschrieben und/oder gefordert werden, die entsprechenden Katalysatoren bei der gleicher Bettlänge auch unverdünnt eingesetzt (verwendet) werden können.

Beispiele

I. Heterogen katalysierte partielle Propandehydrierungen als Propylenquelle

Allgemeiner Versuchsaufbau und Versuchsausführung sowie Ergebnisse (beschrieben wird in allen Fällen der stationäre Betriebszustand).

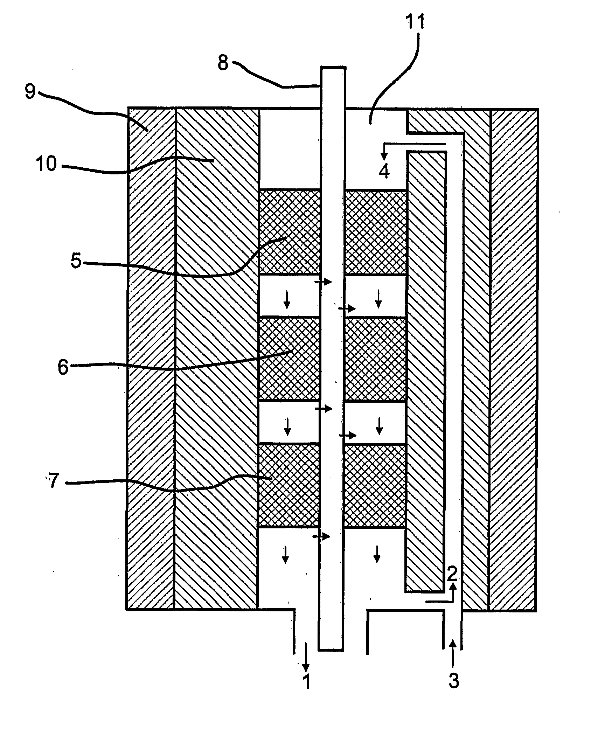

Die heterogen katalysierten partiellen Propandehydrierungen wurden in einem Hordenschlaufenreaktor gemäß Figur 1 durchgeführt, auf die sich die numerischen Adressen im folgenden beziehen.

Ein vertikal stehender Rohrreaktor (11) (Durchmesser: 80 mm) war mit einer thermischen Isolierung (10) versehen in eine Stützheizung (9) eingehaust. Die Temperatur der Stützheizung betrug 500°C. Mittig im Rohrreaktor befand sich ein Zentralrohr (Durchmesser: 20 mm), welches eine Hülse für ein Zugthermoelement und eine Hülse für ein Stufenthermoelement enthielt. Zusätzlich enthielt es in den Rohrreaktor führende Leitungen, über die aus dem Rohrreaktor Reaktionsgasproben entnommen werden konnten, sowie in den Rohrreaktor führende Leitungen, über die in den Rohrreaktor Luft eingedüst werden konnte.

Der Rohrreaktor enthielt drei Horden (5, 6, 7), die aus drei identischen, auf einem E-delstahldrahtnetz aufgebrachten Schüttungen aus Inertmaterial und Dehydrierkatalysator bestanden. In Strömungsrichtung des Reaktionsgases durch den Dehydrierreaktor waren die Schüttungen wie folgt aufgebaut:

Zunächst auf einer Schüttlänge von 60 mm eine Schüttung aus Steatitkugeln (Durchmesser: 4 bis 5 mm) aus Steatit C-220 der Fa. CeramTec.. Anschließend auf einer Schüttlänge von 120 mm Dehydrierkatalysator (Pt/Sn-Legierung, die mit den Elementen Cs, K und La in oxidischer Form promoviert war und die auf die äußere und innere Oberfläche von $ZrO_2 \cdot SiO_2$ Mischoxidträgerstränglingen (mittlere Länge (gaußverteilt im Bereich von 3 mm bis 12 mm mit Maximum bei ca. 6 mm) : 6 mm, Durchmesser: 2 mm) in der Elementstöchiometrie (Massenverhältnisse einschließlich Träger) $Pt_{0,3}Sn_{0,6}La_{3,0}Cs_{0,5}K_{0,2}(ZrO_2)_{88,3}(SiO_2)_{7,1}$ aufgebracht war (Katalysatorvorläuferherstellung und Aktivierung zum aktiven Katalysator wie in Beispiel 4 der DE-A 10219879).

Das aus der letzten Horde austretende Produktgasgemisch 1 wurde in zwei Hälften identischer Zusammensetzung aufgeteilt. Die eine Hälfte (2) wurde als Bestandteil des Reaktionsgasausgangsgemisches (4) in die Dehydrierung rückgeführt. Die andere Hälfte (1) wurde aus der Dehydrierzone herausgeführt.

Durch Absorption in technischem Tetradecan der Fa. Haltermann, DE vom Typ PKWF 4/7 af als Absorptionsmittel und nachfolgendes Strippen mit Luft können (wie in der DE-A 10 2004 032 129 beschrieben) die von Propylen und Propan verschiedenen Bestandteile weitgehend abgetrennt und ein für die Partialoxidation von Propylen zu Acrolein und/oder Acrylsäure unmittelbar geeignetes Beschickungsgasgemisch erhalten werden. Das Reaktionsgasausgangsgemisch (4) bestand aus Dehydrierkreisgas (2) sowie Wasserdampf, Oxidationskreisgas (zur Vereinfachung simuliert durch ein Gemisch aus Sauerstoff und Stickstoff) und Frischpropan (alle drei hier zusammenfassend durch die Adresse (3) symbolisiert).

Das Frischpropan wies nachfolgende Gehalte auf:

|  | Vol.-% |
|---|---|
| Methan | 0,005 |
| Ethan | 0,005 |
| Ethen | 0 |
| Propan | 99,982 |
| Propen (Propylen) | 0,003 |

(fortgesetzt)

|  | Vol.-% |
|---|---|
| iso-Butan | 0,005 |

Die Belastung der ersten Katalysatorschüttung mit Propan betrug in allen Fällen 305 Nl/l•h.

Der Eingangsdruck des Reaktionsgasausgangsgemischs lag bei 2,3 bar. Der Druckverlust über den Dehydrierreaktor betrug ca. 200 mbar. Vor der zweiten und vor der dritten Katalysatorschüttung (in Strömungsrichtung) wurde dem Reaktionsgasgemisch gegebenenfalls Luft (500°C, Reaktionsdruck) zugedüst. Die nachfolgenden Tabellen 1 bis 3 weisen die in drei Versuchen in Abhängigkeit der verschiedenen Reaktionsgasausgangsgemische (4) sowie der unterschiedlichen Luftzudosierungen erhaltenen Ergebnisse aus.

Dabei gelten folgenden Symbole:

L2 = Luftzugabe vor der zweiten Katalysatorschüttung in Nl/h
L3 = Luftzugabe vor der dritten Katalysatorschüttung in Nl/h
RA = Gehalte des Reaktionsgasausgangsgemischs (4) (enthielt in allen Fällen, bezogen auf die enthaltene Menge an Propan, ca. 70 Vol.-% Wasserdampf)
R1 = Gehalte des Reaktionsgases, nach Durchtritt durch die erste Katalysatorschüttung
R2 = Gehalte des Reaktionsgases, nach Durchtritt durch die zweite Katalysatorschüttung
P = Gehalte des Produktgases, das ist das Reaktionsgas nach Durchtritt durch die dritte Katalysatorschüttung
TE = Eintrittstemperatur des Reaktionsgasausgangsgemischs in die erste Katalysatorschüttung in °C
T1 = höchste Temperatur in der ersten Katalysatorschüttung in °C
T2 = höchste Temperatur in der zweiten Katalysatorschüttung in °C
T3 = höchste Temperatur in der dritten Katalysatorschüttung in °C
TA = Austrittstemperatur des Produktgases
B = Gehalte des resultierenden Beschickungsgasgemischs für eine erfindungsgemäße Partialoxidation (alle Beschickungsgasgemische waren nicht explosiv)
U = Propanumsatz, bezogen auf Frischpropan und einmaligen Durchgang durch den Dehydrierreaktor
S = Selektivität der Propylenbildung

(alle Gehalte in Vol.-% bezogen auf das Gesamtgas, wasserfrei gerechnet).

Beispiel 1/Tabelle 1

| L2 = 50 | | | | | | | | | | | | |
|---|---|---|---|---|---|---|---|---|---|---|---|---|
| L3 = 70 | | | | | | | | | | | | |
|  | RA | R1 | R2 | P | TE | T1 | T2 | T3 | TA | U | S | B |
| Methan | 0,31 | 0,33 | 0,53 | 0,63 | 498 | 592 | 574 | 579 | 575 | 31 | 84 | |
| Ethan | 0,18 | 0,23 | 0,27 | 0,36 | | | | | | | | |
| Ethen | 0,21 | 0,20 | 0,39 | 0,43 | | | | | | | | |
| Propan | 17,6 | 16,15 | 15,22 | 14,09 | | | | | | | | 20,2 |
| Propylen | 2,67 | 4,54 | 4,72 | 5,35 | | | | | | | | 7,69 |
| $H_2$ | 6,17 | 5,40 | 4,83 | 5,30 | | | | | | | | |
| $O_2$ | 1,77 | - | - | - | | | | | | | | 14,6 |
| $N_2$ | 70,65 | 72,63 | 73,38 | 73,06 | | | | | | | | 54,5 |
| CO | 0,08 | 0,09 | 0,13 | 0,17 | | | | | | | | |
| $CO_2$ | 0,30 | 0,41 | 0,51 | 0,60 | | | | | | | | |

Beispiel 2/Tabelle 2

| L2 = 40 | | | | | | | | | | | |
|---|---|---|---|---|---|---|---|---|---|---|---|
| L3 = 50 | | | | | | | | | | | |
| | RA | R1 | R2 | P | TE | T1 | T2 | T3 | TA | U | S | B |
| Methan | 0,31 | 0,69 | 0,65 | 0,61 | 537 | 589 | 562 | 569 | 564 | 36 | 86 | |
| Ethan | 0,38 | 0,79 | 0,78 | 0,76 | | | | | | | | |
| Ethen | 0,04 | 0,11 | 0,08 | 0,07 | | | | | | | | |
| Propan | 17,36 | 14,88 | 12,80 | 13,55 | | | | | | | | 16,62 |
| Propylen | 3,28 | 5,79 | 6,33 | 6,56 | | | | | | | | 8,05 |
| $H_2$ | 7,12 | 7,61 | 7,17 | 7,19 | | | | | | | | |
| $O_2$ | 1,77 | - | - | - | | | | | | | | 15,29 |
| $N_2$ | 69,28 | 69,26 | 71,28 | 70,33 | | | | | | | | 57,0 |
| CO | 0,06 | 0,13 | 0,11 | 0,12 | | | | | | | | |
| $CO_2$ | 0,40 | 0,74 | 0,80 | 0,80 | | | | | | | | |

Beispiel 3/Tabelle 3

| L2 = 0 | | | | | | | | | | | |
|---|---|---|---|---|---|---|---|---|---|---|---|
| L3 = 70 | | | | | | | | | | | |
| | RA | R1 | R2 | P | TE | T1 | T2 | T3 | TA | U | S | B |
| Methan | 0,09 | 0,121 | 0,128 | 0,175 | 463 | 566 | 541 | 547 | 541 | 25 | 93 | |
| Ethan | 0,08 | 0,101 | 0,116 | 0,150 | | | | | | | | |
| Ethen | 0,05 | 0,073 | 0,070 | 0,103 | | | | | | | | |
| Propan | 18,60 | 15,58 | 17,12 | 16,02 | | | | | | | | 23,6 |
| Propylen | 2,50 | 3,94 | 4,30 | 5,00 | | | | | | | | 7,36 |
| $H_2$ | 5,90 | 4,54 | 4,47 | 4,76 | | | | | | | | |
| $O_2$ | 1,77 | - | - | - | | | | | | | | 14,0 |
| $N_2$ | 70,79 | 73,33 | 73,43 | 73,34 | | | | | | | | 52,1 |
| CO | 0,05 | 0,062 | 0,062 | 0,097 | | | | | | | | |
| $CO_2$ | 0,18 | 0,251 | 0,300 | 0,351 | | | | | | | | |

II. Erfindungsgemäße heterogen katalysierte zweistufige Partialoxidation von Propylen zu Acrylsäure. (beschrieben wird der stationäre Betriebszustand)

Versuchsanordnung

Erste Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1:    50 cm Länge
               Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 20 Gew.-% (alternativ 30 Gew.-%) an Steatitringen der Geometrie 5 mm x 3 mm x 2 mm (Außendurchmesser x Länge x Innendurchmesser) und 80 Gew.-% (alternativ 70 Gew.-%) Vollkatalysator aus Abschnitt 3.

Abschnitt 3: 160 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9 \times 2WO_3]_{0,5} [MO_{12}Co_{5,5}Fe_{2,94}Si_{1,59}K_{0,08}O_x]_1$).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

Zweite Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 90 cm Länge
Katalysatorbeschickung mit einem homogenen Gemisch aus 25 Gew.-% (alternativ 30 Gew.-%) Steatit-Ringen der Geometrie 7 mm x 3 mm x 4 mm (Außendurchmesser x Länge x Innendurchmesser) und 75 Gew.-% (alternativ 70 Gew.-%) Schalenkatalysator aus Abschitt 4.

Abschnitt 3: 50 cm Länge
Katalysatorbeschickung mit einem homogenen Gemisch aus 15 Gew.-% (alternativ 20 Gew.-%) an Steatit-Ringen der Geometrie 7 mm x 3 mm x 4 mm (Außendurchmesser x Länge x Innendurchmesser) und 85 Gew.-% (alternativ 80 Gew.-%) Schalenkatalysator aus Abschnitt 4.

Abschnitt 4: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $MO_{12}V_3W_{1,2}Cu_{2,4}O_x$).

Von oben nach unten werden die ersten 175 cm mittels eines im Gegenstrom gepumpten Salzbades C thermostatisiert. Die zweiten 175 cm werden mittels eines im Gegenstrom gepumpten Salzbades D thermostatisiert. Propylenquelle und zweistufige Partialoxidation zu Acrylsäure

Zunächst wird ein Hordenreaktor wie in I betrieben. Die Zusammensetzung des Reaktionsgasausgangsgemischs für die heterogen katalysierte Dehydrierung ist wie folgt (Vol.-%, bezogen auf Gesamtgas):

|  | Vol.-% |
|---|---|
| Acrylsäure | 0,02 |
| Essigsäure | 0,03 |
| Wasser | 9,23 |
| 1-Buten | 0,01 |
| iso-Buten | 0,02 |
| Propan | 18,46 |
| Propylen | 3,98 |
| Ethan | 1,16 |
| Ethylen | 0,22 |

(fortgesetzt)

|  | Vol.-% |
|---|---|
| $CO_2$ | 2,34 |
| CO | 0,26 |
| $N_2$ | 59,7 |
| $O_2$ | 1,62 |
| $CH_4$ | 0,12 |
| $H_2$ | 2,83 |

Es besteht aus:

- 41,9 Vol.-% Oxidationskreisgas, das folgende Gehalte aufweist:

|  | Vol.-% |
|---|---|
| Acrylsäure | 0,02 |
| Essigsäure | 0,04 |
| $H_2O$ | 2,73 |
| iso-Buten | 0,01 |
| Acrolein | 0,05 |
| Propan | 17,30 |
| Propylen | 0,32 |
| Ethan | 1,20 |
| Ethylen | 0,22 |
| $CO_2$ | 2,41 |
| CO | 0,61 |
| $N_2$ | 71,21 |
| $O_2$ | 3,87 |

- 3,9 Vol.-% Frischpropan, das folgende Gehalte aufweist:

|  | Vol.-% |
|---|---|
| Propan | 98,91 |
| iso-Butan | 0,05 |
| Propylen | 0,1 |
| Ethan | 0,92 |
| Ethylen | 0,01 |

- 1,02 Vol.-% Wasserstoff
- 2,03 Vol.-% Wasserdampf und
- 51,15 Vol.-% Dehydrierkreisgas.

Das restliche Produktgasgemisch ist wie folgt zusammengesetzt:

|  | Vol.-% |
|---|---|
| Acrylsäure | 0,02 |
| Essigsäure | 0,03 |
| $H_2O$ | 11,84 |
| iso-Buten | 0,01 |
| Propan | 14,32 |
| Propylen | 7,52 |
| Ethan | 1,21 |
| Ethylen | 0,26 |

(fortgesetzt)

| | Vol.-% |
|---|---|
| $CO_2$ | 2,61 |
| $N_2$ | 58,41 |
| $O_2$ | 0,23 |
| $H_2$ | 3,55 |

Das im Produktgasgemisch der heterogen katalysierten Propandehydrierung enthaltene Propan und Propylen wird wie in I beschrieben absorptiv abgetrennt und mittels Luft unter Erhalt des folgenden Beschickungsgases für die Partialoxidation wieder freigestrippt, das die folgenden Gehalte aufweist:

| | Vol.-% |
|---|---|
| $H_2O$ | 2,39 |
| Tetradekan | 0,01 |
| iso-Buten | 0,01 |
| Propan | 15,15 |
| Propylen | 7,95 |
| Ethan | 1,10 |
| Ethylen | 0,20 |
| $CO_2$ | 1,05 |
| $N_2$ | 56,99 |
| $O_2$ | 15,16 |

Mit diesem Beschickungsgasgemisch (es liegt außerhalb des Explosionsbereichs) wird die beschriebene erste Partialoxidationsreaktionsstufe beschickt. Die Propylenbelastung der Festbettkatalysatorbeschickung wird zu 185 Nl/l • h gewählt. Der Druck am Eingang der ersten Reaktionsstufe beträgt 3,1 bar. $T_A$ = 322°C; $T_B$ = 328°C;

Das die erste Reaktionsstufe verlassende Produktgasgemisch weist folgende Gehalte auf:

| | Vol.-% |
|---|---|
| Acrylsäure | 0,46 |
| Essigsäure | 0,14 |
| $H_2O$ | 10,65 |
| 1-Buten | 0,01 |
| Acrolein | 6,99 |
| Propan | 15,16 |
| Propylen | 0,17 |
| Ethan | 1,10 |
| Ethylen | 0,20 |
| $CO_2$ | 1,62 |
| CO | 0,23 |
| $N_2$ | 57,02 |
| $O_2$ | 6,25 |

$U^P_A$, der Propenumsatz am Ende der Reaktionszone A, beträgt 64,5 mol.-%. $U^P_B$, der Propenumsatz am Ende der Reaktionszone B, beträgt 94,9 mol.-%.

Dem Produktgasgemisch der ersten Stufe wird soviel Luft (25°C) zudosiert, dass das Verhältnis Acrolein: $O_2$ im resultierenden Gemisch 6,59 zu 7,07 beträgt.

Mit diesem Gemisch wird dann die zweite Reaktionsstufe unmittelbar beschickt (T = 231,7°C). Die Acroleinbelastung des Katalysatorfestbetts beträgt 152 Nl/l • h.

$T_C$ = 263°C; $T_D$ = 269°C. Der Druck am Eingang der zweiten Reaktionsstufe beträgt 2,1 bar.

Das die zweite Reaktionsstufe verlassende Produktgasgemisch weist folgende Gehalte auf:

|  | Vol.-% |
|---|---|
| Acrylsäure | 6,72 |
| Essigsäure | 0,22 |
| $H_2O$ | 11,06 |
| Formaldehyd | 0,14 |
| Acrolein | 0,05 |
| Ameisensäure | 0,03 |
| Maleinsäureanhydrid | 0,06 |
| Benzolsäure | 0,01 |
| Propan | 14,62 |
| Propylen | 0,28 |
| Ethan | 1,02 |
| Ethylen | 0,18 |
| $CO_2$ | 2,03 |
| CO | 0,52 |
| $N_2$ | 59,86 |
| $O_2$ | 3,20 |
| Propionsäure | 0,0032 |

$U^A_C$, der Acroleinumsatz am Ende der Reaktionszone C, beträgt 68,1 mol.-%.

$U^A_D$, der Acroleinumsatz am Ende der Reaktionszone D, beträgt 98,8 mol.-%.

Der auf die enthaltene Acrylsäuremenge bezogene Propionsäuregehalt liegt signifikant unter jenen der Beispiele der WO 01/96270.

In beiden Reaktionsstufen durchströmt das Reaktionsgasgemisch die beiden Kontaktrohre von oben nach unten.

Die Gehaltsanalysen erfolgen mittels gaschromatographischer Analyse.

Die Acrylsäure wird aus dem Produktgasgemisch wie in den beispielhaften Ausführungen der DE-A 10 2004 032 129 abgetrennt und das Restgas als Oxidationskreisgas in die heterogen katalysierte Dehydrierung rückgeführt.

**Patentansprüche**

1. Verfahren zur Herstellung von Acrolein oder Acrylsäure oder deren Gemisch durch heterogen katalysierte partielle Gasphasenoxidation von Propen, bei dem man ein die Reaktanden Propylen und molekularer Sauerstoff sowie die inerten Verdünnungsgase molekularer Stickstoff und Propan enthaltendes Reaktionsgasausgangsgemisch 2, das den molekularen Sauerstoff und das Propylen in einem molaren Verhältnis $O_2 : C_3H_6 \geq 1$ enthält, bei erhöhter Temperatur durch ein Katalysatorfestbett führt, dessen Aktivmasse wenigstens ein die Elemente Mo, Fe und Bi enthaltendes Multimetalloxid ist, **dadurch gekennzeichnet, dass** das Reaktionsgasausgangsgemisch 2, bezogen auf sein Gesamtvolumen, die nachfolgenden Gehalte

| 6 bis 9 Vol.-% | Propylen, |
|---|---|
| 8 bis 18 Vol.-% | molekularer Sauerstoff, |
| 6 bis 30 Vol.-% | Propan und |
| 32 bis 72 Vol.-% | molekularer Stickstoff, |

mit der Maßgabe aufweist, dass das molare Verhältnis $V_1$ von im Reaktionsgasausgangsgemisch 2 enthaltenem Propan zu im Reaktionsgasausgangsgemisch 2 enthaltenem Propylen 1 bis 4, das molare Verhältnis $V_2$ von im Reaktionsgasausgangsgemisch 2 enthaltenem molekularem Stickstoff zu im Reaktionsgasausgangsgemisch 2 enthaltenem molekularem Sauerstoff 2 bis 6 und das molare Verhältnis $V_3$ von im Reaktionsgasausgangsgemisch 2 enthaltenem molekularem Sauerstoff zu im Reaktionsgasausgangsgemisch 2 enthaltenem Propylen 1,3 bis 2,4 beträgt.

2. Verfahren nach Anspruch 1, **dadurch gekennzeichnet, dass** das Reaktionsgasausgangsgemisch 2 die nachfolgenden Gehalte

| | |
|---|---|
| 7 bis 9 Vol.-% | Propylen, |
| 9,8 bis 16 Vol.-% | molekularer Sauerstoff, |
| 9 bis 25 Vol.-% | Propan und |
| 35 bis 65 Vol.-% | molekularer Stickstoff, |

mit der Maßgabe aufweist, dass

$V_1$ = 1 bis 3,5
$V_2$ = 3 bis 4,5 und
$V_3$ = 1,4 bis 2,2 beträgt.

3. Verfahren nach Anspruch 1, **dadurch gekennzeichnet, dass** das Reaktionsgasausgangsgemisch 2 die nachfolgenden Gehalte

| | |
|---|---|
| 7 bis 9 Vol.-% | Propylen, |
| 9,8 bis 15 Vol.-% | molekularer Sauerstoff, |
| 10,5 bis 20 Vol.-% | Propan und |
| 40 bis 60 Vol.-% | molekularer Stickstoff, |

mit der Maßgabe aufweist, dass

$V_1$ = 1,5 bis 2,5
$V_2$ = 3,5 bis 4,5 und
$V_3$ = 1,4 bis 2,14 beträgt.

4. Verfahren nach Anspruch 3, **dadurch gekennzeichnet, dass** $V_2$ 3,5 bis 4 beträgt.

5. Verfahren nach Anspruch 3 oder 4, **dadurch gekennzeichnet, dass** $V_3$ 1,5 bis 2,0 beträgt.

6. Verfahren nach Anspruch 1, **dadurch gekennzeichnet**, das dass Reaktionsgasausgangsgemisch 2 die nachfolgenden Gehalte

| | |
|---|---|
| 7 bis 9 Vol.-% | Propylen, |
| 9,8 bis 15,5 Vol.-% | molekularer Sauerstoff, |
| 10,5 bis 15,5 Vol.-% | Propan und |
| 40 bis 60 Vol.-% | molekularer Stickstoff, |

mit der Maßgabe aufweist, dass

$V_1$ = 1,5 bis 2,2
$V_2$ = 3,5 bis 4,5 und
$V_3$ = 1,5 bis 2,14 beträgt.

7. Verfahren nach Anspruch 6, **dadurch gekennzeichnet, dass** $V_2$ 3,5 bis 4 beträgt.

8. Verfahren nach Anspruch 6 oder 7, **dadurch gekennzeichnet, dass** $V_3$ 1,5 bis 2,0 beträgt.

9. Verfahren nach Anspruch 1, **dadurch gekennzeichnet, dass** das Reaktionsgasausgangsgemisch 2 die nachfolgenden Gehalte

| | |
|---|---|
| 7 bis 8 Vol.-% | Propylen, |
| 11,9 bis 15,5 Vol.-% | molekularer Sauerstoff, |
| 11,9 bis 15,5 Vol.-% | Propan und |
| 50 bis 60 Vol.-% | molekularer Stickstoff, |

mit der Maßgabe aufweist, dass

$V_1$ = 1,7 bis 2,1
$V_2$ = 3,5 bis 4,5 und
$V_3$ = 1,7 bis 2,1 beträgt.

10. Verfahren nach Anspruch 9, **dadurch gekennzeichnet, dass** $V_2$ 3,5 bis 4 beträgt.

11. Verfahren nach Anspruch 9 oder 10, **dadurch gekennzeichnet, dass** $V_3$ 1,8 bis 2,0 beträgt.

12. Verfahren nach Anspruch 1, **dadurch gekennzeichnet, dass** das Reaktionsgasausgangsgemisch 2 die nachfolgenden Gehalte

| | |
|---|---|
| 7 bis 9 Vol.-% | Propylen, |
| 9,8 bis 15 Vol.-% | molekularer Sauerstoff, |
| 21 bis 28 Vol.-% | Propan und |
| 40 bis 60 Vol.-% | molekularer Stickstoff, |

mit der Maßgabe aufweist, dass

$V_1$ = 3 bis 4
$V_2$ = 3,5 bis 4,5 und
$V_3$ = 1,4 bis 2,14 beträgt.

13. Verfahren nach Anspruch 12, **dadurch gekennzeichnet, dass** $V_2$ 3,5 bis 4 beträgt.

14. Verfahren nach Anspruch 12 oder 13, **dadurch gekennzeichnet, dass** $V_3$ 1,5 bis 2,0 beträgt.

15. Verfahren nach einem der Ansprüche 1 bis 14, **dadurch gekennzeichnet, dass** der Gesamtgehalt des Reaktionsgasausgangsgemischs 2 an von Propylen, molekularem Sauerstoff, Propan und molekularem Stickstoff verschiedenen Bestandteilen $\leq$ 10 Vol.-% beträgt.

16. Verfahren nach einem der Ansprüche 1 bis 15, **dadurch gekennzeichnet, dass** das Reaktionsgasausgangsgemisch 2 zu 0,5 bis 8 Vol.-% wenigstens eine der Verbindungen Methan und Ethan enthält.

17. Verfahren nach einem der Ansprüche 1 bis 15, **dadurch gekennzeichnet, dass** das Reaktionsgasausgangsgemisch 2 zu 0,5 bis 5 Vol.-% wenigstens eine der Verbindungen Methan und Ethan enthält.

18. Verfahren nach einem der Ansprüche 1 bis 15, **dadurch gekennzeichnet, dass** das Reaktionsgasausgangsgemisch 2 zu 0,5 bis 3 Vol.-% wenigstens eine der Verbindungen Methan und Ethan enthält.

19. Verfahren nach einem der Ansprüche 1 bis 17, **dadurch gekennzeichnet, dass** das Reaktionsgasausgangsgemisch 2 $\leq$ 5 Vol.-% Wasser und $\leq$ 5 Vol.-% Kohlenoxide enthält.

20. Verfahren nach einem der Ansprüche 1 bis 17, **dadurch gekennzeichnet, dass** das Reaktionsgasausgangsgemisch 2 $\leq$ 3 Vol.-% Wasser und $\leq$ 3 Vol.-% Kohlenoxide enthält.

21. Verfahren nach einem der Ansprüche 1 bis 17, **dadurch gekennzeichnet, dass** das Reaktionsgasausgangsgemisch 2 $\leq$ 2 Vol.-% Wasser und $\leq$ 2 Vol.-% Kohlenoxide enthält.

22. Verfahren nach einem der Ansprüche 1 bis 21, **dadurch gekennzeichnet, dass** das Reaktionsgasausgangsgemisch 2 $\geq$ 0,5 Vol.-% Wasser enthält.

23. Verfahren nach einem der Ansprüche 1 bis 22, **dadurch gekennzeichnet, dass** der Gesamtgehalt des Reaktionsgasausgangsgemischs 2 an von Propylen, molekularem Sauerstoff, Propan und molekularem Stickstoff verschiedenen Bestandteilen $\leq$ 5 Vol.-% beträgt.

**24.** Verfahren nach einem der Ansprüche 1 bis 22, **dadurch gekennzeichnet, dass** der Gesamtgehalt des Reaktionsgasausgangsgemischs 2 an von Propylen, molekularem Sauerstoff, Propan und molekularem Stickstoff verschiedenen Bestandteilen ≤ 3 Vol.-% beträgt.

**25.** Verfahren nach einem der Ansprüche 1 bis 24, **dadurch gekennzeichnet, dass** als Quelle für das im Reaktionsgasausgangsgemisch 2 enthaltene Propylen in Verfahren der kontinuierlichen heterogen katalysierten partiellen Dehydrierung und/oder Oxidehydrierung von Propan in der Gasphase gebildetes Propylen verwendet wird, ohne dass von diesem Propylen das in der heterogen katalysierten partiellen Dehydrierung und/oder Oxidehydrierung nicht umgesetztes Propan zuvor abgetrennt wird.

**26.** Verfahren nach Anspruch 25, **dadurch gekennzeichnet, dass** das Verfahren der heterogen katalysierten partiellen Dehydrierung von Propan eine autotherme Dehydrierung ist.

**27.** Verfahren nach Anspruch 25 oder 26, **dadurch gekennzeichnet, dass** das Verfahren der heterogen katalysierten partiellen Dehydrierung von Propan ein solches ist, bei dem

- einer Dehydrierzone ein das zu dehydrierende Propan enthaltendes Reaktionsgasausgangsgemisch 1 kontinuierlich zugeführt wird,
- das Reaktionsgasausgangsgemisch 1 in der Dehydrierzone durch wenigstens ein Katalysatorfestbett geführt wird, an welchem durch katalytische Dehydrierung molekularer Wasserstoff und Propylen gebildet werden,
- dem Reaktionsgasausgangsgemisch 1 vor und/oder nach Eintritt in die Dehydrierzone wenigstens ein molekularen Sauerstoff enthaltendes Gas zugesetzt wird,
- der molekulare Sauerstoff in der Dehydrierzone im Reaktionsgasgemisch 1 enthaltenen molekularen Wasserstoff teilweise zu Wasserdampf oxidiert und
- der Dehydrierzone ein Produktgas, das molekularen Wasserstoff, Wasserdampf, Propylen und nicht umgesetztes Propan enthält mit der Maßgabe entnommen wird, dass das der Dehydrierzone entnommene Produktgas in zwei Teilmengen identischer Zusammensetzung aufgeteilt und eine der beiden Teilmengen als Dehydrierkreisgas in die Dehydrierzone rückgeführt wird.

**28.** Verfahren nach Anspruch 27, **dadurch gekennzeichnet, dass** das Dehydrierkreisgas in das Reaktionsgasausgangsgemisch 1 rückgeführt wird.

**29.** Verfahren nach Anspruch 28, **dadurch gekennzeichnet, dass** das Reaktionsgasausgangsgemisch 1 enthält:

| 15 bis 25 Vol.-% | Propan, |
|---|---|
| 2 bis 6 Vol.-% | Propylen, |
| 5 bis 20 Vol.-% | Wasserdampf, |
| 2 bis 10 Vol.-% | molekularer Wasserstoff, |
| 40 bis 75 Vol.-% | molekularer Stickstoff und |
| > 0 bis 3 Vol.-% | molekularer Sauerstoff. |

**30.** Verfahren nach Anspruch 28 oder 29, **dadurch gekennzeichnet, dass** das Produktgas Propan und Propylen im molekularen Verhältnis Propen zu Propan von 0,3 bis 0,66 enthält.

**31.** Verfahren nach einem der Ansprüche 1 bis 24, **dadurch gekennzeichnet, dass** als Quelle für das im Reaktionsgasausgangsgemisch 2 enthaltene Propylen das Produktgasgemisch einer partiellen Propandehydrierung verwendet wird, nachdem wenigstens 50 Vol.-% der im Produktgas der Propandehydrierung enthaltenen, von Propan und Propylen verschiedenen, Bestandteile abgetrennt worden sind.

**32.** Verfahren nach einem der Ansprüche 1 bis 31, **dadurch gekennzeichnet, dass** die Belastung des Katalysatorfestbetts mit Propylen ≥ 135 Nl/l•h bis 300 Nl/l•h beträgt.

**33.** Verfahren nach einem der Ansprüche 1 bis 31, **dadurch gekennzeichnet, dass** der Propylenumsatz bei einmaligem Durchgang ≥ 90 mol.-% und die damit einhergehende Selektivität der Acroleinbildung sowie der Acrylsäurenebenproduktbildung zusammengenommen ≥ 90 mol.-% betragen und

a) die Belastung des Katalysatorfestbetts mit dem im Reaktionsgasausgangsgemisch 2 enthaltenen Propylen ≥ 160 Nl Propylen/l Katalysatorfestbett • h beträgt,

b) das Katalysatorfestbett aus einem in zwei räumlich aufeinanderfolgenden Reaktionszonen A*, B* angeordneten Katalysatorfestbett besteht, wobei die Temperatur der Reaktionszone A* 300 bis 390°C und die Temperatur der Reaktionszone B* 305 bis 420°C beträgt und gleichzeitig wenigstens 5°C oberhalb der Temperatur der Reaktionszone A* liegt,

c) das Reaktionsgasausgangsgemisch 2 die Reaktionszonen A*, B* in der zeitlichen Abfolge "erst A*", "dann B*" durchströmt und

d) sich die Reaktionszone A* bis zu einem Umsatz des Propens von 40 bis 80 mol.-% erstreckt.

34. Verfahren nach einem der Ansprüche 1 bis 33, **dadurch gekennzeichnet, dass** sich an das Verfahren nach einem der Ansprüche 1 bis 33 ein Verfahren der heterogen katalysierten partiellen Gasphasenoxidation von in einem Verfahren nach einem der Ansprüche 1 bis 33 gebildetem Acrolein zu Acrylsäure anschließt, bei dem man ein das Acrolein enthaltende Reaktionsgasausgangsgemisch 3 durch ein Katalysatorfestbett führt, dessen Aktivmasse wenigstens ein die Elemente Mo und V enthaltendes Multimetalloxid ist.

35. Verfahren nach Anspruch 34, **dadurch gekennzeichnet, dass** das Reaktionsgasausgangsgemisch 3 die nachfolgenden Gehalte aufweist:

| | |
|---|---|
| 4,5 bis 8 Vol.-% | Acrolein, |
| 2,25 bis 9 Vol.-% | molekularer Sauerstoff, |
| 6 bis 30 Vol.-% | Propan, |
| 32 bis 72 Vol.-% | molekularer Stickstoff, |
| 5 bis 15 Vol.-% | Wasserdampf. |

36. Verfahren nach Anspruch 35, **dadurch gekennzeichnet, dass** das Reaktionsgasausgangsgemisch 3 4,5 bis 9 Vol.-% molekularen Sauerstoff enthält.

37. Verfahren nach Anspruch 34, **dadurch gekennzeichnet, dass** das Reaktionsgasausgangsgemisch 3 die folgenden Gehalte aufweist:

| | |
|---|---|
| 5,5 bis 8 Vol.-% | Acrolein, |
| 2,75 bis 9 Vol.-% | molekularer Sauerstoff, |
| 10 bis 25 Vol.-% | Propan, |
| 40 bis 70 Vol.-% | molekularer Stickstoff, |
| 5 bis 15 Vol.-% | Wasserdampf. |

38. Verfahren nach Anspruch 37, **dadurch gekennzeichnet, dass** das Reaktionsgasausgangsgemisch 3 5,5 bis 9 Vol.-% molekularen Sauerstoff enthält.

39. Verfahren nach Anspruch 34, **dadurch gekennzeichnet, dass** das Reaktionsgasausgangsgemisch 3 die nachfolgenden Gehalte aufweist:

| | |
|---|---|
| 6 bis 8 Vol.-% | Acrolein, |
| 3 bis 9 Vol.-% | molekularer Sauerstoff, |
| 10 bis 20 Vol.-% | Propan, |
| 50 bis 65 Vol.-% | molekularer Stickstoff und |
| 7 bis 13 Vol.-% | Wasserdampf. |

40. Verfahren nach Anspruch 39, **dadurch gekennzeichnet, dass** das Reaktionsgasausgangsgemisch 3 6 bis 9 Vol.-% molekularen Sauerstoff enthält.

**41.** Verfahren nach Anspruch 39 oder 40, **dadurch gekennzeichnet, dass** das Reaktionsgasausgangsgemisch 3 6 bis 7 Vol.-% Acrolein enthält.

**42.** Verfahren nach einem der Ansprüche 39 bis 41, **dadurch gekennzeichnet, dass** das Reaktionsgasausgangsgemisch 3 10 bis 16 Vol.-% Propan enthält.

**43.** Verfahren nach einem der Ansprüche 35 bis 42, **dadurch gekennzeichnet, dass** das Reaktionsgasausgangsgemisch 3 0,5 bis 8 Vol.-% an Methan und/oder Ethan enthält.

**44.** Verfahren nach einem der Ansprüche 34 bis 43, **dadurch gekennzeichnet, dass** die Acroleinbelastung des Katalysatorfestbetts ≥ 135 Nl/l•h bis 290 Nl/l•h beträgt.

**45.** Verfahren nach einem der Ansprüche 34 bis 43, **dadurch gekennzeichnet, dass** der Acroleinumsatz bei einmaligem Durchgang ≥ 90 mol.-% und die damit einhergehende Selektivität der Acrylsäurebildung ≥ 90 mol.-% betragen, und

a) die Belastung des Katalysatorfestbetts mit dem im Reaktionsgasausgangsgemisch 3 enthaltenen Acrolein ≥ 150 Nl Acrolein/l Katalysatorfestbett • h beträgt,
b) das Katalysatorfestbett aus einem in zwei räumlich aufeinanderfolgenden Reaktionszonen C*, D* angeordneten Katalysatorfestbett besteht, wobei die Temperatur der Reaktionszone C* 230 bis 270°C und die Temperatur der Reaktionszone D* 205 bis 300°C beträgt und gleichzeitig wenigstens 5°C oberhalb der Temperatur der Reaktionszone C* liegt,
c) das Reaktionsgasausgangsgemisch 3 die Reaktionszonen C*, D* in der zeitli-chen Abfolge "erst C*", "dann D*" durchströmt und
d) sich die Reaktionszone C* bis zu einem Umsatz des Acroleins von 55 bis 85 mol.-% erstreckt.

**46.** Verfahren nach einem der Ansprüche 1 bis 24, **dadurch gekennzeichnet, dass**

- sich an das Verfahren nach einem der Ansprüche 1 bis 24 gegebenenfalls ein Verfahren der heterogen katalysierten partiellen Gasphasenoxidation von in einem Verfahren nach einem der Ansprüche 1 bis 24 gebildetem Acrolein anschließt in welchem man dieses Acrolein als Bestandteil eines Reaktionsgasausgangsgemischs 3 durch ein Katalysatorfestbett führt, dessen Aktivmasse wenigstens ein die Elemente Mo und V enthaltendes Multimetalloxid ist, und man
- in einer dem Verfahren nach einem der Ansprüche 1 bis 24 vorgeschalteten ersten Stufe Propan als Bestandteil eines Reaktionsgasausgangsgemischs 1 einer partiellen heterogen katalysierten Dehydrierung in der Gasphase unter Bildung eines Produktgasgemischs 1, das Propylen und nicht umgesetztes Propan enthält, unterwirft,
- aus dem Propylen und nicht umgesetztes Propan enthaltenden Produktgasgemisch 1 der vorgeschalteten Stufe von den darin enthaltenen, von Propan und Propylen verschiedenen Bestandteilen gegebenenfalls eine Teilmenge abtrennt und es dann als Bestandteil des Reaktionsgasausgangsgemisch 2 verwendet,
- aus dem im Rahmen der partiellen Gasphasenoxidation resultierenden Produktgasgemisch Acrolein, Acrylsäure oder deren Gemisch als Zielprodukt abtrennt und wenigstens dabei verbleibendes nicht umgesetztes Propan in die vorgeschaltete erste Stufe zurückführt und
- dem Reaktionsgasausgangsgemisch 2 und/oder dem Reaktionsgasausgangsgemisch 3 Frischpropan zusetzt.

**47.** Verfahren nach einem der Ansprüche 34 bis 46, **dadurch gekennzeichnet, dass** sich ein Verfahren anschließt, bei dem man das Produktgasgemisch der Acroleinpartialoxidation nach gegebenenfalls erfolgter direkter und/oder indirekter Kühlung in einer trennwirksame Einbauten enthaltenden Kolonne aufsteigend unter Seitenabzug einer rohen Acrylsäure fraktionierend kondensiert und/oder mit Wasser und/oder wässriger Lösung absorbiert.

**48.** Verfahren nach Anspruch 47, **dadurch gekennzeichnet, dass** sich ein Verfahren anschließt, bei dem die rohe Acrylsäure einer Suspensionskristallisation unter Bildung von Acrylsäuresuspensionskristallisat und verbleibender Mutterlauge unterworfen wird.

**49.** Verfahren nach Anspruch 48, **dadurch gekennzeichnet, dass** sich ein Verfahren anschließt, bei dem das Acrylsäuresuspensionskristallisat mittels einer Waschkolonne von verbliebener Mutterlauge abgetrennt wird.

**50.** Verfahren nach Anspruch 49, **dadurch gekennzeichnet, dass** die Waschkolonne eine solche mit erzwungenem Transport des Kristallbetts ist.

**51.** Verfahren nach Anspruch 49 oder 50, **dadurch gekennzeichnet, dass** die Waschkolonne eine hydraulische Waschkolonne ist.

**52.** Verfahren nach einem der Ansprüche 49 bis 51, **dadurch gekennzeichnet, dass** als Waschflüssigkeit die Schmelze von in der Waschkolonne vorab abgetrennten Acrylsäurekristallen verwendet wird.

**53.** Verfahren nach einem der Ansprüche 49 bis 52, **dadurch gekennzeichnet, dass** sich ein Verfahren anschließt, bei dem das abgetrennte Acrylsäureauspensionskristallisat aufgeschmolzen und in Polymerisate radikalisch einpolymerisiert wird.

**Claims**

**1.** A process for preparing acrolein or acrylic acid or a mixture thereof by heterogeneously catalyzed partial gas phase oxidation of propene, in which a starting reaction gas mixture 2 which comprises the propylene and molecular oxygen reactants and the inert molecular nitrogen and propane diluent gases and comprises the molecular oxygen and the propylene in a molar $O_2:C_3H_6$ ratio of $\geq 1$ is conducted at elevated temperature through a fixed catalyst bed whose active composition is at least one multimetal oxide comprising the elements Mo, Fe and Bi, wherein starting reaction gas mixture 2, based on its total volume, has the following contents:

from 6 to 9% by volume of propylene,
from 8 to 18% by volume of molecular oxygen,
from 6 to 30% by volume of propane and
from 32 to 72% by volume of molecular nitrogen,

with the proviso that the molar ratio $V_1$ of propane present in starting reaction gas mixture 2 to propylene present in starting reaction gas mixture 2 is from 1 to 4, the molar ratio $V_2$ of molecular nitrogen present in starting reaction gas mixture 2 to molecular oxygen present in starting reaction gas mixture 2 is from 2 to 6 and the molar ratio $V_3$ of molecular oxygen present in starting reaction gas mixture 2 to propylene present in starting reaction gas mixture 2 is from 1.3 to 2.4.

**2.** The process according to claim 1, wherein starting reaction gas mixture 2 has the following contents:

from 7 to 9% by volume of propylene,
from 9.8 to 16% by volume of molecular oxygen,
from 9 to 25% by volume of propane and
from 35 to 65% by volume of molecular nitrogen,

with the proviso that

$V_1$ = from 1 to 3.5
$V_2$ = from 3 to 4.5 and
$V_3$ = from 1.4 to 2.2.

**3.** The process according to claim 1, wherein starting reaction gas mixture 2 has the following contents:

from 7 to 9% by volume of propylene,
from 9.8 to 15% by volume of molecular oxygen,
from 10.5 to 20% by volume of propane and
from 40 to 60% by volume of molecular nitrogen,

with the proviso that

$V_1$ = from 1.5 to 2.5
$V_2$ = from 3.5 to 4.5 and
$V_3$ = from 1.4 to 2.14.

**4.** The process according to claim 3, wherein $V_2$ is from 3.5 to 4.

**5.** The process according to claim 3 or 4, wherein $V_3$ is from 1.5 to 2.0.

**6.** The process according to claim 1, wherein starting reaction gas mixture 2 has the following contents:

from 7 to 9% by volume of propylene,
from 9.8 to 15.5% by volume of molecular oxygen,
from 10.5 to 15.5% by volume of propane and
from 40 to 60% by volume of molecular nitrogen,

with the proviso that

$V_1$ = from 1.5 to 2.2
$V_2$ = from 3.5 to 4.5 and
$V_3$ = from 1.5 to 2.14.

**7.** The process according to claim 6, wherein $V_2$ is from 3.5 to 4.

**8.** The process according to claim 6 or 7, wherein $V_3$ is from 1.5 to 2.0.

**9.** The process according to claim 1, wherein starting reaction gas mixture 2 has the following contents:

from 7 to 8% by volume of propylene,
from 11.9 to 15.5% by volume of molecular oxygen,
from 11.9 to 15.5% by volume of propane and
from 50 to 60% by volume of molecular nitrogen,

with the proviso that

$V_1$ = from 1.7 to 2.1
$V_2$ = from 3.5 to 4.5 and
$V_3$ = from 1.7 to 2.1.

**10.** The process according to claim 9, wherein $V_2$ is from 3.5 to 4.

**11.** The process according to claim 9 or 10, wherein $V_3$ is from 1.8 to 2.0.

**12.** The process according to claim 1, wherein starting reaction gas mixture 2 has the following contents:

from 7 to 9% by volume of propylene,
from 9.8 to 15% by volume of molecular oxygen,
from 21 to 28% by volume of propane and
from 40 to 60% by volume of molecular nitrogen,

with the proviso that

$V_1$ = from 3 to 4
$V_2$ = from 3.5 to 4.5 and
$V_3$ = from 1.4 to 2.14.

**13.** The process according to claim 12, wherein $V_2$ is from 3.5 to 4.

**14.** The process according to claim 12 or 13, wherein $V_3$ is from 1.5 to 2.0.

**15.** The process according to any of claims 1 to 14, wherein the total content in starting reaction gas mixture 2 of constituents other than propylene, molecular oxygen, propane and molecular nitrogen is ≤10% by volume.

**16.** The process according to any of claims 1 to 15, wherein starting reaction gas mixture 2 comprises from 0.5 to 8% by volume of at least one of the compounds methane and ethane.

**17.** The process according to any of claims 1 to 15, wherein starting reaction gas mixture 2 comprises from 0.5 to 5% by volume of at least one of the compounds methane and ethane.

**18.** The process according to any of claims 1 to 15, wherein starting reaction gas mixture 2 comprises from 0.5 to 3% by volume of at least one of the compounds methane and ethane.

**19.** The process according to any of claims 1 to 17, wherein starting reaction gas mixture 2 comprises ≤5% by volume of water and ≤5% by volume of carbon oxides.

**20.** The process according to any of claims 1 to 17, wherein starting reaction gas mixture 2 comprises ≤3% by volume of water and ≤3% by volume of carbon oxides.

**21.** The process according to any of claims 1 to 17, wherein starting reaction gas mixture 2 comprises ≤2% by volume of water and ≤2% by volume of carbon oxides.

**22.** The process according to any of claims 1 to 21, wherein starting reaction gas mixture 2 comprises ≥0.5% by volume of water.

**23.** The process according to any of claims 1 to 22, wherein the total content in starting reaction gas mixture 2 of constituents other than propylene, molecular oxygen, propane and molecular nitrogen is ≤5% by volume.

**24.** The process according to any of claims 1 to 22, wherein the total content in starting reaction gas mixture 2 of constituents other than propylene, molecular oxygen, propane and molecular nitrogen is ≤3% by volume.

**25.** The process according to any of claims 1 to 24, wherein the source used for the propylene present in starting reaction gas mixture 2 is propylene formed in processes for the continuous heterogeneously catalyzed partial dehydrogenation and/or oxydehydrogenation of propane in the gas phase, without propane unconverted in the heterogeneously catalyzed partial dehydrogenation and/or oxydehydrogenation being removed beforehand from this propylene.

**26.** The process according to claim 25, wherein the process of a heterogeneously catalyzed partial dehydrogenation of propane is an autothermal dehydrogenation.

**27.** The process according to claim 25 or 26, wherein the process for the heterogeneously catalyzed partial dehydrogenation of propane is one in which

- a starting reaction gas mixture 1 comprising the propane to be dehydrogenated is fed continuously to a dehydrogenation zone,
- in the dehydrogenation zone, starting reaction gas mixture 1 is conducted through at least one fixed catalyst bed over which molecular hydrogen and propylene are formed by catalytic dehydrogenation,
- at least one molecular oxygen-comprising gas is added to starting reaction gas mixture 1 before and/or after entry into the dehydrogenation zone,
- the molecular oxygen is oxidized in the dehydrogenation zone partly to steam in the molecular hydrogen present in reaction gas mixture 1 and
- a product gas which comprises molecular hydrogen, steam, propylene and unconverted propane is withdrawn from the dehydrogenation zone, with the proviso that the product gas withdrawn from the dehydrogenation zone is divided into two portions of identical composition and one of the two portions is recycled into the dehydrogenation zone as dehydrogenation cycle gas.

**28.** The process according to claim 27, wherein the dehydrogenation cycle gas is recycled into starting reaction gas mixture 1.

**29.** The process according to claim 28, wherein starting reaction gas mixture 1 comprises:

from 15 to 25% by volume of propane,
from 2 to 6% by volume of propylene,
from 5 to 20% by volume of steam,
from 2 to 10% by volume of molecular hydrogen,
from 40 to 75% by volume of molecular nitrogen and

from > 0 to 3% by volume of molecular oxygen.

30. The process according to claim 28 or 29, wherein the product gas comprises propane and propylene in a molecular propene to propane ratio of from 0.3 to 0.66.

31. The process according to any of claims 1 to 24, wherein the source used for the propylene present in starting reaction gas mixture 2 is the product gas mixture of a partial propane dehydrogenation in which at least 50% by volume of the constituents other than propane and propylene present in the product gas of the propane dehydrogenation have been removed.

32. The process according to any of claims 1 to 31, wherein the hourly space velocity on the fixed catalyst bed of propylene is from $\geq$ 135 1 (STP)/l•h to 300 1 (STP)/l•h.

33. The process according to any of claims 1 to 31, wherein the propylene conversion in single pass is $\geq$ 90 mol% and the associated selectivity of acrolein formation and of acrylic acid by-product formation taken together is $\geq$ 90 mol% and

a) the hourly space velocity on the fixed catalyst bed of propylene present in starting reaction gas mixture 2 is $\geq$ 160 1 (STP) of propylene/l of fixed catalyst bed • h,
b) the fixed catalyst bed consists of one fixed catalyst bed arranged in two spatially successive reaction zones A*, B*, the temperature of reaction zone A* being from 300 to 390°C and a temperature of reaction zone B* being from 305 to 420°C and at the same time at least 5°C above the temperature of reaction zone A*,
c) starting reaction gas mixture 2 flows through reaction zones A*, B* in the time sequence "first A*", "then B*" and
d) reaction zone A* extends up to a conversion of propene of from 40 to 80 mol%.

34. The process according to any of claims 1 to 33, which is followed by a process for the heterogeneously catalyzed partial gas phase oxidation of acrolein formed in the process according to any of claims 1 to 33 to acrylic acid, in which a starting reaction gas mixture 3 comprising acrolein is conducted through a fixed catalyst bed whose active composition is at least one multimetal oxide comprising the elements Mo and V.

35. The process according to claim 34, wherein starting reaction gas mixture 3 has the following contents:

from 4.5 to 8% by volume of acrolein,
from 2.25 to 9% by volume of molecular oxygen,
from 6 to 30% by volume of propane,
from 32 to 72% by volume of molecular nitrogen,
from 5 to 15% by volume of steam.

36. The process according to claim 35, wherein starting reaction gas mixture 3 comprises from 4.5 to 9% by volume of molecular oxygen.

37. The process according to claim 34, wherein starting reaction gas mixture 3 has the following contents:

from 5.5 to 8% by volume of acrolein,
from 2.75 to 9% by volume of molecular oxygen,
from 10 to 25% by volume of propane,
from 40 to 70% by volume of molecular nitrogen,
from 5 to 15% by volume of steam.

38. The process according to claim 37, wherein starting reaction gas mixture 3 comprises from 5.5 to 9% by volume of molecular oxygen.

39. The process according to claim 34, wherein starting reaction gas mixture 3 has the following contents:

from 6 to 8% by volume of acrolein,
from 3 to 9% by volume of molecular oxygen,
from 10 to 20% by volume of propane,
from 50 to 65% by volume of molecular nitrogen,

from 7 to 13% by volume of steam.

40. The process according to claim 39, wherein starting reaction gas mixture 3 comprises from 6 to 9% by volume of molecular oxygen.

41. The process according to claim 39 or 40, wherein starting reaction gas mixture 3 comprises from 6 to 7% by volume of acrolein.

42. The process according to any of claims 39 to 41, wherein starting reaction gas mixture 3 comprises from 10 to 16% by volume of propane.

43. The process according to any of claims 35 to 42, wherein starting reaction gas mixture 3 comprises from 0.5 to 8% by volume of methane and/or ethane.

44. The process according to any of claims 34 to 43, wherein the acrolein hourly space velocity on the fixed catalyst bed is from ≥ 135 1 (STP)/l•h to 290 1 (STP)/l•h.

45. The process according to any of claims 34 to 43, wherein the acrolein conversion in single pass is ≥ 90 mol% and the associated selectivity of acrylic acid formation is ≥ 90 mol%, and

a) the hourly space velocity on the fixed catalyst bed of acrolein present in starting reaction gas mixture 3 is ≥ 150 1 (STP) of acrolein/l of fixed catalyst bed • h,
b) the fixed catalyst bed consists of one fixed catalyst bed arranged in two spatially successive reaction zones C*, D*, the temperature of reaction zone C* being from 230 to 270°C and a temperature of reaction zone D* being from 205 to 300°C and at the same time at least 5°C above the temperature of reaction zone C*,
c) starting reaction gas mixture 3 flows through reaction zones C*, D* in the time sequence "first C*", "then D*" and
d) reaction zone C* extends up to a conversion of acrolein of from 55 to 85 mol%.

46. The process according to any of claims 1 to 24,

- which is followed if appropriate by a process for the heterogeneously catalyzed partial gas phase oxidation of acrolein formed in the process according to any of claims 1 to 24, in which this acrolein is conducted as a constituent of a starting reaction gas mixture 3 through a fixed catalyst bed whose active composition is at least one multimetal oxide comprising the elements Mo and V, and,
- in a first stage preceding the process according to any of claims 1 to 24, propane is subjected, as a constituent of a starting reaction gas mixture 1, to a partial heterogeneously catalyzed dehydrogenation in the gas phase to form a product gas mixture 1 which comprises propylene and unconverted propane,
- a portion is optionally removed from the constituents other than propane and propylene present in the product gas mixture 1, comprising propylene and unconverted propane, of the preceding stage and it is then used as a constituent of starting reaction gas mixture 2,
- acrolein, acrylic acid or a mixture thereof is removed as the target product from the product gas mixture resulting from the partial gas phase oxidation and at least unconverted propane remaining in this removal is recycled into the preceding first stage and
- fresh propane is added to starting reaction gas mixture 2 and/or to starting reaction gas mixture 3.

47. The process according to any of claims 34 to 46, which is followed by a process in which the product gas mixture of the acrolein partial oxidation, optionally after direct and/or indirect cooling, is fractionally condensed ascending within a column comprising separating internals with side draw removal of crude acrylic acid and/or absorbed with water and/or aqueous solution.

48. The process according to claim 47, which is followed by a process in which the crude acrylic acid is subjected to a suspension crystallization to form acrylic acid suspension crystals and remaining mother liquor.

49. The process according to claim 48, which is followed by a process in which the acrylic acid suspension crystals are removed from remaining mother liquor by means of a wash column.

50. The process according to claim 49, wherein the wash column is one with forced transport of the crystal bed.

**51.** The process according to claim 49 or 50, wherein the wash column is a hydraulic wash column.

**52.** The process according to any of claims 49 to 51, wherein the wash liquid used is the melt of acrylic acid crystals which have been removed beforehand in the wash column.

**53.** The process according to any of claims 49 to 52, which is followed by a process in which the removed acrylic acid suspension crystals are melted and free-radically polymerized to polymers.


**Revendications**

**1.** Procédé pour la préparation d'acroléine ou d'acide acrylique ou de leur mélange par oxydation partielle en phase gazeuse, catalysée par voie hétérogène, de propylène, dans lequel on fait passer un mélange 2 initial gazeux de réaction contenant les réactifs propylène et oxygène moléculaire ainsi que les gaz de dilution inertes azote moléculaire et propane, qui contient l'oxygène moléculaire et le propylène dans un rapport molaire $O_2 : C_3H_6 \geq 1$, à température augmentée à travers un lit fixe catalytique dont la masse active est au moins un oxyde multimétallique contenant les éléments Mo, Fe et Bi, **caractérisé en ce que** le mélange 2 initial gazeux de réaction, par rapport à son volume total, présente les teneurs suivantes
6 à 9% en volume de propylène,
8 à 18% en volume d'oxygène moléculaire,
6 à 30% en volume de propane et
32 à 72% en volume d'azote moléculaire,
de manière telle que le rapport molaire $V_1$, du propane contenu dans le mélange 2 initial gazeux de réaction au propylène contenu dans le mélange 2 initial gazeux de réaction, vaut 1 à 4, le rapport molaire $V_2$, de l'azote moléculaire contenu dans le mélange 2 initial gazeux de réaction à l'oxygène moléculaire contenu dans le mélange 2 initial gazeux de réaction, vaut 2 à 6 et le rapport molaire $V_3$, de l'oxygène moléculaire contenu dans le mélange 2 initial gazeux de réaction au propylène contenu dans le mélange 2 initial gazeux de réaction, vaut 1,3 à 2,4.

**2.** Procédé selon la revendication 1, **caractérisé en ce que** le mélange 2 initial gazeux de réaction présente les teneurs suivantes
7 à 9% en volume de propylène,
9,8 à 16% en volume d'oxygène moléculaire,
9 à 25% en volume de propane et
35 à 65% en volume d'azote moléculaire,
de manière telle que

$V_1$ = 1 à 3,5
$V_2$ = 3 à 4,5 et
$V_3$ = 1,4 à 2,2.

**3.** Procédé selon la revendication 1, **caractérisé en ce que** le mélange 2 initial gazeux de réaction présente les teneurs suivantes
7 à 9% en volume de propylène,
9,8 à 15% en volume d'oxygène moléculaire,
10,5 à 20% en volume de propane et
40 à 60% en volume d'azote moléculaire,
de manière telle que

$V_1$ = 1,5 à 2,5
$V_2$ = 3,5 à 4,5 et
$V_3$ = 1, 4 à 2, 14.

**4.** Procédé selon la revendication 3, **caractérisé en ce que** $V_2$ vaut 3,5 à 4.

**5.** Procédé selon la revendication 3 ou 4, **caractérisé en ce que** $V_3$ vaut 1,5 à 2,0.

**6.** Procédé selon la revendication 1, **caractérisé en ce que** le mélange 2 initial gazeux de réaction présente les teneurs suivantes

7 à 9% en volume de propylène,
9,8 à 15,5% en volume d'oxygène moléculaire,
10,5 à 15,5% en volume de propane et
40 à 60% en volume d'azote moléculaire,
de manière telle que

$$V_1 = 1,5 \text{ à } 2,2$$
$$V_2 = 3,5 \text{ à } 4,5 \text{ et}$$
$$V_3 = 1,5 \text{ à } 2, 14 .$$

7. Procédé selon la revendication 6, **caractérisé en ce que** $V_2$ vaut 3,5 à 4.

8. Procédé selon la revendication 6 ou 7, **caractérisé en ce que** $V_3$ vaut 1,5 à 2,0.

9. Procédé selon la revendication 1, **caractérisé en ce que** le mélange 2 initial gazeux de réaction présente les teneurs suivantes
7 à 8% en volume de propylène,
11,9 à 15,5% en volume d'oxygène moléculaire,
11,9 à 15,5% en volume de propane et
50 à 60% en volume d'azote moléculaire,
de manière telle que

$$V_1 = 1, 7 \text{ à } 2, 1$$
$$V_2 = 3,5 \text{ à } 4,5 \text{ et}$$
$$V_3 = 1, 7 \text{ à } 2,1.$$

10. Procédé selon la revendication 9, **caractérisé en ce que** $V_2$ vaut 3,5 à 4.

11. Procédé selon la revendication 9 ou 10, **caractérisé en ce que** $V_3$ vaut 1,8 à 2,0.

12. Procédé selon la revendication 1, **caractérisé en ce que** le mélange 2 initial gazeux de réaction présente les teneurs suivantes
7 à 9% en volume de propylène,
9,8 à 15% en volume d'oxygène moléculaire,
21 à 28% en volume de propane et
40 à 60% en volume d'azote moléculaire,
de manière telle que

$$V_1 = 3 \text{ à } 4$$
$$V_2 = 3,5 \text{ à } 4,5 \text{ et}$$
$$V_3 = 1,4 \text{ à } 2, 14.$$

13. Procédé selon la revendication 12, **caractérisé en ce que** $V_2$ vaut 3,5 à 4.

14. Procédé selon la revendication 12 ou 13, **caractérisé en ce que** $V_3$ vaut 1,5 à 2,0.

15. Procédé selon l'une quelconque des revendications 1 à 14, **caractérisé en ce que** la teneur totale du mélange 2 initial gazeux de réaction en constituants différents du propylène, de l'oxygène moléculaire, du propane et de l'azote moléculaire est ≤ 10% en volume.

16. Procédé selon l'une quelconque des revendications 1 à 15, **caractérisé en ce que** le mélange 2 initial gazeux de réaction contient, à raison de 0,5 à 8% en volume, au moins un des composés méthane et éthane.

17. Procédé selon l'une quelconque des revendications 1 à 15, **caractérisé en ce que** le mélange 2 initial gazeux de réaction contient, à raison de 0,5 à 5% en volume, au moins un des composés méthane et éthane.

18. Procédé selon l'une quelconque des revendications 1 à 15, **caractérisé en ce que** le mélange 2 initial gazeux de réaction contient, à raison de 0,5 à 3% en volume, au moins un des composés méthane et éthane.

19. Procédé selon l'une quelconque des revendications 1 à 17, **caractérisé en ce que** le mélange 2 initial gazeux de réaction contient ≤ 5% en volume d'eau et ≤ 5% en volume d'oxydes de carbone.

20. Procédé selon l'une quelconque des revendications 1 à 17, **caractérisé en ce que** le mélange 2 initial gazeux de réaction contient ≤ 3% en volume d'eau et ≤ 3% en volume d'oxydes de carbone.

21. Procédé selon l'une quelconque des revendications 1 à 17, **caractérisé en ce que** le mélange 2 initial gazeux de réaction contient ≤ 2% en volume d'eau et ≤ 2% en volume d'oxydes de carbone.

22. Procédé selon l'une quelconque des revendications 1 à 21, **caractérisé en ce que** le mélange 2 initial gazeux de réaction contient ≤ 0,5% en volume d'eau.

23. Procédé selon l'une quelconque des revendications 1 à 22, **caractérisé en ce que** la teneur totale du mélange 2 initial gazeux de réaction en constituants différents du propylène, de l'oxygène moléculaire, du propane et de l'azote moléculaire est ≤ 5% en volume.

24. Procédé selon l'une quelconque des revendications 1 à 22, **caractérisé en ce que** la teneur totale du mélange 2 initial gazeux de réaction en constituants différents du propylène, de l'oxygène moléculaire, du propane et de l'azote moléculaire est ≤ 3% en volume.

25. Procédé selon l'une quelconque des revendications 1 à 24, **caractérisé en ce qu'**on utilise, comme source pour le propylène contenu dans le mélange 2 initial gazeux de réaction, du propylène formé dans la phase gazeuse dans le procédé de la déshydrogénation et/ou de l'oxydéshydrogénation partielle, catalysée par voie hétérogène, continue de propane, sans qu'on ne sépare au préalable de ce propylène le propane non transformé dans la déshydrogénation et/ou l'oxydéshydrogénation partielle catalysée par voie hétérogène.

26. Procédé selon la revendication 25, **caractérisé en ce que** le procédé de la déshydrogénation partielle catalysée par voie hétérogène de propane est une déshydrogénation autothermique.

27. Procédé selon la revendication 25 ou 26, **caractérisé en ce que** le procédé de la déshydrogénation partielle catalysée par voie hétérogène de propane est un procédé dans lequel

- une zone de déshydrogénation est alimentée en continu en mélange 1 réactionnel gazeux initial contenant du propane à déshydrogéner,
- le mélange 1 réactionnel gazeux initial est guidé dans la zone de déshydrogénation à travers au moins un lit fixe catalytique sur lequel de l'hydrogène moléculaire et du propylène sont formés par la déshydrogénation catalytique,
- le mélange 1 réactionnel gazeux initial est additionné, avant et/ou après l'entrée dans la zone de déshydrogénation, d'au moins un gaz contenant de l'oxygène moléculaire,
- l'oxygène moléculaire oxyde partiellement l'hydrogène moléculaire contenu dans la zone de déshydrogénation dans le mélange 1 gazeux réactionnel en vapeur d'eau et
- un gaz produit, qui contient de l'hydrogène moléculaire, de la vapeur d'eau, du propylène et du propane non transformé, est prélevé de la zone de déshydrogénation de manière telle que le gaz produit prélevé de la zone de déshydrogénation est réparti en deux quantités partielles de composition identique et une des deux quantités partielles est recyclée en tant que gaz en circulation de déshydrogénation dans la zone de déshydrogénation.

28. Procédé selon la revendication 27, **caractérisé en ce que** le gaz en circulation de déshydrogénation est recyclé dans le mélange 1 initial gazeux de réaction.

29. Procédé selon la revendication 28, **caractérisé en ce que** le mélange 1 initial gazeux de réaction contient :

15 à 25% en volume de propane,
2 à 6% en volume de propylène,
5 à 20% en volume de vapeur d'eau,
2 à 10% en volume d'hydrogène moléculaire,
40 à 75% en volume d'azote moléculaire et
> 0 à 3% en volume d'oxygène moléculaire.

**30.** Procédé selon la revendication 28 ou 29, **caractérisé en ce que** le gaz produit contient du propane et du propylène dans un rapport molaire de propylène à propane de 0,3 à 0,66.

**31.** Procédé selon l'une quelconque des revendications 1 à 24, **caractérisé en ce qu'**on utilise, comme source pour le propylène contenu dans le mélange 2 initial gazeux de réaction, le mélange gazeux produit d'une déshydrogénation partielle de propane après qu'au moins 50% en volume des constituants différents du propane et du propylène contenus dans le gaz produit de la déshydrogénation du propane aient été séparés.

**32.** Procédé selon l'une quelconque des revendications 1 à 31, **caractérisé en ce que** la charge du lit fixe catalytique en propylène est ≥ 135 Nl/l * h à 300 Nl/l * h.

**33.** Procédé selon l'une quelconque des revendications 1 à 31, **caractérisé en ce que** la conversion du propylène lors d'un passage unique est ≥ 90% en mole et la sélectivité de la formation d'acroléine ainsi que de la formation du produit secondaire, acide acrylique, dans sa totalité, allant de pair avec celle-ci, est ≥ 90% en mole et

a) la charge du lit fixe catalytique en propylène contenu dans le mélange 2 réactionnel gazeux initial est ≥ 160 Nl de propylène/l de lit fixe catalytique * h,
b) le lit fixe catalytique est constitué par un lit fixe catalytique disposé dans deux zones de réaction consécutives dans l'espace A*, B*, la température de la zone de réaction A* étant de 300 à 390°C et la température de la zone de réaction B* étant de 305 à 420°C et simultanément supérieure d'au moins 5°C à la température de la zone de réaction A*,
c) le mélange 2 réactionnel gazeux initial s'écoule, dans les zones de réaction A*, B* dans un ordre dans le temps, "d'abord A*", "puis B*" et
d) la zone de réaction A* s'étend jusqu'à une conversion du propylène de 40 à 80% en mole.

**34.** Procédé selon l'une quelconque des revendications 1 à 33, **caractérisé en ce que** le procédé selon l'une quelconque des revendications 1 à 33 est suivi par un procédé d'oxydation partielle en phase gazeuse, catalysée par voie hétérogène, d'acroléine, formé dans un procédé selon l'une quelconque des revendications 1 à 33, en acide acrylique, dans lequel on guide un mélange 3 initial gazeux de réaction contenant l'acroléine à travers un lit fixe catalytique, dont la masse active est au moins un oxyde multimétallique contenant les éléments Mo et V.

**35.** Procédé selon la revendication 34, **caractérisé en ce que** le mélange 3 initial gazeux de réaction présente les teneurs suivantes :
4,5 à 8% en volume d'acroléine,

2,25 à 9% en volume d'oxygène moléculaire,
6 à 30% en volume de propane,
32 à 72% en volume d'azote moléculaire,
5 à 15% en volume de vapeur d'eau.

**36.** Procédé selon la revendication 35, **caractérisé en ce que** le mélange 3 initial gazeux de réaction contient 4,5 à 9% en volume d'oxygène moléculaire.

**37.** Procédé selon la revendication 34, **caractérisé en ce que** le mélange 3 initial gazeux de réaction présente les teneurs suivantes :

5,5 à 8% en volume d'acroléine,
2,75 à 9% en volume d'oxygène moléculaire,
10 à 25% en volume de propane,
40 à 70% en volume d'azote moléculaire,
5 à 15% en volume de vapeur d'eau.

**38.** Procédé selon la revendication 37, **caractérisé en ce que** le mélange 3 initial gazeux de réaction contient 5,5 à 9% en volume d'oxygène moléculaire.

**39.** Procédé selon la revendication 34, **caractérisé en ce que** le mélange 3 initial gazeux de réaction présente les teneurs suivantes :

6 à 8% en volume d'acroléine,
3 à 9% en volume d'oxygène moléculaire,
10 à 20% en volume de propane,
50 à 65% en volume d'azote moléculaire et
7 à 13% en volume de vapeur d'eau.

**40.** Procédé selon la revendication 39, **caractérisé en ce que** le mélange 3 initial gazeux de réaction contient 6 à 9% en volume d'oxygène moléculaire.

**41.** Procédé selon la revendication 39 ou 40, **caractérisé en ce que** le mélange 3 initial gazeux de réaction contient 6 à 7% en volume d'acroléine.

**42.** Procédé selon l'une quelconque des revendications 39 à 41, **caractérisé en ce que** le mélange 3 initial gazeux de réaction contient 10 à 16% en volume de propane.

**43.** Procédé selon l'une quelconque des revendications 35 à 42, **caractérisé en ce que** le mélange 3 initial gazeux de réaction contient 0,5 à 8% en volume de méthane et/ou d'éthane.

**44.** Procédé selon l'une quelconque des revendications 34 à 43, **caractérisé en ce que** la charge du lit fixe catalytique en acroléine est $\geq$ 135 Nl/l * h à 290 Nl/l * h.

**45.** Procédé selon l'une quelconque des revendications 34 à 43, **caractérisé en ce que** la conversion d'acroléine lors d'un passage unique est $\geq$ 90% en mole et la sélectivité de la formation d'acide acrylique, allant de pair avec celle-ci, est $\geq$ 90% en mole, et

a) la charge du lit fixe catalytique en acroléine contenue dans le mélange 3 réactionnel gazeux initial est $\geq$ 150 Nl d'acroléine/l de lit fixe catalytique * h,
b) le lit fixe catalytique est constitué par un lit fixe catalytique disposé dans deux zones de réaction consécutives dans l'espace C*, D*, la température de la zone de réaction C* étant de 230 à 270°C et la température de la zone de réaction D* étant de 205 à 300°C et simultanément supérieure d'au moins 5°C à la température de la zone de réaction C*,
c) le mélange 3 réactionnel gazeux initial s'écoule, dans les zones de réaction C*, D* dans un ordre dans le temps, "d'abord C*", "puis D*" et
d) la zone de réaction C* s'étend jusqu'à une conversion de l'acroléine de 55 à 85% en mole.

**46.** Procédé selon l'une quelconque des revendications 1 à 24, **caractérisé en ce que**

- le procédé selon l'une quelconque des revendications 1 à 24 est le cas échéant suivi par un procédé d'oxydation partielle en phase gazeuse, catalysée par voie hétérogène, d'acroléine, formée dans un procédé selon l'une quelconque des revendications 1 à 24, dans lequel on guide cette acroléine comme constituant d'un mélange 3 initial gazeux de réaction à travers un lit fixe catalytique, dont la masse active est au moins un oxyde multimétallique contenant les éléments Mo et V et
- on soumet, dans une première étape disposée en amont du procédé selon l'une quelconque des revendications 1 à 24, le propane en tant que constituant d'un mélange 1 initial gazeux de réaction à une déshydrogénation partielle catalysée par voie hétérogène en phase gazeuse avec formation d'un mélange 1 gazeux produit qui contient du propylène et du propane non transformé,
- on sépare le cas échéant, du mélange 1 gazeux produit contenant du propylène et du propane non transformé de l'étape disposée en amont, une quantité partielle des constituants différents du propane et du propylène qui y sont contenus et on l'utilise ensuite comme constituant du mélange 2 initial gazeux de réaction,
- on sépare, du mélange gazeux produit obtenu dans le cadre de l'oxydation partielle en phase gazeuse, l'acroléine, l'acide acrylique ou leur mélange comme produit cible et on recycle au moins le propane non transformé résiduel dans la première étape disposée en amont et
- on ajoute du propane frais au mélange 2 initial gazeux de réaction et/ou au mélange 3 initial gazeux de réaction.

**47.** Procédé selon l'une quelconque des revendications 34 à 46, **caractérisé en ce qu'**il est suivi par un procédé, dans lequel on condense par fractionnement le mélange gazeux produit de l'oxydation partielle d'acroléine, le cas échéant après un refroidissement direct et/ou indirect, montant dans une colonne contenant des chicanes actives en séparation, avec soutirage latéral d'un acide acrylique brut et/ou on l'absorbe avec de l'eau et/ou une solution aqueuse.

**48.** Procédé selon la revendication 47, **caractérisé en ce qu'**il est suivi par un procédé dans lequel l'acide acrylique brut est soumis à une cristallisation en suspension avec formation d'un produit cristallisé de suspension d'acide acrylique et de lessive-mère résiduelle.

**49.** Procédé selon la revendication 48, **caractérisé en ce qu'**il est suivi par un procédé dans lequel le produit cristallisé de suspension d'acide acrylique est séparé au moyen d'une colonne de lavage de la lessive-mère résiduelle.

**50.** Procédé selon la revendication 49, **caractérisé en ce que** la colonne de lavage est une colonne avec un transport forcé du lit de cristaux.

**51.** Procédé selon la revendication 49 ou 50, **caractérisé en ce que** la colonne de lavage est une colonne de lavage hydraulique.

**52.** Procédé selon l'une quelconque des revendications 49 à 51, **caractérisé en ce qu'**on utilise comme liquide de lavage la masse fondue de cristaux d'acide acrylique séparés au préalable dans la colonne de lavage.

**53.** Procédé selon l'une quelconque des revendications 49 à 52, **caractérisé en ce qu'**il est suivi par un procédé, dans lequel le produit cristallisé de suspension d'acide acrylique séparé est fondu et copolymérisé par voie radicalaire dans des polymères.

EP 1 765 753 B1

# IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1106598 A
- WO 9736849 A
- EP 293224 A
- WO 0196271 A
- DE 19837517 A
- EP 274681 A
- DE 19837519 A
- DE 19837520 A
- EP 117146 A
- WO 0311804 A
- US 3161670 A
- WO 0196270 A
- DE 19508558 A
- DE 3313573 A
- DE 10245585 A
- WO 03076370 A
- DE 10316039 A
- WO 04031106 A
- DE 102004032129 A
- WO 04085369 A
- WO 03011804 A
- WO 0196370 A
- WO 0376370 A
- EP 990636 A
- EP 1070700 A
- WO 0053556 A
- WO 0485365 A
- WO 0485367 A
- WO 0485369 A
- WO 0485370 A
- WO 0485363 A
- WO 0053559 A
- WO 0485362 A
- WO 0053557 A
- DE 19948248 A
- DE 10246119 A
- DE 10219879 A
- EP 731077 A
- DE 10211275 A
- DE 10131297 A
- WO 9946039 A
- US 4788371 A
- EP 0705136 A
- WO 9929420 A
- US 4220091 A
- US 5430220 A

- US 5877369 A
- EP 0117146 A
- DE 19937196 A
- DE 19937105 A
- DE 19937107 A
- WO 0251547 A
- WO 0251540 A
- DE 102005002127 A
- EP 1180508 A
- US 4886928 A
- US 5430209 A
- US 5530171 A
- US 5527979 A
- US 5563314 A
- DE 10235419 A
- DE 4308087 A
- WO 04007405 A
- WO 0196170 A
- EP 700714 A
- EP 700893 A
- DE 10313212 A
- EP 1159248 A
- EP 1159246 A
- EP 1159247 A
- DE 10101695 A
- WO 04085368 A
- DE 102004021764
- WO 04085362 A
- WO 04085370 A
- WO 04085365 A
- WO 04085367 A
- EP 1007007 A
- EP 253409 A
- DE 4431957 A
- DE 102004025445 A
- DE 4431949 A
- DE 10325488 A
- DE 10325487 A
- DE 10353954 A
- DE 10344149 A
- DE 10351269 A
- DE 10350812 A
- DE 10350822 A
- DE 19955176 A
- DE 19948523 A
- DE 19955168 A
- DE 10046957 A
- DE 10063162 A
- DE 3338380 C

- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- DE 4023239 A
- DE 2909671 A
- EP 293859 A
- EP 714700 A
- DE 10046928 A
- DE 19815281 A
- DE 4335973 A
- EP 668104 A
- DE 19736105 A
- DE 19740493 A
- DE 19528646 A
- DE 19910506 A
- EP 1159244 A
- WO 04085363 A
- DE 19910508
- DE 10121592 A
- EP 911313 A
- EP 979813 A
- DE 2830765 A
- DE 19910508 A
- WO 0408536 A
- DE 19948523
- DE 19910506
- DE 19948241
- DE 2830765 C
- DE 2513405 C
- US 3147084 A
- DE 2201528 A
- EP 383224 A
- DE 2903218 A
- EP 468290 B
- DE 2201528 B
- EP 382098 A
- WO 0136364 A
- DE 10261186 A
- EP 20219277 A
- EP 200219278 A
- EP 20219279 A
- EP 0214187 W
- EP 0214188 W
- EP 0214189 W
- EP 873783 A
- EP 1270065 A
- DE 4442346 A
- EP 1388533 A
- EP 1388532 A
- DE 10235847 A
- EP 792867 A
- WO 9801415 A
- EP 1015411 A
- EP 1015410 A
- WO 9950219 A
- WO 0053560 A
- WO 0209839 A
- WO 03041833 A
- DE 10223058 A
- DE 10243625 A
- DE 10336386 A
- EP 854129 A
- US 4317926 A
- DE 19606877 A
- DE 190501325 A
- DE 10247240 A
- DE 19740253 A
- EP 695736 A
- EP 982287 A
- EP 1041062 A
- DE 4335172 A
- DE 4436243 A
- DE 19924532 A
- DE 10332758 A
- DE 19924533 A
- EP 982289 A
- DE 10115277 A
- DE 19740252 A
- DE 19627847 A
- EP 920408 A
- EP 1068174 A
- EP 1066239 A
- EP 1066240 A
- WO 0053561 A
- DE 10053086 A
- EP 982288 A
- WO 2004063138 A
- WO 2004035514 A
- WO 0177056 A
- WO 03041832 A
- WO 02055469 A
- WO 03078378 A